# EUROPEAN PATENT SPECIFICATION

(11) **EP 3 860 561 B1**
(45) Date of publication and mention of the grant of the patent: **21.06.2023**
(21) Application number: 19789841.4
(22) Date of filing: 27.09.2019
(51) Int. Cl.: A61K 9/00, A61K 9/127, A61K 31/7088, C07C 211/00, C07C 219/00, C12N 15/88, C07D 295/15, A61K 31/713, A61K 47/60, A61K 47/69, A61P 37/02, C07C 217/08, C07C 219/06, C07C 229/30, C12N 15/11

(54) **BIODEGRADABLE LIPIDS FOR THE DELIVERY OF ACTIVE AGENTS**
BIOLOGISCH ABBAUBARE LIPIDE ZUR FREISETZUNG VON WIRKSTOFFEN
LIPIDES BIODÉGRADABLES POUR L'ADMINISTRATION D'AGENTS ACTIFS

(30) Priority: 01.10.2018 US 201862739548 P
(43) Date of publication of application: 11.08.2021
(60) Divisional application: 23157471.6
(73) Proprietor: Alnylam Pharmaceuticals, Inc., Cambridge, MA 02142 (US)
(72) Inventor: JAYARAMAN, Muthusamy, Cambridge, MA 02142 (US); HE, Guo, Cambridge, MA 02142 (US); MAIER, Martin, Cambridge, MA 02142 (US)
(74) Representative: Müller-Boré & Partner Patentanwälte PartG mbB
(86) International application number: PCT/US2019/053617
(87) International publication number: WO 2020/072324

(56) References cited:
- WO-A1-2014/089239
- STEVEN L COLLETTI ET AL: "Novel low molecular weight, biodegradable cationic lipids for oligonucleotide delivery", WO2013116126 A1, vol. 1014, 1 January 2013 (2013-01-01), pages 1-40, XP055653409, -& WO 2013/116126 A1 (MERCK SHARP & DOHME [US]; COLLETTI STEVEN L [US] ET AL.) 8 August 2013 (2013-08-08)

## Description

The present invention relates to biodegradable lipids, lipid particles comprising the same, pharmaceutical composition comprising said lipid particles, and to their use in methods of modulating the expression of a target gene in a cell.

Therapeutic nucleic acids include, e.g., small interfering RNA (siRNA), micro RNA (miRNA), antisense oligonucleotides, ribozymes, plasmids, immune stimulating nucleic acids, antisense, antagomir, antimir, microRNA mimic, supermir, U1 adaptor, and aptamer. In the case of siRNA or miRNA, these nucleic acids can down-regulate intracellular levels of specific proteins through a process termed RNA interference (RNAi). The therapeutic applications of RNAi are extremely broad, since siRNA and miRNA constructs can be synthesized with any nucleotide sequence directed against a target protein. To date, siRNA constructs have shown the ability to specifically down-regulate target proteins in both *in vitro* and *in vivo* models. In addition, siRNA constructs are currently being evaluated in clinical studies.

However, two problems currently faced by siRNA or miRNA constructs are, first, their susceptibility to nuclease digestion in plasma and, second, their limited ability to gain access to the intracellular compartment where they can bind the protein RISC when administered systemically as the free siRNA or miRNA. Lipid nanoparticles formed from cationic lipids with other lipid components, such as cholesterol and PEG lipids, and oligonucleotides (such as siRNA and miRNA) have been used to facilitate the cellular uptake of the oligonucleotides.

In this context, WO 2014/089239 A1 describes lipid nanoparticles containing a biodegradable cationic lipid which provide delivery of active pharmaceutical ingredients, such as siRNA. Further, WO 2013/116126 A1 describes cationic lipids that can be used in combination with other lipid components such as cholesterol and PEG-lipids to form lipid nanoparticles with oligonucleotides.There remains a need for improved cationic lipids and lipid nanoparticles for the delivery of oligonucleotides. Preferably, these lipid nanoparticles would provide high drug:lipid ratios, protect the nucleic acid from degradation and clearance in serum, be suitable for systemic delivery, and provide intracellular delivery of the nucleic acid. In addition, these lipid-nucleic acid particles should be well-tolerated and provide an adequate therapeutic index, such that patient treatment at an effective dose of the nucleic acid is not associated with significant toxicity and/or risk to the patient.

The present invention is set out in the appended claims.

Disclosed are the following embodiments.

One embodiment is a lipid particle comprising a biodegradable cationic lipid, a neutral lipid, a sterol, and a lipid capable of reducing aggregation (e.g., PEG-modified lipid), where the molar ratio of the biodegradable cationic lipid to the sterol ranges from about 1.6:1 to about 2.0:1 and/or the molar ratio of the biodegradable cationic lipid to the neutral lipid ranges from about 5.5:1 to about 5.9:1. The inventors have surprisingly found that lipid particles having certain higher contents of biodegradable cationic lipid relative to the amount sterol and/or neutral lipid exhibit enhanced efficacy for the delivery of an active agent (e.g., siRNA). In one embodiment, the biodegradable cationic lipid comprises a lipid moiety, where the lipid moiety has one or more biodegradable groups (such as an ester group (-C(O)O- or-OC(O)-). In one preferred embodiment, the lipid capable of reducing aggregation is 1,2-dimyristoyl-sn-glycerol-methoxy polyethylene glycol (PEG-DMG), such as PEG-DMG with an average polyethylene glycol molecular weight of 2000.

In a further embodiment, the molar ratio of the biodegradable cationic lipid to the sterol is from about 1.7 to about 1.9:1, such as about 1.9:1. In another embodiment, the molar ratio of the biodegradable cationic lipid to the neutral lipid ranges from about 5.5:1 to about 5.8:1, such as about 5.8:1.

In one embodiment, the lipid particle comprises from about 55 to about 60 mol % of the biodegradable cationic lipid, such as about 58 mol % (based on 100 mol % of the lipid components in the lipid particle). The lipid particle may comprise from about 28 to about 33 mol % of the sterol, such as from about 28 to about 32 mol % (based on 100 mol % of the lipid components in the lipid particle). In one embodiment, the lipid particle comprises from about 3 to about 12 mol %, such as from about 5 to about 12 mol %, from about 8 to about 12 mol %, or from about 9 to about 11 mol %, of the neutral lipid (based on 100 mol % of the lipid components in the lipid particle). In another embodiment, the lipid particle comprises about 10 mol % of the neutral lipid (based on 100 mol % of the lipid components in the lipid particle). In yet another embodiment, the lipid particle comprises from about 0.5 to about 10 mol %, such as from about 0.5 to about 5 mol % or from about 1 to about 3 mol %, of the lipid capable of reducing aggregation (e.g., a PEG-modified lipid) (based on 100 mol % of the lipid components in the lipid particle).

Another embodiment is a lipid particle comprising a biodegradable cationic lipid, a neutral lipid, a sterol, and a lipid capable of reducing aggregation (e.g., PEG-modified lipid), where the lipid particle comprises from about 55 to about 60 mol % of the biodegradable cationic lipid and from about 33 to about 28 mol % of the sterol (based on 100 mol % of the lipid components in the lipid particle). In one embodiment, the lipid particle comprises about 58 mol % of the biodegradable cationic lipid (based on 100 mol % of the lipid components in the lipid particle). In another embodiment, the lipid particle comprises from about 3 to about 12% of the neutral lipid, and from about 0.5 to about 10 mol % of the lipid capable of reducing aggregation (based on 100 mol % of the lipid components in the lipid particle). In yet another embodiment, the lipid particle comprises about 10 mol % of the neutral lipid (based on 100 mol % of the lipid components in the lipid particle). In yet another embodiment, the lipid particle comprises about 2 mol % of the lipid capable of reducing aggregation (based on 100 mol % of the lipid components in the lipid particle). In one embodiment, the lipid particle comprises from about 55 to about 60 mol % of the cationic lipid, from about 3 to about 12% of the neutral lipid, from about 28 to about 33 mol % of the sterol, and from about 0.5 to about 10 mol % of the lipid capable of reducing aggregation (based on 100 mol % of the lipid components in the lipid particle). In yet another embodiment, the lipid particle comprises about 58% of the cationic lipid, about 10% of the neutral lipid, about 30% of the sterol, and about 2% of the lipid capable of reducing aggregation (based on 100 mol % of the lipid components in the lipid particle). In yet another embodiment, the lipid particle comprises about 55% of the cationic lipid, about 10% of the neutral lipid, about 33% of the sterol, and about 2% of the lipid capable of reducing aggregation (based on 100 mol % of the lipid components in the lipid particle). In one preferred embodiment, the lipid capable of reducing aggregation is 1,2-dimyristoyl-sn-glycerol-methoxy polyethylene glycol (PEG-DMG), such as PEG-DMG with an average polyethylene glycol molecular weight of 2000.

One embodiment is a cationic lipid having the formula (A): 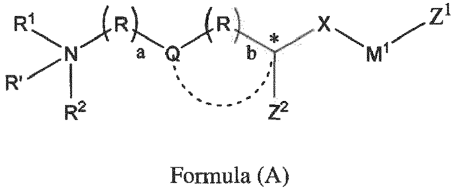 or a salt thereof (e.g., a pharmaceutically acceptable salt thereof), wherein
R' is absent, hydrogen, or alkyl (e.g., C₁-C₄ alkyl);
with respect to R¹ and R²,
   (i) R¹ and R² are each, independently, optionally substituted alkyl, alkenyl, alkynyl, cycloalkylalkyl, heterocycle, or R¹⁰;
   (ii) R¹ and R², together with the nitrogen atom to which they are attached, form an optionally substituted heterocylic ring; or
   (iii) one of R¹ and R² is optionally substituted alkyl, alkenyl, alkynyl, cycloalkyl, cycloalkylalkyl, or heterocycle, and the other forms a 4-10 membered heterocyclic ring or heteroaryl (e.g., a 6-member ring) with (a) the adjacent nitrogen atom and (b) the (R)ₐ group adjacent to the nitrogen atom;
each occurrence of R is, independently, -(CR³R⁴)-;
each occurrence of R³ and R⁴ are, independently H, halogen, OH, alkyl, alkoxy, -NH₂, R¹⁰, alkylamino, or dialkylamino (in one preferred embodiment, each occurrence of R³ and R⁴ are, independently H or C₁-C₄ alkyl);
each occurrence of R¹⁰ is independently selected from PEG and polymers based on poly(oxazoline), poly(ethylene oxide), poly(vinyl alcohol), poly(glycerol), poly(N-vinylpyrrolidone), poly[N-(2-hydroxypropyl)methacrylamide] and poly(amino acid)s, wherein (i) the PEG or polymer is linear or branched, (ii) the PEG or polymer is polymerized by n subunits, (iii) n is a number-averaged degree of polymerization between 10 and 200 units, and (iv) wherein the compound of formula has at most two R¹⁰ groups (preferably at most one R¹⁰ group);
the dashed line to Q is absent or a bond;
when the dashed line to Q is absent then Q is absent or is -O-, -NH-, -N(R⁵)-, -S-, -C(O)-, -C(O)O-, -OC(O)-, -C(O)N(R⁴)-, -N(R⁵)C(O)-, -S-S-, -OC(O)O-, -O-N=C(R⁵)-, -C(R⁵)=N-O-,-OC(O)N(R⁵)-, -N(R⁵)C(O)N(R⁵)-, -N(R⁵)C(O)O-, -C(O)S-, -C(S)O- or -C(R⁵)=N-O-C(O)-; or
when the dashed line to Q is a bond then (i) b is 0 and (ii) Q and the tertiary carbon adjacent to it (C*) form a substituted or unsubstituted, mono- or bi-cyclic heterocyclic group having from 5 to 10 ring atoms (e.g., the heteroatoms in the heterocyclic group are selected from O and S, preferably O);
each occurrence of R⁵ is, independently, H or alkyl (e.g. C₁-C₄ alkyl);
X is alkylene or alkenylene (e.g., C₄ to C₂₀ alkylene or C₄ to C₂₀ alkenylene);
M¹ is a biodegradable group (e.g., -OC(O)-, -C(O)O-, -SC(O)-, -C(O)S-, -OC(S)-, -C(S)O-, -S-S-, -C(R⁵)=N-, -N=C(R⁵)-, -C(R⁵)=N-O-, -O-N=C(R⁵)-, -C(O)(NR⁵)-, -N(R⁵)C(O)-,-C(S)(NR⁵)-, -N(R⁵)C(O)-, -N(R⁵)C(O)N(R⁵)-, -OC(O)O-, -OSi(R⁵)₂O-, -C(O)(CR³R⁴)C(O)O-,-OC(O)(CR³R⁴)C(O)-, or 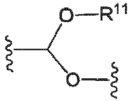 (wherein R¹¹ is a C₂-C₈ alkyl or alkenyl));
a is 1, 2, 3, 4, 5 or 6;
b is 0, 1, 2, or 3;
Z¹ is a C₆-C₁₄ branched alkyl group; and
Z² is a C₄-C₂₀ alkenyl, wherein the alkenyl group may optionally be substituted with one or two fluorine atoms at the alpha position to a double bond which is between the double bond and the terminus of Z² (e.g.,

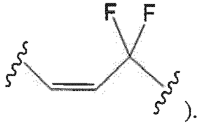

The R'R¹R²N-(R)ₐ-Q-(R)_{b}- group can be any of the head groups described herein, including those shown in Table 1 below, and salts thereof. In one preferred embodiment, R'R¹R²N-(R)ₐ-Q-(R)b- is (CH₃)₂N-(CH₂)₂-, (CH₃)₂N-(CH₂)₃-C(O)O-, (CH₃)₂N-(CH₂)₂-NH-C(O)O-, (CH₃)₂N-(CH₂)₂-OC(O)-NH-, or (CH₃)₂N-(CH₂)₃-C(CH₃)=N-O-. In a preferred embodiment, R'R¹R²N-(R)ₐ-Q-(R)_{b}- is (CH₃)₂N-(CH₂)₂-.

In one embodiment, R¹ and R² are both alkyl (e.g., methyl, ethyl or a combination thereof). In one embodiment, R¹ and R² are both methyl. In another embodiment, one of R¹ and R² is methyl and the other of R¹ and R² is ethyl.

In a further embodiment, a is 2. In another embodiment, b is 0. In another embodiment Q is absent. In yet another embodiment, a is 2, b is 0 and Q is absent. In yet another embodiment, a is 4, b is 0 and Q is -O-.

In another embodiment, X is -(CH₂)ₙ- wherein n is 4 to 20, e.g., 4 to 18, 4 to 16, or 4 to 12. In one embodiment, n is 4, 5, 6, 7, 8, 9, or 10. In one embodiment, X is -(CH₂)₇₋₉-. In one exemplary embodiment, X is -(CH₂)₇-. In one exemplary embodiment, X is -(CH₂)₈-. In one exemplary embodiment, X is -(CH₂)₉-.

In further embodiments, M¹ is -OC(O)- or -C(O)O-. For example, in one embodiment, M¹ is -C(O)O-. In another embodiment, M¹ is -OC(O)-.

In another embodiment, Z¹ is a C₆-C₁₀ branched alkyl group, e.g.,-CH(CH₂CH₃)(CH₂CH₂CH₂CH₃), -CH₂CH(ⁱPr)(CH₂CH₂ⁱPr) or -CH₂CH(n-Bu)₂.

In another embodiment, Z² is C₁₉ alkenyl containing one or two double bonds. For example, Z₂ is -(CH₂)₉CH=CHCH₂CH=CH(CH₂)₄CH₃.

Yet another embodiment is a cationic lipid selected from: 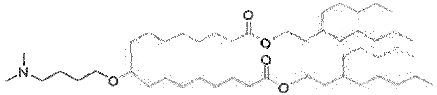 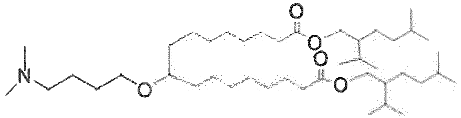 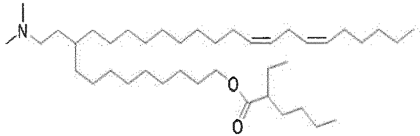 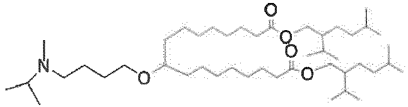 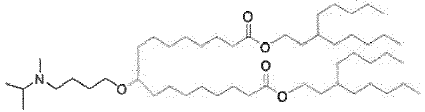 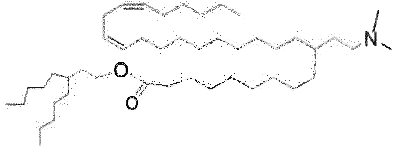 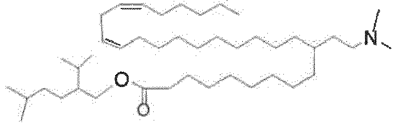 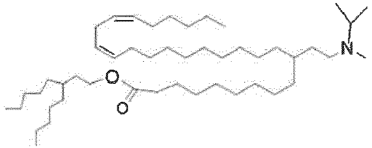 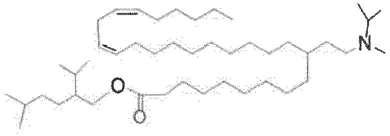 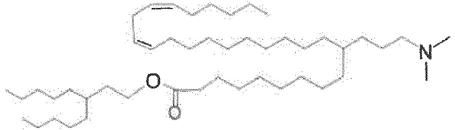 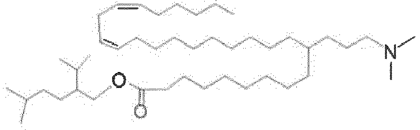 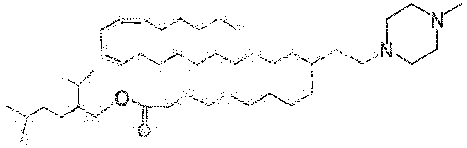 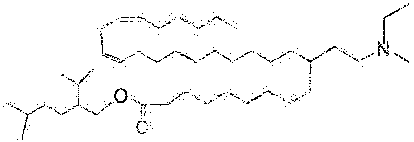 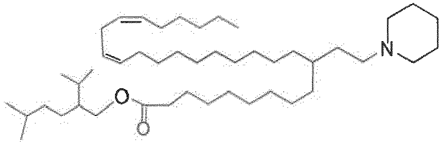 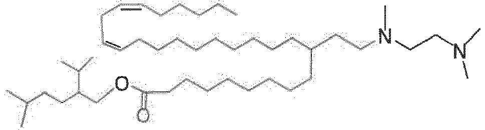 and salts thereof (e.g., pharmaceutically acceptable salts thereof).

Yet embodiment is a compound having the formula (A-I): 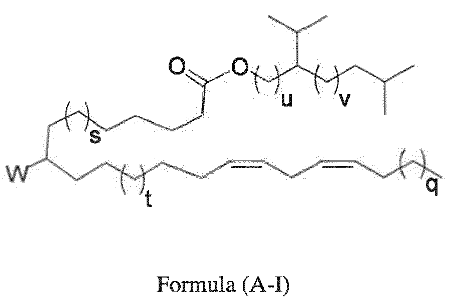 or a salt thereof (e.g., pharmaceutically acceptable salt thereof); wherein
s, t, u, v and q are each, independently, 0, 1, 2, 3, 4, 5, 6 or 7; and
W is a head group (e.g., a protonatable amine group having a pKa of between about 4 and about 11, such as between about 4 and about 7, between about 5 and about 7, or between about 5.5 and about 6.8).

Suitable head groups include any of those described herein (*see,* e.g., Table 1A).

In certain embodiments, the head group is (CH₃)₂N-(CH₂)₂-, (CH₃)₂N-(CH₂)₃-C(O)O-, (CH₃)₂N-(CH₂)₂-NH-C(O)O-, (CH₃)₂N-(CH₂)₂-OC(O)-NH-, or (CH₃)₂N-(CH₂)₃-C(CH₃)=N-O-. In one embodiment, the head group is (CH₃)₂N-(CH₂)₂-.

In one embodiment, variable s is 3 to 5, such as 4.

In one embodiment, variable t is 4 to 6, such as 5.

In one embodiment, variable q is 2 to 4, such as 3.

In one embodiment, variable u is 0 to 2, such as 1.

In one embodiment, variable v is 0 to 2, such as 1.

Yet another embodiment is a compound having the formula (A-II): 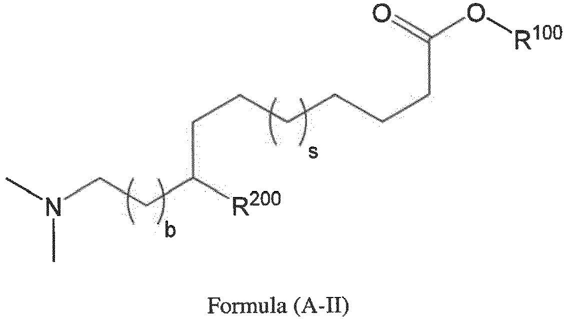 or a salt thereof (e.g., pharmaceutically acceptable salt thereof) wherein
b is 0, 1, 2 or 3;
s is 0,1, 2, 3,4 or 5;
R²⁰⁰ is C₁₂-C₂₂ alkyl, C₁₂-C₂₂ alkyenyl, or C₁₂-C₂₂ alkynyl; and
R¹⁰⁰ is C₅-C₁₅ alkyl, C₅-C₁₅ alkyenyl, or C₅-C₁₅ alkynyl.

In one embodiment, b is 1.

In one embodiment, s is 2, 3, or 4, such as 3.

In one embodiment, R¹⁰⁰ is a C₈-C₁₂ alkyl.

In one embodiment, R²⁰⁰ is C₁₂-C₁₄ alkyl, C₁₂-C₁₄ alkyenyl, or C₁₂-C₁₄ alkynyl.

In another embodiment, R²⁰⁰ is C₁₈-C₂₀ alkyl, C₁₈-C₂₀ alkyenyl, or C₁₈-C₂₀ alkynyl.

These cationic lipids described herein can be incorporated into lipid particles. One embodiment is a lipid particle comprising a cationic lipid, such as those described above (including those of formula (A), (A-I) and (A-II)). Each of the cationic lipids described herein includes one or more biodegradable groups. The biodegradable groups are located in a lipidic moiety (e.g., a hydrophobic chain) of the cationic lipid. These cationic lipids may be incorporated into a lipid particle for delivering an active agent, such as a nucleic acid (e.g., an siRNA). The incorporation of the biodegradable group(s) into the lipid results in faster metabolism and removal of the lipid from the body following delivery of the active agent to a target area. As a result, these lipids have lower toxicity than similar lipids without the biodegradable groups.

The lipid particles described herein may further comprise an active agent. Non-limiting examples of active agents include nucleic acids, such as plasmids, immunostimulatory oligonucleotides, siRNAs, antisense oligonucleotides, microRNAs, antagomirs, aptamers, and ribozymes. In one preferred embodiment, the nucleic acid is an siRNA.

The lipid particles described herein may be incorporated into a pharmaceutical composition. One embodiment is a pharmaceutical composition comprising a lipid particle described herein and a pharmaceutically acceptable carrier. The lipid particle preferably includes an active agent, such as a nucleic acid. In one preferred embodiment, the active agent is an siRNA.

Yet another embodiment is a method of modulating the expression of a target gene in a cell, comprising providing to the cell a lipid particle described herein. In one embodiment, the active agent is a nucleic acid is an siRNA.

Yet another embodiment is a method of treating a disease or disorder characterized by the overexpression of a polypeptide in a subject, comprising providing to the subject a pharmaceutical composition described herein. In one embodiment, the active agent is a nucleic acid selected from the group consisting of an siRNA, a microRNA, and an antisense oligonucleotide, and wherein the siRNA, microRNA, or antisense oligonucleotide includes a polynucleotide that specifically binds to a polynucleotide that encodes the polypeptide, or a complement thereof.

Yet another embodiment is a method of treating a disease or disorder characterized by underexpression of a polypeptide in a subject, comprising providing to the subject a pharmaceutical composition described herein. In one preferred embodiment, the active agent is a plasmid that encodes the polypeptide or a functional variant or fragment thereof.

Yet another embodiment is a method of inducing an immune response in a subject, comprising providing to the subject a pharmaceutical composition described herein. In one preferred embodiment, the active agent is an immunostimulatory oligonucleotide.

The figures show:
Figure 1 is a bar graph depicting the relative Factor VII protein levels following administration of the lipid formulations described in Example 4.
Figure 2 is a bar graph depicting the relative Factor VII protein levels following administration of the lipid formulations described in Example 4.
Figure 3 is a bar graph depicting the relative Factor VII protein levels following administration of the lipid formulations described in Example 4.
Figure 4 is a bar graph depicting the relative Factor VII protein levels following administration of the lipid formulations described in Example 4.
Figure 5 is a graph depicting the relative Factor XII plasma levels following administration of the lipid formulations described in Example 5.
Figure 6A is a graph depicting the relative Factor XII plasma levels following administration of the lipid formulations AF-094 (0.03, 0.1, and 0.3 mg/kg) and AF-011 (0.3 mg/kg) described in Example 6.
Figure 6B is a graph depicting the relative Factor XII plasma levels following administration of the lipid formulations AF-079 (0.3 mg/kg) and AF-011 (0.3 mg/kg) described in Example 6.
Figure 7 is a graph depicting the relative Factor XII plasma levels following administration of the lipid formulations AF-073 (0.03, 0.1, and 0.3 mg/kg) and AF-011 (0.3 mg/kg) described in Example 6.
Figure 8 is a graph depicting the relative Factor XII plasma levels following administration of the lipid formulations AF-093 (0.03, 0.1, and 0.3 mg/kg) and AF-011 (0.3 mg/kg) described in Example 6.
Figure 9 is a graph depicting the relative Factor XII plasma levels following administration of the lipid formulations AF-083 (0.1 and 0.3 mg/kg) and AF-011 (0.3 mg/kg) described in Example 6.

### The Cationic Lipid

The cationic lipid can be any biodegradable cationic lipid known in the art, such as those described in International Publication Nos. WO 2011/153493, WO 2013/086322, WO 2013/086354, and WO 2013/086373, U.S. Patent Nos. 9,012,498, 9,061,063, and 9,463,247, and U.S. Patent Publication No. 2014/0308304. These biodegradable cationic lipids include one or more biodegradable groups. This results in faster metabolism and removal of the cationic lipid from the body following delivery of the active agent to a target area. As a result, these cationic lipids have substantially lower toxicity than similar cationic lipids without the biodegradable groups. In one embodiment, one or more biodegradable groups are located in the mid- or distal section of a lipidic moiety (e.g., a hydrophobic chain) of the cationic lipid.

In one embodiment, the biodegradable cationic lipid has the formula (A): 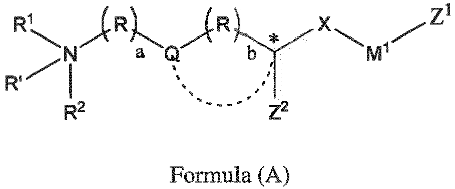 or a salt thereof (e.g., a pharmaceutically acceptable salt thereof), wherein
R' is absent, hydrogen, or alkyl (e.g., C₁-C₄ alkyl);
with respect to R¹ and R²,
   (i) R¹ and R² are each, independently, optionally substituted alkyl, alkenyl, alkynyl, cycloalkylalkyl, heterocycle, or R¹⁰;
   (ii) R¹ and R², together with the nitrogen atom to which they are attached, form an optionally substituted heterocylic ring; or
   (iii) one of R¹ and R² is optionally substituted alkyl, alkenyl, alkynyl, cycloalkyl, cycloalkylalkyl, or heterocycle, and the other forms a 4-10 membered heterocyclic ring or heteroaryl (e.g., a 6-member ring) with (a) the adjacent nitrogen atom and (b) the (R)ₐ group adjacent to the nitrogen atom;
each occurrence of R is, independently, -(CR³R⁴)-;
each occurrence of R³ and R⁴ are, independently H, halogen, OH, alkyl, alkoxy, -NH₂, R¹⁰, alkylamino, or dialkylamino (in one preferred embodiment, each occurrence of R³ and R⁴ are, independently H or C₁-C₄ alkyl);
each occurrence of R¹⁰ is independently selected from PEG and polymers based on poly(oxazoline), poly(ethylene oxide), poly(vinyl alcohol), poly(glycerol), poly(N-vinylpyrrolidone), poly[N-(2-hydroxypropyl)methacrylamide] and poly(amino acid)s, wherein (i) the PEG or polymer is linear or branched, (ii) the PEG or polymer is polymerized by n subunits, (iii) n is a number-averaged degree of polymerization between 10 and 200 units, and (iv) wherein the compound of formula has at most two R¹⁰ groups (preferably at most one R¹⁰ group);
the dashed line to Q is absent or a bond;
when the dashed line to Q is absent then Q is absent or is -O-, -NH-, -N(R⁵)-, -S-, -C(O)-, -C(O)O-, -OC(O)-, -C(O)N(R⁴)-, -N(R⁵)C(O)-, -S-S-, -OC(O)O-, -O-N=C(R⁵)-, -C(R⁵)=N-O-,-OC(O)N(R⁵)-, -N(R⁵)C(O)N(R⁵)-, -N(R⁵)C(O)O-, -C(O)S-, -C(S)O- or -C(R⁵)=N-O-C(O)-; or
when the dashed line to Q is a bond then (i) b is 0 and (ii) Q and the tertiary carbon adjacent to it (C^{∗}) form a substituted or unsubstituted, mono- or bi-cyclic heterocyclic group having from 5 to 10 ring atoms (e.g., the heteroatoms in the heterocyclic group are selected from O and S, preferably O);
each occurrence of R⁵ is, independently, H or alkyl (e.g. C₁-C₄ alkyl);
X is alkylene or alkenylene (e.g., C₄ to C₂₀ alkylene or C₄ to C₂₀ alkenylene);
M¹ is a biodegradable group (e.g., -OC(O)-, -C(O)O-, -SC(O)-, -C(O)S-, -OC(S)-, -C(S)O-, -S-S-, -C(R⁵)=N-, -N=C(R⁵)-, -C(R⁵)=N-O-, -O-N=C(R⁵)-, -C(O)(NR⁵)-, -N(R⁵)C(O)-,-C(S)(NR⁵)-, -N(R⁵)C(O)-, -N(R⁵)C(O)N(R⁵)-, -OC(O)O-, -OSi(R⁵)₂O-, -C(O)(CR³R⁴)C(O)O-,-OC(O)(CR³R⁴)C(O)-, or 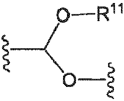 (wherein R¹¹ is a C₂-C₈ alkyl or alkenyl));
a is 1, 2, 3, 4, 5 or 6;
b is 0, 1, 2, or 3;
Z¹ is a C₆-C₁₄ branched alkyl group; and
Z² is a C₄-C₂₀ alkenyl, wherein the alkenyl group may optionally be substituted with one or two fluorine atoms at the alpha position to a double bond which is between the double bond and the terminus of Z² (e.g.,

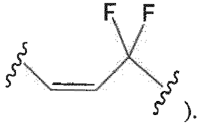

The R'R¹R²N-(R)ₐ-Q-(R)_{b}- group can be any of the head groups described herein, including those shown in Table 1 below, and salts thereof. In one preferred embodiment, R'R¹R²N-(R)ₐ-Q-(R)_{b}- is (CH₃)₂N-(CH₂)₂-, (CH₃)₂N-(CH₂)₃-C(O)O-, (CH₃)₂N-(CH₂)₂-NH-C(O)O-, (CH₃)₂N-(CH₂)₂-OC(O)-NH-, or (CH₃)₂N-(CH₂)₃-C(CH₃)=N-O-. In a preferred embodiment, R'R¹R²N-(R)ₐ-Q-(R)_{b}- is (CH₃)₂N-(CH₂)₂-.

In one embodiment, R¹ and R² are both alkyl (e.g., methyl, ethyl or a combination thereof). In one embodiment, R¹ and R² are both methyl. In another embodiment, one of R¹ and R² is methyl and the other of R¹ and R² is ethyl.

In a further embodiment, a is 2. In another embodiment, b is 0. In another embodiment Q is absent. In yet another embodiment, a is 2, b is 0 and Q is absent. In yet another embodiment, a is 4, b is 0 and Q is -O-.

In another embodiment, X is -(CH₂)ₙ- wherein n is 4 to 20, e.g., 4 to 18, 4 to 16, or 4 to 12. In one embodiment, n is 4, 5, 6, 7, 8, 9, or 10. In one embodiment, X is -(CH₂)₇₋₉-. In one exemplary embodiment, X is -(CH₂)₇-. In one exemplary embodiment, X is -(CHz)s-. In one exemplary embodiment, X is -(CH₂)₉-.

In further embodiments, M¹ is -OC(O)- or -C(O)O-. For example, in one embodiment, M¹ is -C(O)O-. In another embodiment, M¹ is -OC(O)-.

In another embodiment, Z¹ is a C₆-C₁₀ branched alkyl group, e.g.,-CH(CH₂CH₃)(CH₂CH₂CH₂CH₃), -CH₂CH(ⁱPr)(CH₂CH₂ⁱPr) or -CH₂CH(n-Bu)₂.

In another embodiment, Z² is a C₁₉ alkenyl having one or two double bonds. For example, Z₂ can be -(CH₂)₉CH=CHCH₂CH=CH(CH₂)₄CH₃.

Yet another embodiment is a cationic lipid selected from: 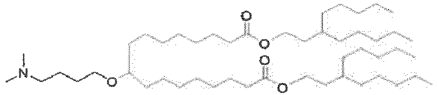 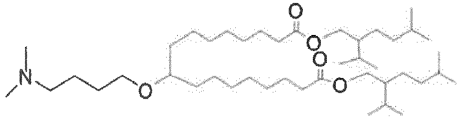 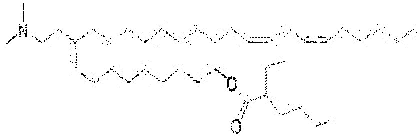 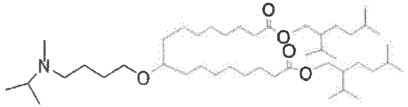 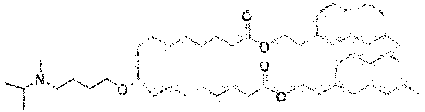 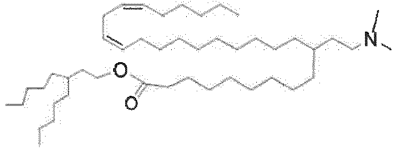 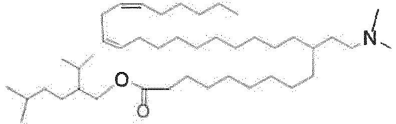 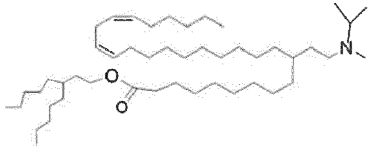 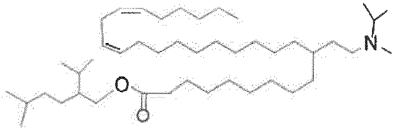 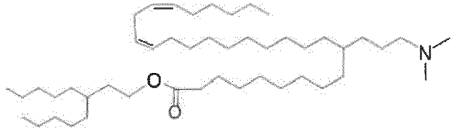 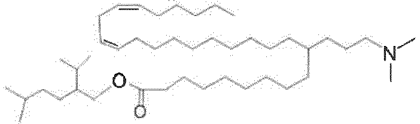 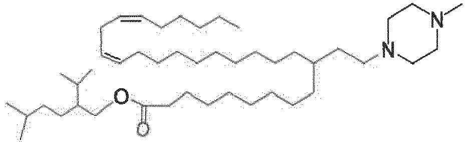 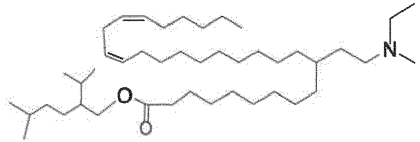 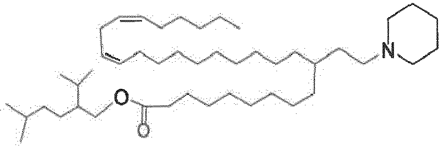 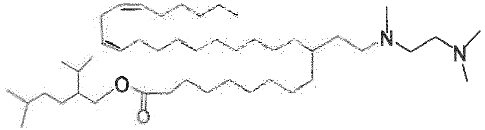 and salts thereof (e.g., pharmaceutically acceptable salts thereof).

In certain embodiments, the biodegradable group present in the cationic lipid is selected from an ester (e.g., -C(O)O- or -OC(O)-), disulfide (-S-S-), oxime (e.g., -C(H)=N-O- or -ON=C(H)-), -C(O)-O-, -OC(O)-, -C(R⁵)=N-, -N=C(R⁵)-, -C(R⁵)=N-O-, -O-N=C(R⁵)-, -O-C(O)O-, -C(O)N(R⁵), -N(R⁵)C(O)-, -C(S)(NR⁵)-, (NR⁵)C(S)-, -N(R⁵)C(O)N(R⁵)-, -C(O)S-, -SC(O)-, - C(S)O-, -OC(S)-, -OSi(R⁵)₂O-, -C(O)(CR³R⁴)C(O)O-, or -OC(O)(CR³R⁴)C(O)-.

In one embodiment, the aliphatic group in one or both of the hydrophobic tails of the cationic lipid includes at least one carbon-carbon double bond.

A suitable cholesterol moiety for the cationic lipids (including compounds of formulas (A), (A-I) and (A-II) has the formula:

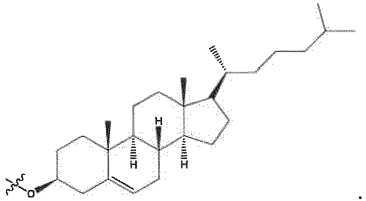

Additional embodiments include a cationic lipid having a head group, one or more hydrophobic tails, and a linker between the head group and the one or more tails. The head group can include an amine; for example, an amine having a desired pKₐ. The pKₐ can be influenced by the structure of the lipid, particularly the nature of head group; e.g., the presence, absence, and location of functional groups such as anionic functional groups, hydrogen bond donor functional groups, hydrogen bond acceptor groups, hydrophobic groups (e.g., aliphatic groups), hydrophilic groups (e.g., hydroxyl or methoxy), or aryl groups. The head group amine can be a cationic amine; a primary, secondary, or tertiary amine; the head group can include one amine group (monoamine), two amine groups (diamine), three amine groups (triamine), or a larger number of amine groups, as in an oligoamine or polyamine. The head group can include a functional group that is less strongly basic than an amine, such as, for example, an imidazole, a pyridine, or a guanidinium group. The head group can be zwitterionic. Other head groups are suitable as well.

The one or more hydrophobic tails can include two hydrophobic chains, which may be the same or different. The tails can be aliphatic, for example, they can be composed of carbon and hydrogen, either saturated or unsaturated but without aromatic rings. The tails can be fatty acid tails. Some such groups include octanyl, nonanyl, decyl, lauryl, myristyl, palmityl, stearyl, α-linoleyl, stearidonyl, linoleyl, γ-linolenyl, arachadonyl, and oleyl. Other hydrophobic tails are suitable as well.

The linker can include, for example, a glyceride linker, an acyclic glyceride analog linker, or a cyclic linker (including a spiro linker, a bicyclic linker, and a polycyclic linker). The linker can include functional groups such as an ether, an ester, a phosphate, a phosphonate, a phosphorothioate, a sulfonate, a disulfide, an acetal, a ketal, an imine, a hydrazone, or an oxime. Other linkers and functional groups are suitable as well.

In one embodiment, the cationic lipid is a racemic mixture. In another embodiment, the cationic lipid is enriched in one diastereomer, e.g. the cationic lipid has at least 95%, at least 90%, at least 80% or at least 70% diastereomeric excess. In yet another embodiment, the cationic lipid is enriched in one enantiomer, e.g. the lipid has at least 95%, at least 90%, at least 80% or at least 70% enantiomer excess. In yet another embodiment, the cationic lipid is chirally pure, e.g. is a single optical isomer. In yet another embodiment, the cationic lipid is enriched for one optical isomer.

Where a double bond is present (e.g., a carbon-carbon double bond or carbon-nitrogen double bond), there can be isomerism in the configuration about the double bond (i.e. cis/trans or E/Z isomerism). Where the configuration of a double bond is illustrated in a chemical structure, it is understood that the corresponding isomer can also be present. The amount of isomer present can vary, depending on the relative stabilities of the isomers and the energy required to convert between the isomers. Accordingly, some double bonds are, for practical purposes, present in only a single configuration, whereas others (e.g., where the relative stabilities are similar and the energy of conversion low) may be present as inseparable equilibrium mixture of configurations.

In some cases, a double-bonded unsaturation can be replaced by a cyclic unsaturation. The cyclic unsaturation can be a cycloaliphatic unsaturation, e.g., a cyclopropyl, cyclobutyl, cyclopentyl, cyclohexyl, cycloheptyl, or cyclooctyl group. In some cases, the cyclic group can be a polycyclic group, e.g., a bicyclic group or tricyclic group. A bicyclic group can be bridged, fused, or have a spiro structure.

In some cases, a double bond moiety can be replaced by a cyclopropyl moiety, e.g., 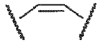 can be replaced by 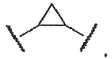 For example, the moiety shown below has two carbon-carbon double bonds, each of which can independently be replaced by a cyclic moiety, e.g., a cyclopropyl moiety. Thus, substitutes for: include: 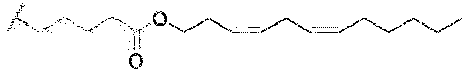 can 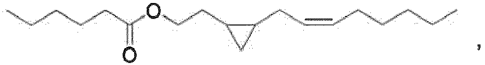 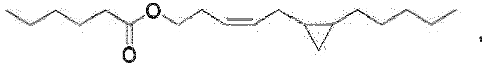 and

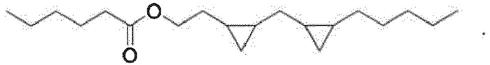

For further example, substitutes for 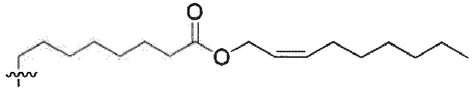 include:

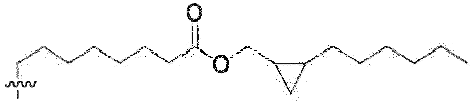

For further example, substitutes for 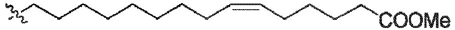 include:

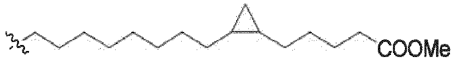

For further example, substitutes for 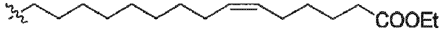 include:

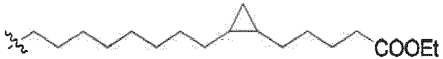

The cationic lipid includes one or more biodegradable groups. The biodegradable group(s) include one or more bonds that may undergo bond breaking reactions in a biological environment, e.g., in an organism, organ, tissue, cell, or organelle. Functional groups that contain a biodegradable bond include, for example, esters, dithiols, and oximes. Biodegradation can be a factor that influences the clearance of the compound from the body when administered to a subject. Biodegradation can be measured in a cell based assay, where a formulation including a cationic lipid is exposed to cells, and samples are taken at various time points. The lipid fractions can be extracted from the cells and separated and analyzed by LC-MS. From the LC-MS data, rates of biodegradation (e.g., as t_{1/2} values) can be measured.

For example, the compound 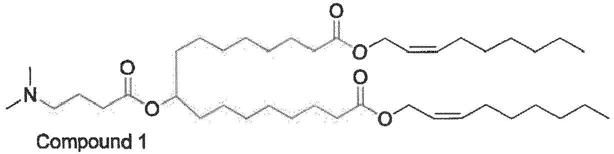 includes an ester linkage in each aliphatic chain, which can undergo hydrolysis in a biological environment, for example, when exposed to, e.g., a lipase or an esterase. The structure of the compound, of course, influences the rate at which the compound undergoes biodegradation. Thus, a related compound such as 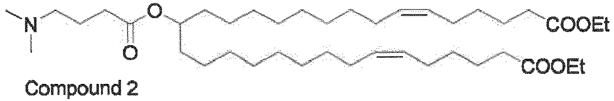 would be expected to exhibit a different rate of biodegradation. Greater effects on that rate would be expected from changes in the structure of the compound at the site of hydrolysis. One modification that can influence the rate of hydrolysis, and thereby influence the rate of biodegradation and clearance from a subject's body, is to make the leaving group of the hydrolysis reaction have a primary, rather than secondary, alcohol.

In one embodiment, a cationic lipid of any of the embodiments described herein has an *in vivo* half life (t_{1/2}) (e.g., in the liver, spleen or plasma) of less than about 3 hours, such as less than about 2.5 hours, less than about 2 hours, less than about 1.5 hours, less than about 1 hour, less than about 0.5 hour or less than about 0.25 hours. The cationic lipid preferably remains intact or has a half-life sufficient to form a stable lipid nanoparticle which effectively delivers the desired active pharmaceutical ingredient (e.g., a nucleic acid) to its target but thereafter rapidly degrades to minimize any side effects to the subject. For instance, in mice, the cationic lipid preferably has a t_{1/2} in the spleen of from about 1 to about 7 hours.

In another embodiment, a cationic lipid of any of the embodiments described herein containing a biodegradable group or groups has an *in vivo* half life (t_{1/2}) (e.g., in the liver, spleen or plasma) of less than about 10% (e.g., less than about 7.5%, less than about 5%, less than about 2.5%) of that for the same cationic lipid without the biodegrable group or groups.

Some cationic lipids can be conveniently represented as a hydrophobic group combined via a central moiety (such as a carbon atom) with a headgroup. By way of example, the compound: 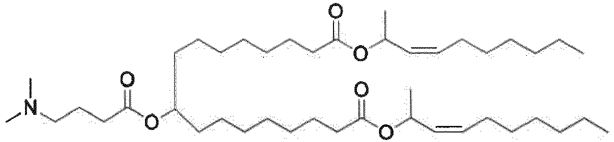 can be thought of as a combination of a headgroup, a central moiety, and two hydrophobic groups as follows:

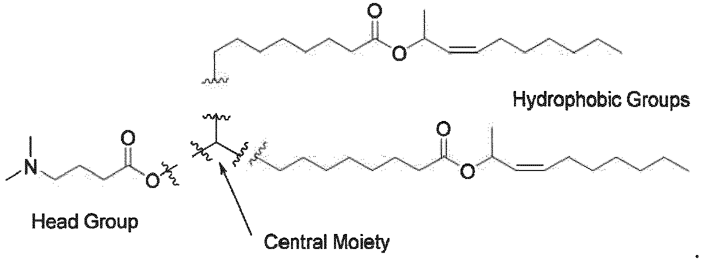

Suitable cationic lipids include compounds composed of any combination of the head and hydrophobic groups listed below (in combination with a central moiety (such as a central carbon atom).

Some suitable head groups include those depicted in Table 1A:

**TABLE 1A**

| | | |
|---|---|---|
| 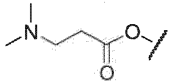 | 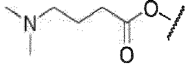 | 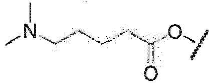 |
| 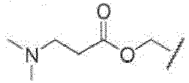 | 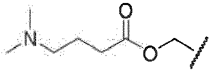 | 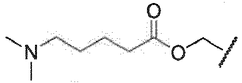 |
| 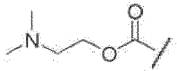 | 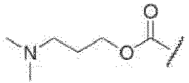 | 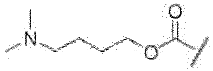 |
| 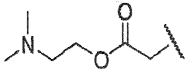 | 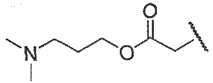 | 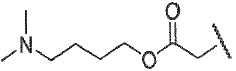 |
| 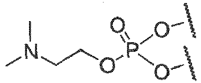 | 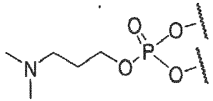 | 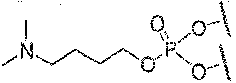 |
| 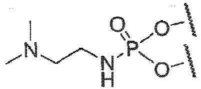 | 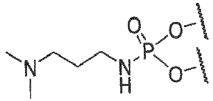 | 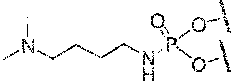 |
| 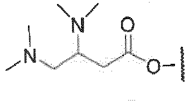 | 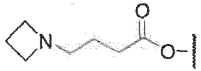 | 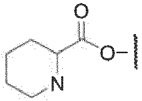 |
| 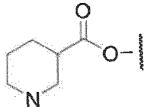 | 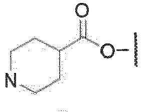 | 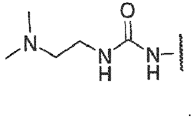 |
| 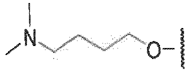 | 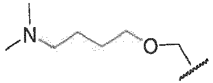 | 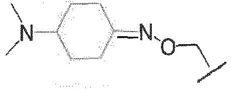 |
| 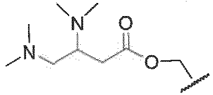 | 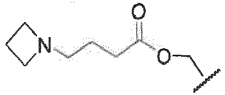 | 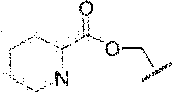 |
| 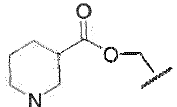 | 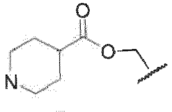 | 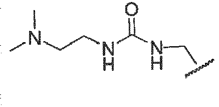 |
| 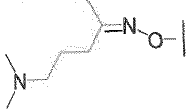 | 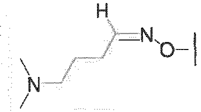 | 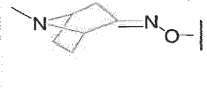 |
| 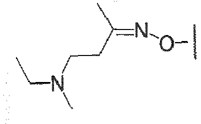 | 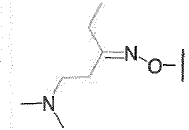 | 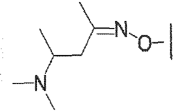 |
| 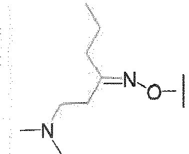 | 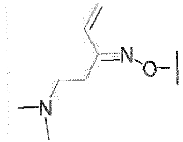 | 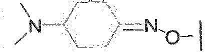 |
| 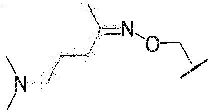 | 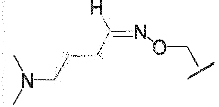 | 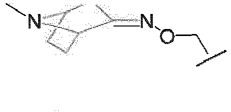 |
| 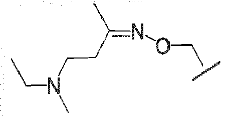 | 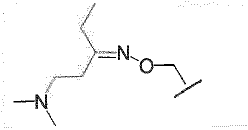 | 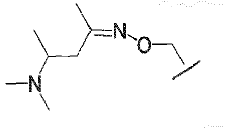 |
| 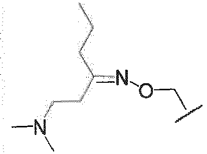 | 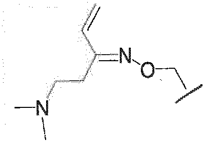 | 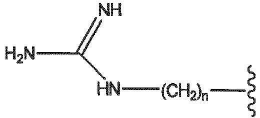 (where n is 0-5) |
| 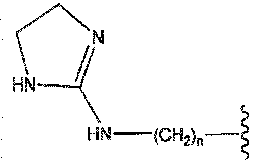 (where n is 0-5) | 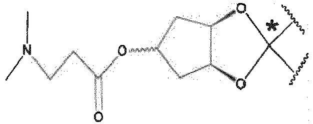 (the carbon with an asterisk is the tertiary carbon of the cation lipid and is not part of the head group) | 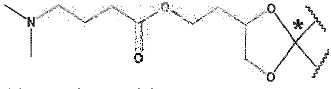 (the carbon with an asterisk is the tertiary carbon of the cation lipid and is not part of the head group) |
| 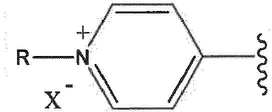 R = H, alkyl (e.g., methyl) | 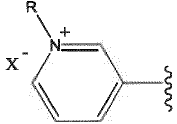 R = H, alkyl (e.g., methyl) | 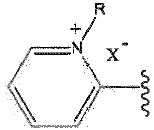 R = H, alkyl (e.g., methyl) |
| X = halogen (e.g., Cl) | X = halogen (e.g., Cl) | X = halogen (e.g., Cl) |
| 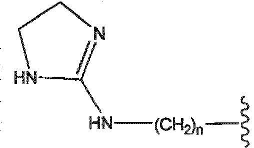 (where n is 0-5) | | |

Suitable primary groups include, but are not limited to, those that are a combination of a head group from table 1A with a central carbon atom. Other suitable primary groups include those in table 1B below:

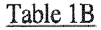

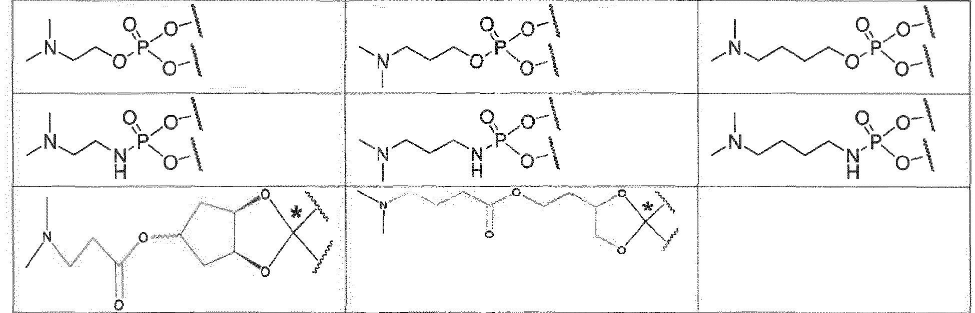

Some suitable hydrophobic tail groups include those depicted in Table 2:

**TABLE 2**

| | |
|---|---|
| 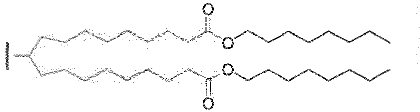 | 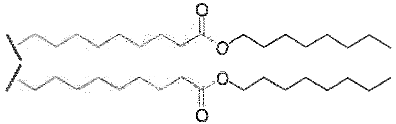 |
| 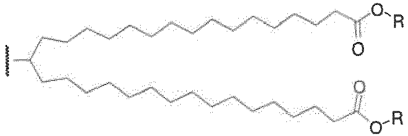 R = Me, Et | 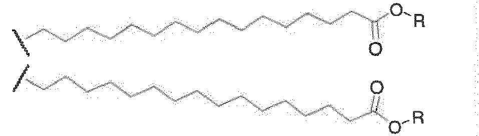 R = Me, Et |
| 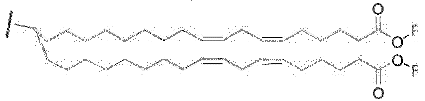 R = Me, Et | 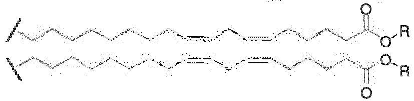 R = Me, Et |
| 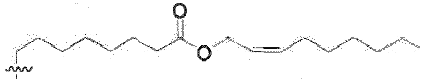 | 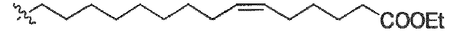 |
| 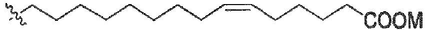 | |
| 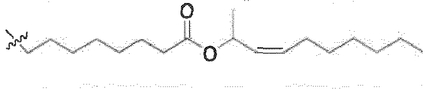 | 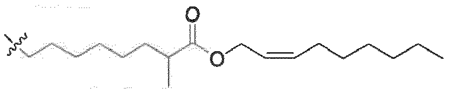 |
| 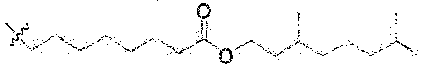 | 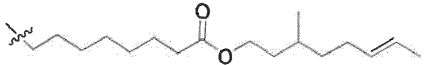 |
| 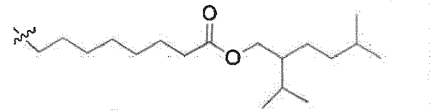 | 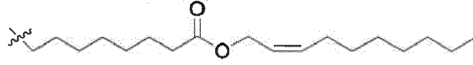 |
| 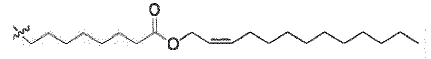 | 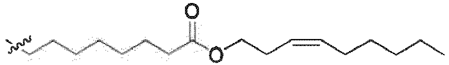 |
| 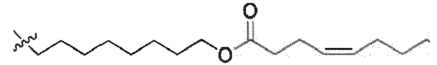 | 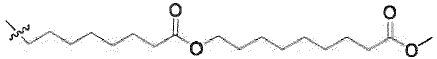 |
| 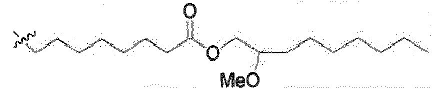 | 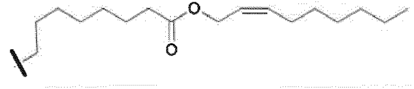 |
| 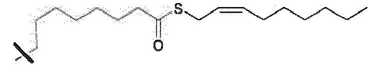 | 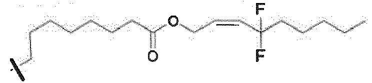 |
| 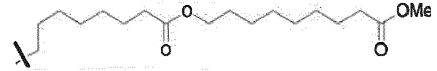 | 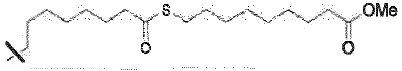 |
| 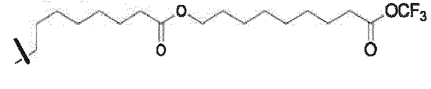 | 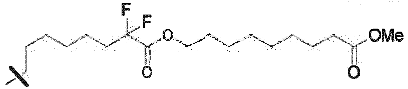 |
| 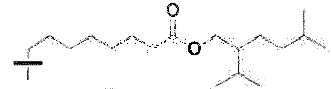 | 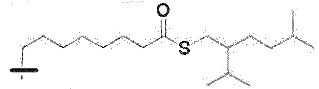 |
| 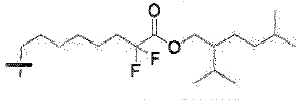 | 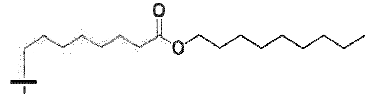 |
| 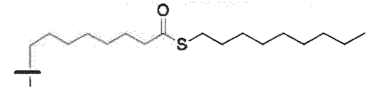 | 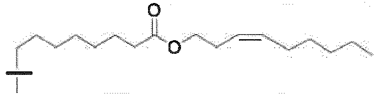 |
| 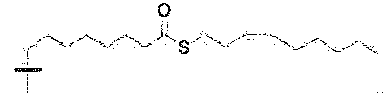 | 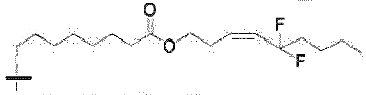 |
| 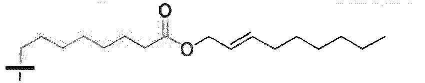 | 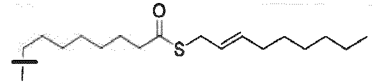 |
| 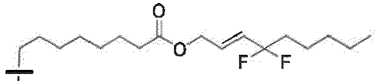 | 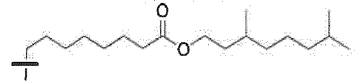 |
| 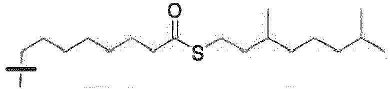 | 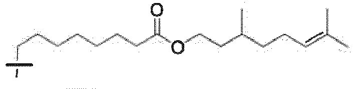 |
| 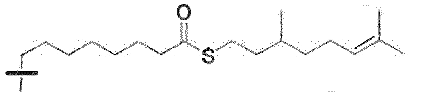 | 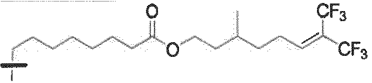 |
| 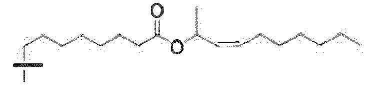 | 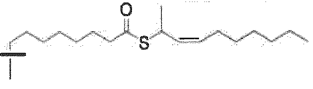 |
| 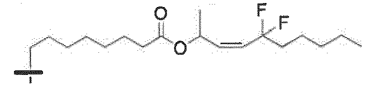 | 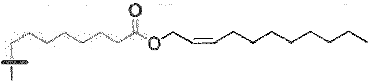 |
| 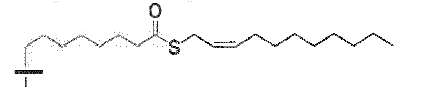 | 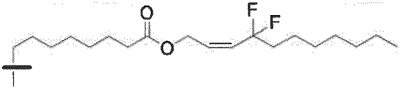 |
| 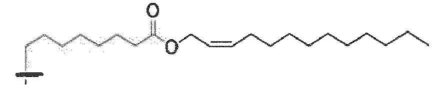 | 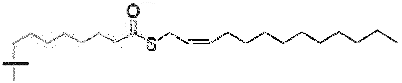 |
| 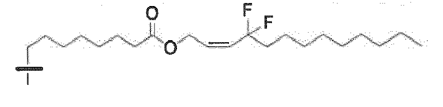 | 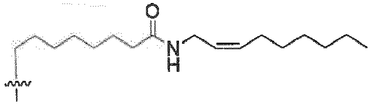 |
| 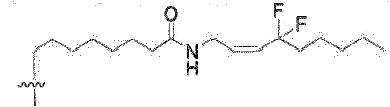 | 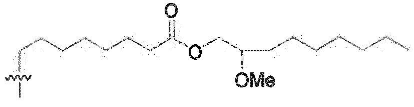 |
| 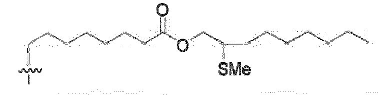 | 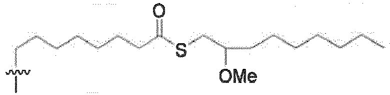 |
| 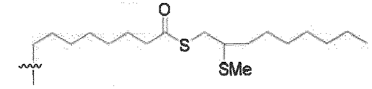 | 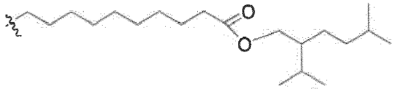 |
| 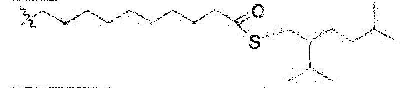 | 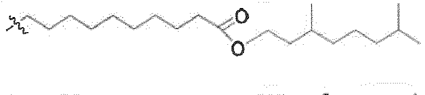 |
| 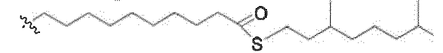 | 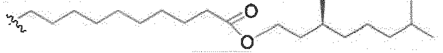 |
| 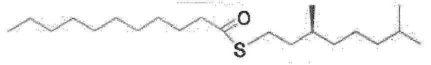 | 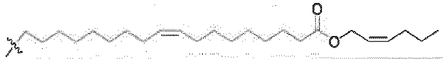 |
| 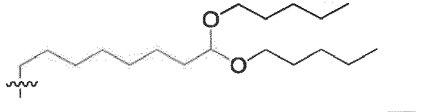 | 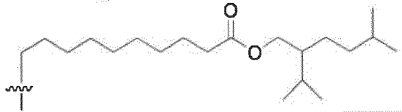 |

Cationic lipids include those having alternative fatty acid groups and other dialkylamino groups, including those in which the alkyl substituents are different (*e.g.,* N-ethyl-N-methylamino-, N-propyl-N-ethylamino- and the like). For those embodiments in which R¹ and R² are both long chain alkyl, alkenyl, alkynyl, or cycloalkylalkyl groups, they can be the same or different. In general, lipids (e.g., a cationic lipid) having less-saturated acyl chains are more easily sized, particularly when the complexes are sized below about 0.3 microns, for purposes of filter sterilization. Cationic lipids containing unsaturated fatty acids with carbon chain lengths in the range of C₁₀ to C₂₀ are typical. Other scaffolds can also be used to separate the amino group (e.g., the amino group of the cationic lipid) and the fatty acid or fatty alkyl portion of the cationic lipid. Suitable scaffolds are known to those of skill in the art.

In certain embodiments, cationic lipids have at least one protonatable or deprotonatable group, such that the lipid is positively charged at a pH at or below physiological pH (e.g. pH 7.4), and neutral at a second pH, preferably at or above physiological pH. Such lipids are also referred to as cationic lipids. It will, of course, be understood that the addition or removal of protons as a function of pH is an equilibrium process, and that the reference to a charged or a neutral lipid refers to the nature of the predominant species and does not require that all of the lipid be present in the charged or neutral form. The lipids can have more than one protonatable or deprotonatable group, or can be zwiterrionic.

In certain embodiments, protonatable lipids (i.e., cationic lipids) have a pKₐ of the protonatable group in the range of about 4 to about 11. Typically, lipids will have a pKₐ of about 4 to about 7, e.g., between about 5 and 7, such as between about 5.5 and 6.8, when incorporated into lipid particles. Such lipids will be cationic at a lower pH formulation stage, while particles will be largely (though not completely) surface neutralized at physiological pH around pH 7.4. One of the benefits of a pKₐ in the range of between about 4 and 7 is that at least some nucleic acid associated with the outside surface of the particle will lose its electrostatic interaction at physiological pH and be removed by simple dialysis; thus greatly reducing the particle's susceptibility to clearance. pKₐ measurements of lipids within lipid particles can be performed, for example, by using the fluorescent probe 2-(p-toluidino)-6-napthalene sulfonic acid (TNS), using methods described in Cullis et al., (1986) Chem Phys Lipids 40, 127-144.

In particular embodiments, the lipids are charged lipids. As used herein, the term "charged lipid" is meant to include those lipids having one or two fatty acyl or fatty alkyl chains and a quaternary amino head group. The quaternary amine carries a permanent positive charge. The head group can optionally include a ionizable group, such as a primary, secondary, or tertiary amine that may be protonated at physiological pH. The presence of the quaternary amine can alter the pKa of the ionizable group relative to the pKa of the group in a structurally similar compound that lacks the quaternary amine (e.g., the quaternary amine is replaced by a tertiary amine) In some embodiments, a charged lipid is referred to as an "amino lipid." *See,* for example, provisional U.S. patent application 61/267,419, filed December 7, 2009.

In one embodiment, the cationic lipid is a compound of formula (I), which has a branched alkyl at the alpha position adjacent to the biodegradable group (between the biodegradable group and the teriary carbon): 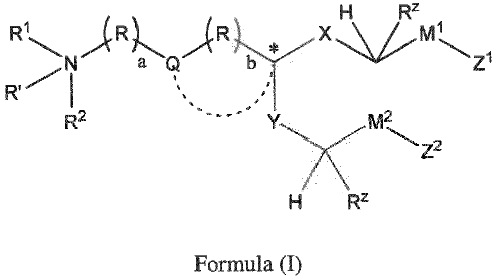 or a salt thereof (e.g., a pharmaceutically acceptable salt thereof), wherein
R' is absent, hydrogen, or alkyl (e.g., C₁-C₄ alkyl);
with respect to R¹ and R²,
   (i) R¹ and R² are each, independently, optionally substituted alkyl, alkenyl, alkynyl, cycloalkylalkyl, heterocycle, or R¹⁰;
   (ii) R¹ and R², together with the nitrogen atom to which they are attached, form an optionally substituted heterocylic ring; or
   (iii) one of R¹ and R² is optionally substituted alkyl, alkenyl, alkynyl, cycloalkyl, cycloalkylalkyl, or heterocycle, and the other forms a 4-10 member heterocyclic ring or heteroaryl (e.g., a 6-member ring) with (a) the adjacent nitrogen atom and (b) the (R)ₐ group adjacent to the nitrogen atom;
each occurrence of R is, independently, -(CR³R⁴)-;
each occurrence of R³ and R⁴ are, independently H, halogen, OH, alkyl, alkoxy, -NH₂, R¹⁰, alkylamino, or dialkylamino (in one preferred embodiment, each occurrence of R³ and R⁴ are, independently H or C₁-C₄ alkyl);
each occurrence of R¹⁰ is independently selected from PEG and polymers based on poly(oxazoline), poly(ethylene oxide), poly(vinyl alcohol), poly(glycerol), poly(N-vinylpyrrolidone), poly[N-(2-hydroxypropyl)methacrylamide] and poly(amino acid)s, wherein (i) the PEG or polymer is linear or branched, (ii) the PEG or polymer is polymerized by n subunits, (iii) n is a number-averaged degree of polymerization between 10 and 200 units, and (iv) wherein the compound of formula has at most two R¹⁰ groups (preferably at most one R¹⁰ group);
the dashed line to Q is absent or a bond;
when the dashed line to Q is absent then Q is absent or is -O-, -NH-, -S-, -C(O)-, -C(O)O-, -OC(O)-, -C(O)N(R⁴)-, -N(R⁵)C(O)-, -S-S-, -OC(O)O-, -O-N=C(R⁵)-, -C(R⁵)=N-O-,-OC(O)N(R⁵)-, -N(R⁵)C(O)N(R⁵)-, -N(R⁵)C(O)O-, -C(O)S-, -C(S)O- or -C(R⁵)=N-O-C(O)-; or
when the dashed line to Q is a bond then (i) b is 0 and (ii) Q and the tertiary carbon adjacent to it (C*) form a substituted or unsubstituted, mono- or bi-cyclic heterocyclic group having from 5 to 10 ring atoms (e.g., the heteroatoms in the heterocyclic group are selected from O and S, preferably O);
each occurrence of R⁵ is, independently, H or alkyl (e.g. C₁-C₄ alkyl);
X and Y are each, independently, alkylene or alkenylene (e.g., C₄ to C₂₀ alkylene or C₄ to C₂₀ alkenylene);
M¹ and M² are each, independently, a biodegradable group (e.g., -OC(O)-, -C(O)O-, - SC(O)-, -C(O)S-, -OC(S)-, -C(S)O-, -S-S-, -C(R⁵)=N-, -N=C(R⁵)-, -C(R⁵)=N-O-, -O-N=C(R⁵)-, - C(O)(NR⁵)-, -N(R⁵)C(O)-, -C(S)(NR⁵)-, -N(R^{S})C(O)-, -N(R⁵)C(O)N(R⁵)-, -OC(O)O-, - OSi(R⁵)₂O-, -C(O)(CR³R⁴)C(O)O-, -OC(O)(CR³R⁴)C(O)-, or 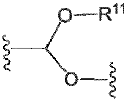 (wherein R¹¹ is a C₂-C₈ alkyl or alkenyl));
each occurrence of R^{z} is, independently, C₁-C₈ alkyl (e.g., methyl, ethyl, isopropyl, n-butyl, n-pentyl, or n-hexyl);
a is 1, 2, 3, 4, 5 or 6;
b is 0, 1, 2, or 3; and
Z¹ and Z² are each, independently, C₈-C₁₄ alkyl or C₈-C₁₄ alkenyl, wherein the alkenyl group may optionally be substituted with one or two fluorine atoms at the alpha position to a double bond which is between the double bond and the terminus of Z¹ or Z² (e.g.,

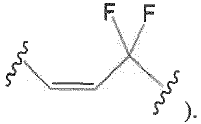

The R'R¹R²N-(R)ₐ-Q-(R)_{b}- group can be any of the head groups described herein, including those shown in Table 1 below, and salts thereof. In one preferred embodiment, R'R¹R²N-(R)ₐ-Q-(R)_{b}- is (CH₃)₂N-(CH₂)₃-C(O)O-, (CH₃)₂N-(CH₂)₂-NH-C(O)O-, (CH₃)₂N-(CH₂)₂-OC(O)-NH-, or (CH₃)₂N-(CH₂)₃-C(CH₃)=N-O-.

In one embodiment, R¹ and R² are both alkyl (e.g., methyl).

In a further embodiment, a is 3. In another embodiment, b is 0.

In a further embodiment, a is 3, b is 0 and R is -CH₂-. In yet a further embodiment, a is 3, b is 0, R is -CH₂- and Q is -C(O)O-. In another embodiment, R¹ and R² are methyl, a is 3, b is 0, R is -CH₂- and Q is -C(O)O-.

In another embodiment, X and Y are each, independently -(CH₂)ₙ- wherein n is 4 to 20, e.g., 4 to 18, 4 to 16, or 4 to 12. In one embodiment, n is 4, 5, 6, 7, 8, 9, or 10. In one exemplary embodiment, X and Y are -(CH₂)₆-. In another embodiment, X and Y are -(CH₂)₇-. In yet another embodiment, X and Y are -(CH₂)₉-. In yet another embodiment, X and Y are -(CH₂)₈-.

In further embodiments, M¹ and M² are each, independently, -OC(O)- or -C(O)O-. For example, in one embodiment, M¹ and M² are each -C(O)O-.

In one embodiment, the cationic lipid is a compound of formula (V), which has an alkoxy or thioalkoxy (i.e., -S-alkyl) group substitution on at least one tail: 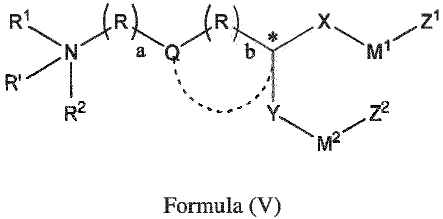 or a salt thereof (e.g., a pharmaceutically acceptable salt thereof), wherein
R', R¹, R², R, R³, R⁴, R¹⁰, Q, R⁵, M¹, M², a, and b are defined as in formula (I);
X and Y are each, independently, alkylene (e.g., C₆-C₈ alkylene) or alkenylene, wherein the alkylene or alkenylene group is optionally substituted with one or two fluorine atoms at the alpha position to the M¹ or M² group (e.g., 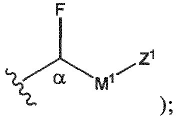
Z¹ and Z² are each, independently, C₈-C₁₄ alkyl or C₈-C₁₄ alkenyl, wherein (i) the C₈-C₁₄ alkyl or C₈-C₁₄ alkenyl of at least one of Z¹ and Z² is substituted by one or more alkoxy (e.g., a C₁-C₄ alkoxy such as -OCH₃) or thioalkoxy (e.g., a C₁-C₄ thioalkoxy such as -SCH₃) groups, and (ii) the alkenyl group may optionally be substituted with one or two fluorine atoms at the alpha position to a double bond which is between the double bond and the terminus of Z¹ or Z² (e.g.,

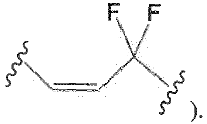

In one embodiment, the alkoxy substitution on Z¹ and/or Z² is at the beta position from the M¹ and/or M² group.

In another embodiment, X and Y are each, independently -(CH₂)ₙ- wherein n is 4 to 20, e.g., 4 to 18, 4 to 16, or 4 to 12. In one embodiment, n is 4, 5, 6, 7, 8, 9, or 10. In one exemplary embodiment, X and Y are -(CH₂)₆-. In another embodiment, X and Y are -(CH₂)₇-. In yet another embodiment, X and Y are -(CH₂)₉-. In yet another embodiment, X and Y are -(CHz)s-.

The R'R¹R²N-(R)ₐ-Q-(R)_{b}- group can be any of the head groups described herein, including those shown in Table 1 below, and salts thereof. In one preferred embodiment, R'R¹R²N-(R)ₐ-Q-(R)_{b}- is (CH₃)₂N-(CH₂)₃-C(O)O-, (CH₃)₂N-(CH₂)₂-NH-C(O)O-, (CH₃)₂N-(CH₂)₂-OC(O)-NH-, or (CH₃)₂N-(CH₂)₃-C(CH₃)=N-O-.

In one embodiment, the cationic lipid is a compound of formula (VIA), which has one or more fluoro substituents on at least one tail at a position that is either alpha to a double bond or alpha to a biodegradable group: 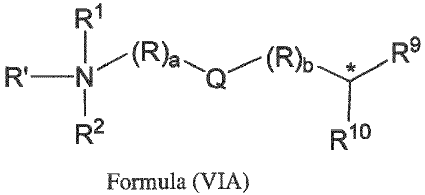 or a salt thereof (e.g., a pharmaceutically acceptable salt thereof), wherein
R¹, R², R, a, and b are as defined with respect to formula (I);
Q is absent or is -O-, -NH-, -S-, -C(O)-, -C(O)O-, -OC(O)-, -C(O)N(R⁴)-, -N(R⁵)C(O)-, - S-S-, -OC(O)O-, -O-N=C(R⁵)-, -C(R⁵)=N-O-, -OC(O)N(R⁵)-, -N(R⁵)C(O)N(R⁵)-, -N(R⁵)C(O)O-, -C(O)S-, -C(S)O- or -C(R⁵)=N-O-C(O)-;
R' is absent, hydrogen, or alkyl (e.g., C₁-C₄ alkyl); and
each of R⁹ and R¹⁰ are independently C₁₂-C₂₄ alkyl (e.g., C₁₂-C₂₀ alkyl), C₁₂-C₂₄ alkenyl (e.g., C₁₂-C₂₀ alkenyl), or C₁₂-C₂₄ alkoxy (e.g., C₁₂-C₂₀ alkoxy) (a) having one or more biodegradable groups and (b) optionally substituted with one or more fluorine atoms at a position which is (i) alpha to a biodegradable group and between the biodegradable group and the tertiary carbon atom marked with an asterisk (*), or (ii) alpha to a carbon-carbon double bond and between the double bond and the terminus of the R⁹ or R¹⁰ group; each biodegradable group independently interrupts the C₁₂-C₂₄ alkyl, alkenyl, or alkoxy group or is substituted at the terminus of the C₁₂-C₂₄ alkyl, alkenyl, or alkoxy group,
wherein
(i) at least one of R⁹ and R¹⁰ contains a fluoro group;
(ii) the compound does not contain the following moiety: 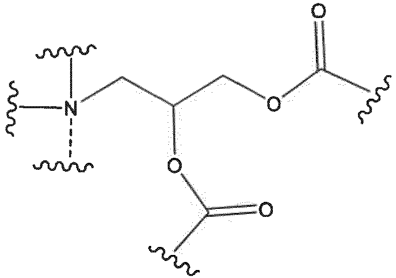 wherein ---- is an optional bond; and
(iii) the terminus of R⁹ and R¹⁰ is separated from the tertiary carbon atom marked with an asterisk (*) by a chain of 8 or more atoms (e.g., 12 or 14 or more atoms).

In one preferred embodiment, the terminus of R⁹ and R¹⁰ is separated from the tertiary carbon atom marked with an asterisk (*) by a chain of 18-22 carbon atoms (e.g., 18-20 carbon atoms).

In another embodiment, the terminus of the R⁹ and/or R¹⁰ has the formula -C(O)O-CF₃.

In another embodiment, the cationic lipid is a compound of formula (VIB), which has one or more fluoro substituents on at least one tail at a position that is either alpha to a double bond or alpha to a biodegradable group: 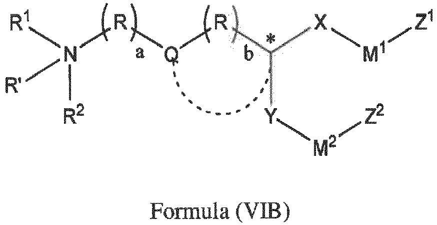 or a salt thereof (e.g., a pharmaceutically acceptable salt thereof), wherein
R', R¹, R², R, R³, R⁴, R¹⁰, Q, R⁵, M¹, M², a, and b are defined as in formula (I);
X and Y are each, independently, alkylene (e.g., C₆-C₈ alkylene) or alkenylene, wherein the alkylene or alkenylene group is optionally substituted with one or two fluorine atoms at the alpha position to the M¹ or M² group (e.g., 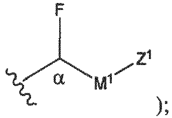 and
Z¹ and Z² are each, independently, C₈-C₁₄ alkyl or C₈-C₁₄ alkenyl, wherein said C₈-C₁₄ alkenyl is optionally substituted by one or more fluorine atoms at a position that is alpha to a double bond (e.g., 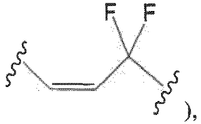
wherein at least one of X, Y, Z¹, and Z² contains a fluorine atom.

In one embodiment, at least one of Z¹ and Z² is substituted by two fluoro groups at a position that is either alpha to a double bond or alpha to a biodegradable group. In one embodiment, at least one of Z¹ and Z² has a terminal -CF₃ group at a position that is alpha to a biodegradable group (i.e., at least one of Z¹ and Z² terminates with an -C(O)OCF₃ group).

For example, at least one of Z¹ and Z² may include one or more of the following moieties:

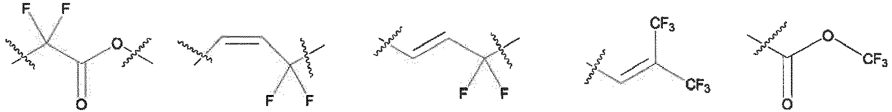

In one embodiment, X and Y are each, independently -(CH₂)ₙ- wherein n is 4 to 20, e.g., 4 to 18, 4 to 16, or 4 to 12. In one embodiment, n is 4, 5, 6, 7, 8, 9, or 10. In one exemplary embodiment, X and Y are -(CH₂)₇-. In another exemplary embodiment, X and Y are -(CH₂)₉-. In yet another embodiment, X and Y are -(CH₂)₈-.

The R'R¹R²N-(R)ₐ-Q-(R)_{b}- group can be any of the head groups described herein, including those shown in Table 1 below, and salts thereof. In one preferred embodiment, R'R¹R²N-(R)ₐ-Q-(R)_{b}- is (CH₃)₂N-(CH₂)₃-C(O)O-, (CH₃)₂N-(CH₂)₂-NH-C(O)O-, (CH₃)₂N-(CH₂)₂-OC(O)-NH-, or (CH₃)₂N-(CH₂)₃-C(CH₃)=N-O-.

In one embodiment, the cationic lipid is a compound of formula (VII), which has an acetal group as a biodegradable group in at least one tail: 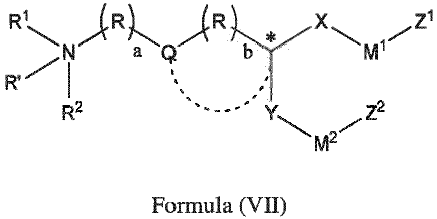 or a salt thereof (e.g., a pharmaceutically acceptable salt thereof), wherein
R', R¹, R², R, R³, R⁴_{,} R¹⁰, Q, R⁵, a, and b are defined as in formula (I);
X and Y are each, independently, alkylene (e.g., C₆-C₈ alkylene) or alkenylene, wherein the alkylene or alkenylene group is optionally substituted with one or two fluorine atoms at the alpha position to the M¹ or M² group (e.g., 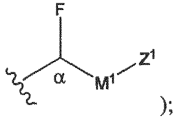
M¹ and M² are each, independently, a biodegradable group (e.g., -OC(O)-, -C(O)O-,-SC(O)-, -C(O)S-, -OC(S)-, -C(S)O-, -S-S-, -C(R⁵)=N-, -N=C(R⁵)-, -C(R⁵)=N-O-, -O-N=C(R⁵)-,-C(O)(NR⁵)-, -N(R⁵)C(O)-, -C(S)(NR⁵)-, -N(R⁵)C(O)-, -N(R⁵)C(O)N(R⁵)-, -OC(O)O-,-OSi(R⁵)₂O-, -C(O)(CR³R⁴)C(O)O-, -OC(O)(CR³R⁴)C(O)-, or 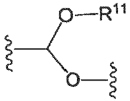 (wherein R¹¹ is a C₄-C₁₀ alkyl or C₄-C₁₀ alkenyl));
with the proviso that at least one of M¹ and M² is 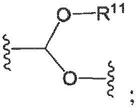 and
Z¹ and Z² are each, independently, C₄-C₁₄ alkyl or C₄-C₁₄ alkenyl, wherein the alkenyl group may optionally be substituted with one or two fluorine atoms at the alpha position to a double bond which is between the double bond and the terminus of Z¹ or Z² (e.g.,

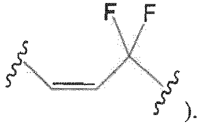

In one embodiment, each of M¹ and M² is

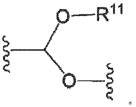

In another embodiment, X and Y are each, independently -(CH₂)ₙ- wherein n is 4 to 20, e.g., 4 to 18, 4 to 16, or 4 to 12. In one embodiment, n is 4, 5, 6, 7, 8, 9, or 10. In one exemplary embodiment, X and Y are -(CH₂)₆-. In another embodiment, X and Y are -(CH₂)₇-. In yet another embodiment, X and Y are -(CH₂)₉-. In yet another embodiment, X and Y are -(CH₂)₈-.

The R'R¹R²N-(R)ₐ-Q-(R)_{b}- group can be any of the head groups described herein, including those shown in Table 1 below, and salts thereof. In one preferred embodiment, R'R¹R²N-(R)ₐ-Q-(R)_{b}- is (CH₃)₂N-(CH₂)₃-C(O)O-, (CH₃)₂N-(CH₂)₂-NH-C(O)O-, (CH₃)₂N-(CH₂)₂-OC(O)-NH-, or (CH₃)₂N-(CH₂)₃-C(CH₃)=N-O-.

In another embodiment, the cationic lipid or a salt thereof has:
(i) a central carbon atom,
(ii) a nitrogen containing head group directly bound to the central carbon atom, and
(iii) two hydrophobic tails directly bound to the central carbon atom, wherein each hydrophobic tail is of the formula -R^{e}-M-R^{f} where R^{e} is a C₄-C₁₄ alkyl or alkenyl, M is a biodegradable group, and R^{f} is a branched alkyl or alkenyl (e.g., a C₁₀-C₂₀ alkyl or C₁₀-C₂₀ alkenyl), such that (i) the chain length of -R^{e}-M-R^{f} is at most 20 atoms (i.e. the total length of the tail from the first carbon atom after the central carbon atom to a terminus of the tail is at most 20), and (ii) the group -R^{e}-M-R^{f} has at least 20 carbon atoms (e.g., at least 21 atoms). Optionally, the alkyl or alkenyl group in R^{e} may be substituted with one or two fluorine atoms at the alpha position to the M¹ or M² group (e.g., 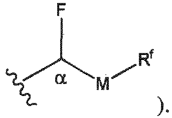 Also, optionally, the alkenyl group in R^{f} may be substituted with one or two fluorine atoms at the alpha position to a double bond which is between the double bond and the terminus of R^{f} (e.g.,

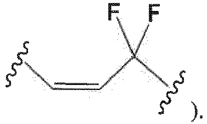

In one embodiment, the cationic lipid (such as of formulas I-VII) has assymetrical hydrophobic groups (i.e., the two hydrophobic groups have different chemical formulas). For example, the cationic lipid can have the formula: 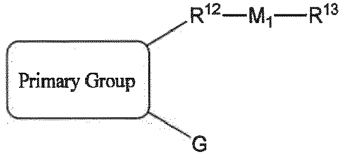 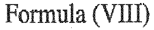 or a salt thereof (e.g., a pharmaceutically acceptable salt thereof), wherein
G is branched or unbranched C₃-C₁₅ alkyl, alkenyl or alkynyl (e.g., a n-C₈ alkyl n-C₉ alkyl, or n-C₁₀ alkyl);
R¹² is a branched or unbranched alkylene or alkenylene (e.g., C₆-C₂₀ alkylene or C₆-C₂₀ alkenylene such as C₁₂-C₂₀ alkylene or C₁₂-C₂₀ alkenylene);
M₁ is a biodegradable group (e.g., -OC(O)-, -C(O)O-, -SC(O)-, -C(O)S-, -OC(S)-, -C(S)O-, -S-S-, -C(R⁵)=N-, -N=C(R⁵)-, -C(R⁵)=N-O-, -O-N=C(R⁵)-, -C(O)(NR⁵)-, -N(R⁵)C(O)-,-C(S)(NR⁵)-, -N(R⁵)C(O)-, -N(R⁵)C(O)N(R⁵)-, -OC(O)O-, -OSi(R⁵)₂O-, -C(O)(CR³R⁴)C(O)O-,-OC(O)(CR³R⁴)C(O)-, or 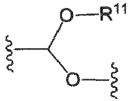 (wherein R¹¹ is a C₂-C₈ alkyl or alkenyl));
R³ and R⁴ are defined as in formula (I);
each occurrence of R⁵ is, independently, H or alkyl (e.g., C₁-C₄ alkyl);
R¹³ is branched or unbranched C₃-C₁₅ alkyl, alkenyl or alkynyl; and 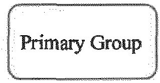 comprises a protonatable group having a pKₐ of from about 4 to about 13, more preferably from about 5 to about 8 (e.g. from about 5 to about 7, or from about 5 to about 6.5, or from about 5.5 to about 6.5, or from about 6 to about 6.5).

In one embodiment, the primary group includes (i) a head group, and (ii) a central moiety (e.g., a central carbon atom) to which both the hydrophobic tails are directly bonded. Representative central moieties include, but are not limited to, a central carbon atom, a central nitrogen atom, a central carbocyclic group, a central aryl group, a central hetrocyclic group (e.g., central tetrahydrofuranyl group or central pyrrolidinyl group) and a central heteroaryl group.

Representative 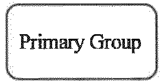 's include, but are not limited to, 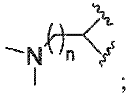 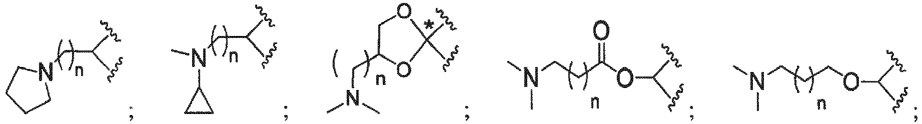 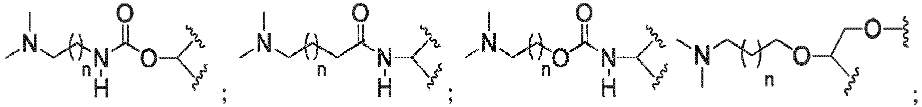 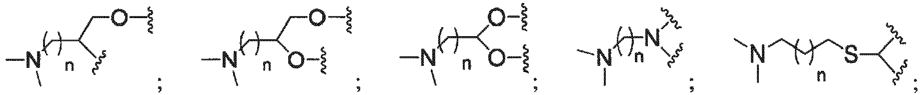 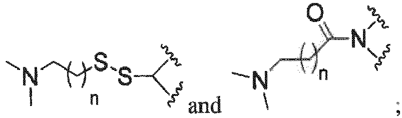 where n is 0-6.

Representative asymmetrical cationic lipids include: 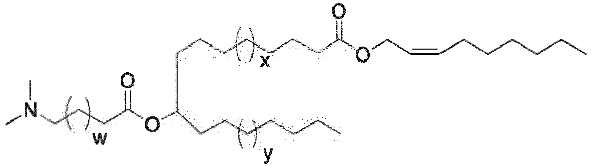 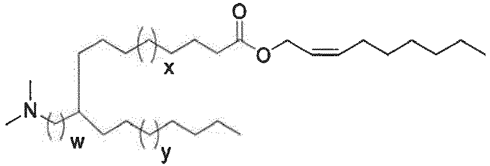 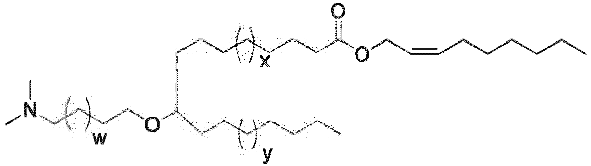 wherein w is 0, 1, 2, or 3; and x and y are each independently 1, 2, 3, 4, 5, 6, or 7.

In one embodiment each R is, independently, -(CR³R⁴)-, wherein R³ and R⁴ are each, independently, H or alkyl (e.g., C₁-C₄ alkyl). For example, in one embodiment each R is, independently, -(CHR⁴)-, wherein each R⁴ is, independently H or alkyl (e.g., C₁-C₄ alkyl). In another embodiment, each R is, independently, -CH₂-, -C(CH₃)₂- or -CH(*i*Pr)- (where *i*Pr is isopropyl). In another embodiment, each R is -CH₂-.

In another embodiment R⁵ is, in each case, hydrogen or methyl. For example, R⁵ can be, in each case, hydrogen.

In one embodiment, Q is absent, -C(O)O-, -OC(O)-, -C(O)N(R⁵)-, -N(R⁵)C(O)-, -S-S-,-OC(O)O-, -C(R⁵)=N-O-, -OC(O)N(R⁵)-, -N(R⁵)C(O)N(R⁵)-, -N(R⁵)C(O)O-, -C(O)S-, -C(S)O- or -C(R⁵)=N-O-C(O)-. In one embodiment, Q is -C(O)O-.

In one embodiment, the dashed line to Q is absent, b is 0 and R'R¹R²N-(R)ₐ-Q- and the tertiary carbon adjacent to it (C*) form the following group: 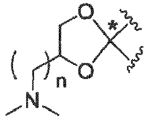 where n is 1 to 4 (e.g., n is 2).

In one embodiment, the dashed line to Q is absent, b is 0 and R'R¹R²N-(R)ₐ-Q- and the tertiary carbon adjacent to it form the following group: 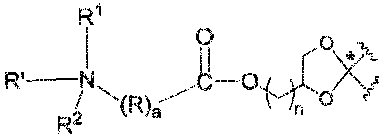 where n is 1 to 4 (e.g., n is 2), and R¹, R², R, a, and b are as defined with respect to formula (I). In one embodiment, a is 3.

In one embodiment, the dashed line to Q is absent, b is 0 and R'R¹R²N-(R)ₐ-Q- and the tertiary carbon adjacent to it form the following group: 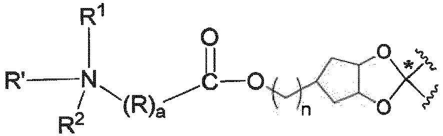 where n is 1 to 4 (e.g., n is 2), and R¹, R², R, a, and b are as defined with respect to formula (I). In one embodiment, a is 0. For example, the group can be:

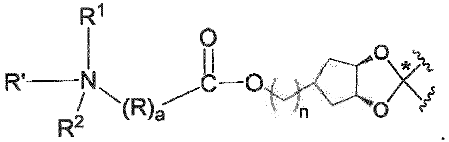

In one embodiment, b is 0. In another embodiment, a is 2, 3, or 4 and b is 0. For example, in one embodiment, a is 3 and b is 0. In another embodiment, a is 3, b is 0, and Q is -C(O)O-.

In certain embodiments, the biodegradable group present in the cationic lipid is selected from an ester (e.g., -C(O)O- or -OC(O)-), disulfide (-S-S-), oxime (e.g., -C(H)=N-O- or -O-N=C(H)-), -C(O)-O-, -OC(O)-, -C(R⁵)=N-, -N=C(R⁵)-, -C(R⁵)=N-O-, -O-N=C(R⁵)-, -O-C(O)O-, -C(O)N(R⁵), -N(R⁵)C(O)-, -C(S)(NR⁵)-, (NR⁵)C(S)-, -N(R⁵)C(O)N(R⁵)-, -C(O)S-, -SC(O)-,-C(S)O-,-OC(S)-, -OSi(R⁵)₂O-, -C(O)(CR³R⁴)C(O)O-, or -OC(O)(CR³R⁴)C(O)-.

In a preferred embodiment of the aforementioned biodegradable cationic lipids, the biodegradable cationic lipid has a logP value of at least 10.1 (as calculated by the software available at http://www.molinspiration.com/services/logp.html from Molinspiration Cheminformatics of Slovensky Grob, Slovak Republic). More preferably, the logP value is at least 10.2 or 10.3.

In another preferred embodiment of the aforementioned biodegradable cationic lipids, the biodegradable cationic lipid in the lipid nanoparticle has a HPLC retention time (relative to the retention time of cholesterol in the lipid nanoparticle), hereafter referred to as t_{lipid} - t_{chol}, of at least 1.4. (The HPLC parameters are provided in the examples below. Unless otherwise specified, the formulation of the lipid nanoparticle used is that described in Example 31). More preferably, the t_{lipid} - t_{chol} value is at least 1.75, 2.0, or 2.25.

Yet another embodiment is a biodegradable cationic lipid having (i) a logP value of at least 10.1 and/or a t_{lipid} - t_{chol}, of at least 1.4, and (ii) one or more biodegradable groups (such as an ester group) located in the mid- or distal section of a lipidic moiety (e.g., a hydrophobic chain) of the cationic lipid.

In a preferred embodiment, the cationic lipid includes a branched alkyl or branched alkenyl group in its biodegradable group(s). In another preferred embodiment, the cationic lipd has a logP of at least 10.2 or 10.3. In yet another preferred embodiment, the cationic lipid has a t_{lipid} - t_{chol}, of at least 1.75, 2.0, or 2.25. The cationic lipid preferably has a pKa of from about 4 to about 7 (such as 6.0 to 6.5).

In one embodiment, the cationic lipid having a logP value of at least 10.1 and/or a t_{lipid} - t_{chol}, of at least 1.4 comprises (a) a head group (preferably a nitrogen containing head group, such as the head groups described herein), (b) at least two hydrophobic tails, each of the formula - (hydrophobic chain)-(biodegradable group)-(hydrophobic chain), and (c) a linker group (for instance, a single central carbon atom) which is bound to the head group and the hydrophobic tails. The cationic lipid preferably has one, two, three, four or more of the properties listed below:
(i) a pKa of from about 4 to about 7 (such as 6.0 to 6.5);
(ii) in at least one hydrophobic tail (and preferably all hydrophobic tails), the biodegradable group is separated from the terminus of the hydrophobic tail by from about 6 to about 12 carbon atoms (for instance, 6 to 8 carbon atoms or 8 to 12 carbon atoms),
(iii) for at least one hydrophobic tail (and preferably all hydrophobic tails), the chain length from the linker group to the terminus of the hydrophobic tail is at most 21 (e.g., at most 20, or from about 17 to about 21, from about 18 to about 20, or from about 16 to about 18) (The atom(s) in the linker group are not counted when calculating the chain length.);
(iv) for at least one hydrophobic tail (and preferably all hydrophobic tails), the total number of carbon atoms in the hydrophobic tail is from about 17 to about 26 (such as from about 19 to about 26, or from about 21 to about 26);
(v) for at least one hydrophobic tail (and preferably all hydrophobic tails), the number of carbon atoms between the linker group and the biodegradable group ranges from about 5 to about 10 (for example, 6 to 10, or 7 to 9);
(vi) for at least one hydrophobic tail (and preferably all hydrophobic tails), the total number of carbon atoms between the linker group and the terminus of the hydrophobic tail is from about 15 to about 20 (such as from 16 to 20, 16 to 18, or 18 to 20);
(vii) for at least one hydrophobic tail (and preferably all hydrophobic tails), the total number of carbon atoms between the biodegradable group and the terminus of the hydrophobic tail is from about 12 to about 18 (such as from 13 to 25);
(viii) for at least one hydrophobic tail (and preferably all hydrophobic tails), the terminal hydrophobic chain in the hydrophobic tail is a branched alkyl or alkenyl group, for example, where the branching occurs at the α, β, γ, or δ position on the hydrophobic chain relative to the biodegradable group;
(ix) when formulated as a lipid nanoparticle (such as in Example 1), the cationic lipid has an *in vivo* half life (t_{1/2}) in the liver of less than about 3 hours, such as less than about 2.5 hours, less than about 2 hours, less than about 1.5 hours, less than about 1 hour, less than about 0.5 hour or less than about 0.25 hours;
(x) when formulated as a lipid nanoparticle (such as in Example 1), the cationic lipid is eliminated from the liver in mice with a greater than 10-fold reduction in lipid levels relative to Cₘₐₓ within the first 24 hours post-dose;
(xi) when formulated as a lipid nanoparticle (such as in Example 1), the cationic lipid is eliminated from the spleen in mice with an equal or greater than 10-fold reduction in lipid levels relative to Cₘₐₓ within the first 168 hours post-dose; and
(xii) when formulated as a lipid nanoparticle (such as in Example 1), the cationic lipid is eliminated from plasma with a terminal plasma half-life (t1/2β) in rodents and non-human primates of 48 hours or shorter.

Suitable cationic lipids include compounds having any combination of some or all of the aforementioned properties. These properties provide a cationic lipid which remains intact until delivery of an active agent, such as a nucleic acid, after which cleavage of the hydrophobic tail occurs *in vivo.* For instance, the compounds can have all of properties (i) to (viii) (in addition to the logP or t_{lipid} - t_{chol} value). In another embodiment, the compounds have properties (i), (ii), (iii), and (viii). In yet another embodiment, the compounds have properties (i), (ii), (iii), (v), (vi), and (viii).

For cationic lipid compounds which contain an atom (e.g., a nitrogen atom) that carries a positive charge, the compound also contains a negatively charged counter ion. The counterion can be any anion, such as an organic or inorganic anion. Suitable examples of anions include, but are not limited to, tosylate, methanesulfonate, acetate, citrate, malonate, tartarate, succinate, benzoate, ascorbate, α-ketoglutarate, α-glycerophosphate, halide (e.g., chloride), sulfate, nitrate, bicarbonate, and carbonate. In one embodiment, the counterion is a halide (e.g., Cl).

Representative central moieties include, but are not limited to, a central carbon atom, a central nitrogen atom, a central carbocyclic group, a central aryl group, a central hetrocyclic group (e.g., central tetrahydrofuranyl group or central pyrrolidinyl group) and a central heteroaryl group. Additionally, the central moiety can include, for example, a glyceride linker, an acyclic glyceride analog linker, or a cyclic linker (including a spiro linker, a bicyclic linker, and a polycyclic linker). The central moiety can include functional groups such as an ether, an ester, a phosphate, a phosphonate, a phosphorothioate, a sulfonate, a disulfide, an acetal, a ketal, an imine, a hydrazone, or an oxime. Other central moieties and functional groups are suitable as well.

In one embodiment, the cationic lipid is a racemic mixture. In another embodiment, the cationic lipid is enriched in one diastereomer, e.g. the cationic lipid has at least 95%, at least 90%, at least 80% or at least 70% diastereomeric excess. In yet another embodiment, the cationic lipid is enriched in one enantiomer, e.g. the lipid has at least 95%, at least 90%, at least 80% or at least 70% enantiomer excess. In yet another embodiment, the cationic lipid is chirally pure, e.g. is a single optical isomer. In yet another embodiment, the cationic lipid is enriched for one optical isomer.

Where a double bond is present (e.g., a carbon-carbon double bond or carbon-nitrogen double bond), there can be isomerism in the configuration about the double bond (i.e. cis/trans or E/Z isomerism). Where the configuration of a double bond is illustrated in a chemical structure, it is understood that the corresponding isomer can also be present. The amount of isomer present can vary, depending on the relative stabilities of the isomers and the energy required to convert between the isomers. Accordingly, some double bonds are, for practical purposes, present in only a single configuration, whereas others (e.g., where the relative stabilities are similar and the energy of conversion low) may be present as inseparable equilibrium mixture of configurations.

In some cases, a double-bonded unsaturation is replaced by a cyclic unsaturation. The cyclic unsaturation can be a cycloaliphatic unsaturation, e.g., a cyclopropyl, cyclobutyl, cyclopentyl, cyclohexyl, cycloheptyl, or cyclooctyl group. In some cases, the cyclic group can be a polycyclic group, e.g., a bicyclic group or tricyclic group. A bicyclic group can be bridged, fused, or have a spiro structure. In some cases, a double bond moiety can be replaced by a cyclopropyl moiety, e.g., 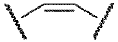 can be replaced by

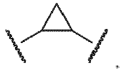

Other suitable tail groups includes those of the formula -R¹²-M¹-R¹³ where R¹² is a C₄-C₁₄ alkyl or C₄-C₁₄ alkenyl, M¹ is a biodegradable group as defined above, and R¹³ is a branched alkyl or alkenyl (e.g., a C₁₀-C₂₀ alkyl or C₁₀-C₂₀ alkenyl), such that (i) the chain length of -R¹²-M¹-R¹³ is at most 21 atoms (i.e., the total length of the tail from the first carbon after the tertiary carbon (marked with an asterisk) to a terminus of the tail is at most 21), and (ii) the group -R¹²-M¹-R¹³ has at least 20 carbon atoms (e.g., at least 21 or 22 carbon atoms).

In one preferred embodiment, the chain length of -R¹²-M¹-R¹³ is at most 21 (e.g., at most 20). For example, the chain length can be from about 17 to about 24 or from about 18 to about 20.

In one embodiment, the total carbon atom content of each tail (-R¹²-M¹-R¹³) is from about 17 to about 26. For example, the total carbon atom content can be from about 19 to about 26 or from about 21 to about 26.

In one embodiment, the tail has the formula: 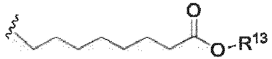 where R¹³ is an alkyl or alkenyl group having from about 13 to about 17 carbon atoms, and the total carbon length of the tail from the first carbon (the leftmost carbon atom above) to a terminus of the tail is at most 20. Preferably, the tail has from about 22 to about 26 carbon atoms. In one embodiment, the maximum length of R¹³ from its attachment point to the ester group of the compound is 12 carbon atoms (e.g., the maximum length can be 11 carbon atoms). In one preferred embodiment, the branch in the alkyl or alkenyl group is at the δ-position or later from the point of attachment of R¹³ to the ester group. Suitable R¹³ groups include, but are not limited to

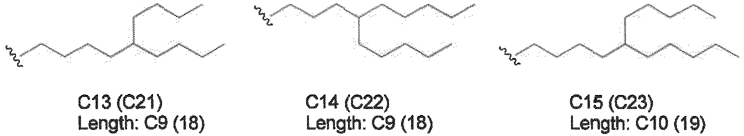

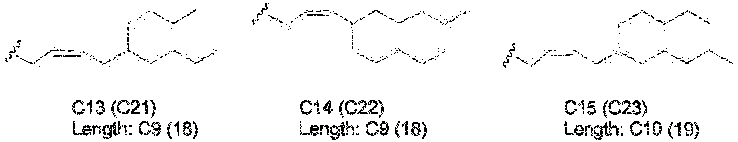

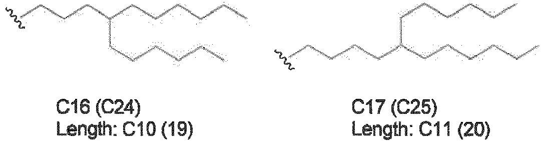

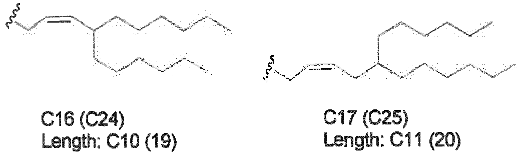

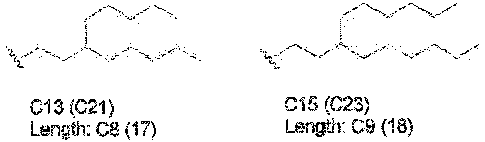

For example, the cationic lipid can be 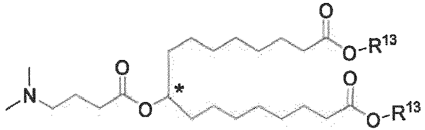 or a salt thereof (e.g., a pharmaceutically acceptable salt thereof), where R¹³ is selected from the groups mentioned above.

Another example is a tail of the formula 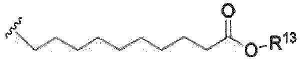 where R¹³ is an alkyl or alkenyl group having from about 13 to about 15 carbon atoms, and the total carbon length of the tail from the first carbon (i.e., the leftmost carbon atom, which is attached to a tertiary carbon) to a terminus of the tail is at most 20. Preferably, the tail has from about 24 to about 26 carbon atoms. In one embodiment, the maximum length of R¹³ from its attachment point to the ester group of the compound is 10 carbon atoms (e.g., the maximum length can be 9 carbon atoms). In one preferred embodiment, the branch in the alkyl or alkenyl group is at the δ-position or later from the point of attachment of R¹³ to the ester group. Suitable R¹³ groups include, but are not limited to

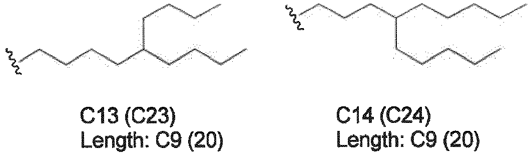

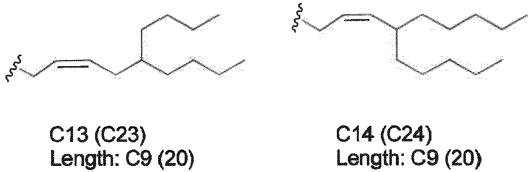

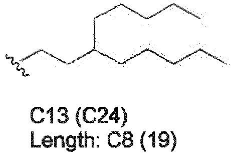

For example, the cationic lipid can be 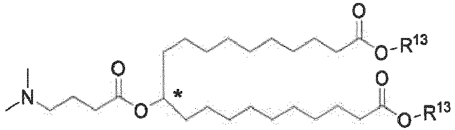 or a salt thereof (e.g., a pharmaceutically acceptable salt thereof), where R¹³ is selected from the groups above.

The R¹³ group may be derived from a natural product, such as dihydrocitgronellol, lavandulol, phytol, or dihydrophytol. In one embodiment, the R¹³ group in the tails above is a dihydrocitronellol group (either as a racemic group or a chirally pure group): 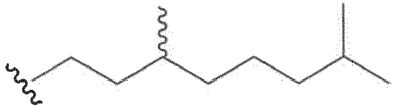 For example, the cationic lipid having a dihydroitronellol group can be 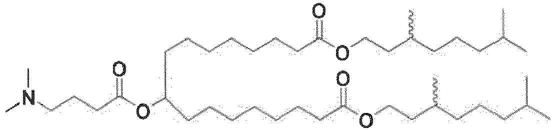 or 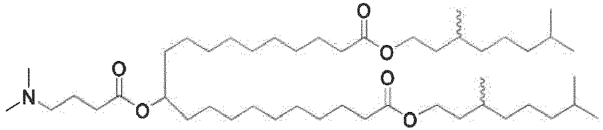 or a salt thereof (e.g., a pharmaceutically acceptable salt thereof).

In another embodiment, the R¹³ group in the tails above is a lavandulol group or a homolog of it as shown below:

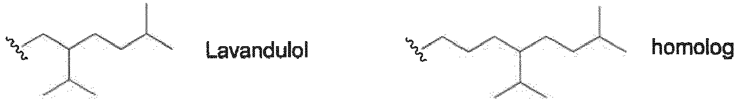

In another embodiment, the R¹³ group in the tails above is a phytol or dihydrophytol group: 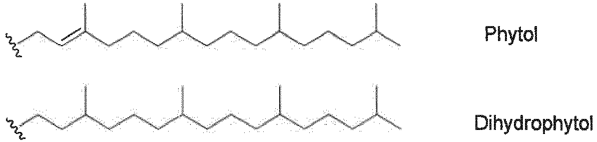 For instance, the cationic lipid can be: 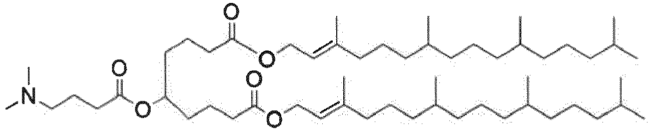 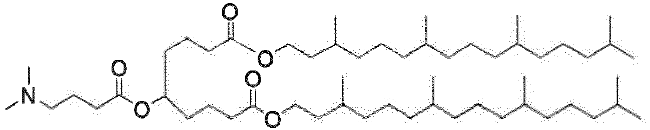 A cationic lipid of the fomula: 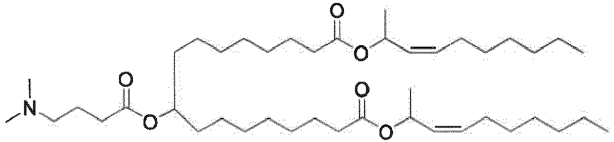 can also be thought of as a combination of a headgroup, a linker moiety, and two parts of the hydrophobic chains as follows:

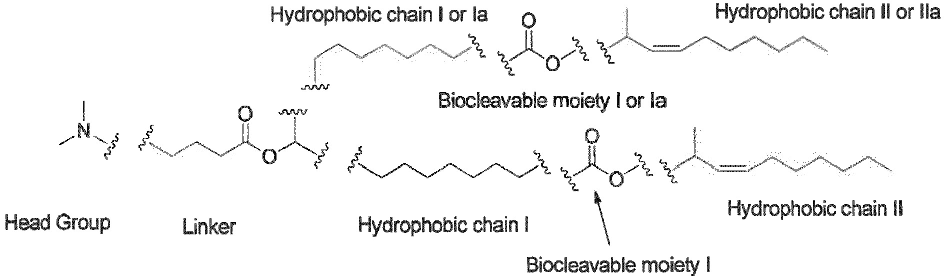

Various headgroups, linker moieties, and hydrophobic chains I and II are listed below. Suitable cationic lipids include compounds composed of any combination of the head, linker, hydrophobic chain I, and hydrophobic chain II groups listed below.

**Table 2A - Representative headgroups**

| | | | | |
|---|---|---|---|---|
| 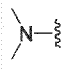 | 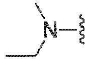 | 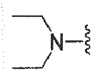 | 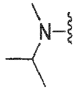 | 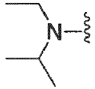 |
| 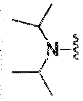 | 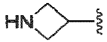 | 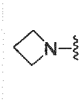 | 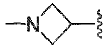 | 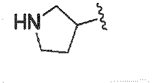 |
| 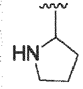 | 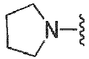 | 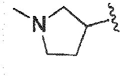 | 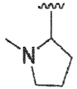 | 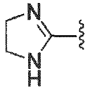 |
| 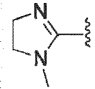 | 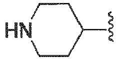 | 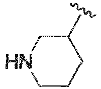 | 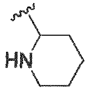 | 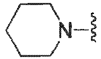 |
| 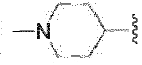 | 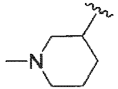 | 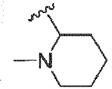 | 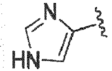 | 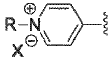 R = H, alkyl; X = halogen |
| 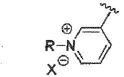 **R = H, alkyl; X = halogen** | 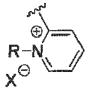 **R** = **H, alkyl; X** = **halogen** | 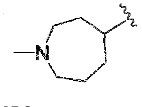 | 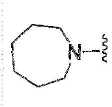 | 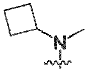 |
| 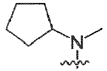 | 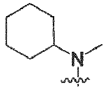 | 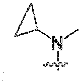 | 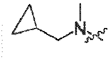 | 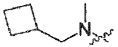 |
| 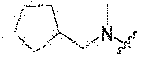 | 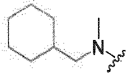 | 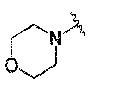 | 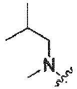 | 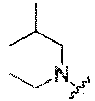 |
| 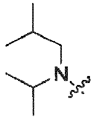 | 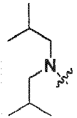 | 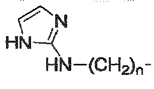 (where n is 0-5) | 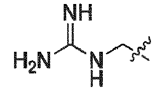 | 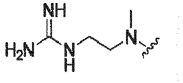 |
| 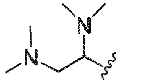 | 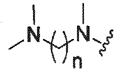 n = 0-6 | 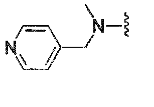 | 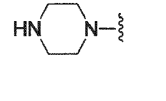 | 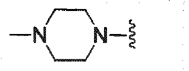 |
| 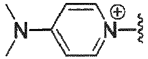 | 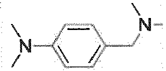 | 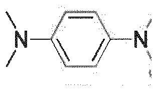 | 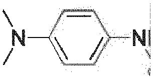 | 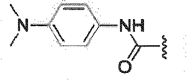 |
| 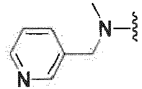 | 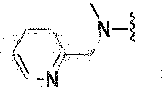 | 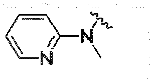 | 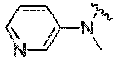 | 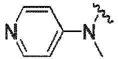 |

**Table 2B - Representative linker groups**

| | | | |
|---|---|---|---|
| 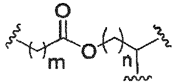 m = 1-5; n = 0-3 | 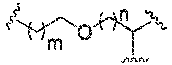 m = 0-5, n = 0-3 | 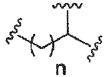 n = 0-5 | 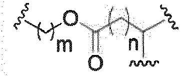 m = 0-5; n = 0-3 |
| 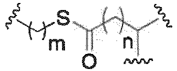 m = 0-5; n = 0-3 | 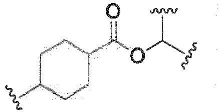 | 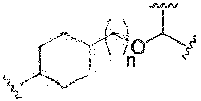 n = 0-3 | 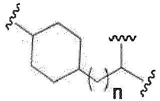 n = 0-3 |
| 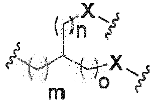 m = 1-4; nlo = 0-3 | 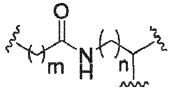 m = 0-5; n = 0-3 | 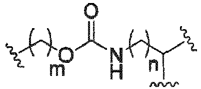 m = 0-5; n = 0-3 | 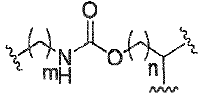 m = 0-5; n = 0-3 |
| x = OorS | | | |
| 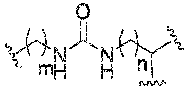 m = 0-5; n = 0-3 | 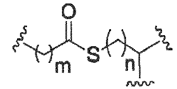 m = 0-5; n = 0-3 | 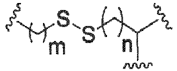 m = 0-5; n = 0-3 | 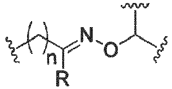 n = 0-5 |
| 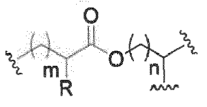 m = 1-4; n = 0-3 | 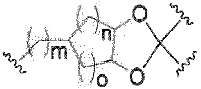 m = 1-4; n/o = 1-3 | 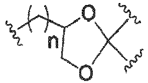 n = 1-5 | 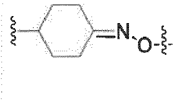 |
| R = COOH, COOMe, COOEt, CN, CONH2 CONHMe | | | |
| 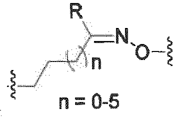 R = H, Me, Et, Pr, allyl | 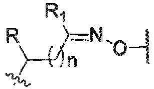 n = 0-5 | 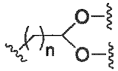 n = 0-6 | 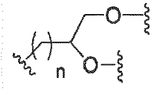 n = 0-6 |
| | R = Me, Et, Pr, allyl | | |
| | R1= Me, Et, Pr, allyl | | |
| 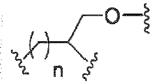 n = 0-6 | 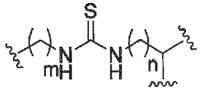 m = 0-5; n = 0-3 | | |

**Table 2C - Representative hydrophobic chain I and/or Ia, and combination thereof**

| | |
|---|---|
| 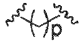 p = 0-15 | 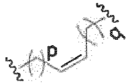 p = 0-15, q = 0-15 |
| 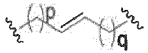 p = 0-15, q = 0-15 | 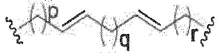 p = 0-15, q = 1-4, r = 0-15 |
| 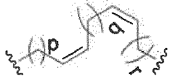 p = 0-15, q = 1-4, r = 0-15 | |
| 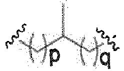 p = 0-15, q = 0-6 | 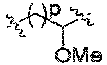 p = 0-15 |
| 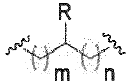 m = 0-4; n = 0-4; R = Me, Et, Pr, iPr, Bu, iBu | 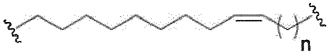 n = 1-7 |
| 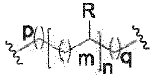 m = 1-4, n = 1-10, p = 0-15, q = 0-15 | |
| R = Me, Et, OMe | |

**Table 2D - Representative biodegradable moieties I and/or Ia and combinations thereof**

| | | | |
|---|---|---|---|
| 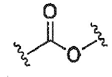 | 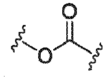 | 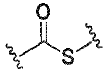 | 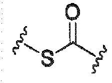 |
| 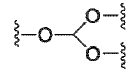 | 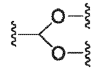 | 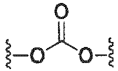 | 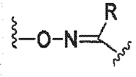 |
| 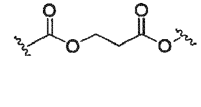 | 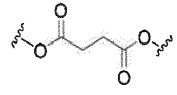 | 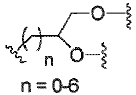 | 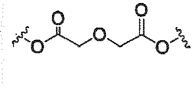 |
| 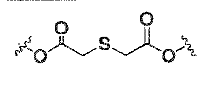 | 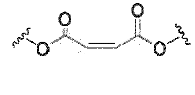 | 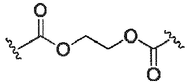 | 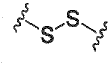 |
| 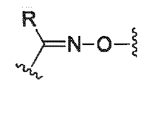 | 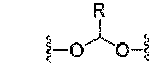 R = H, Me, Et, cyclic alkyl, alicylic, aromatic | 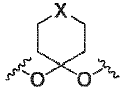 X = CH₂, O. S | |

**Table 2E - Representative hydrophobic chain II and/or IIa and combinations thereof**

| | | |
|---|---|---|
| 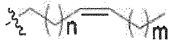 n = 0-6; m = 0-16 | 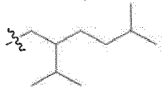 | 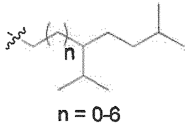 |
| 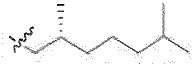 | 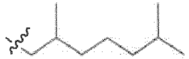 | 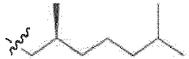 |
| 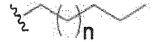 n = 0-8 | 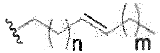 n = 0-8; m = 0-6 | 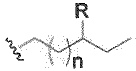 n = 0-8 |
| | | R =OMe, Me, Et, n-Pr, n-Bu |
| 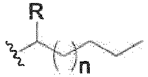 n = 0-8 | 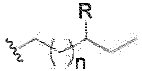 n = 0-8 | 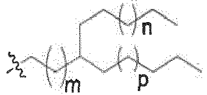 m =0-6; n = 0-6; p = 0-6 |
| R =OMe, Me, Et, Pr | R =OMe, Me, Et, Pr | |
| 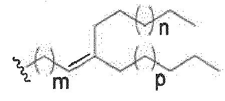 m =0-6; n = 0-6; p = 0-6 | 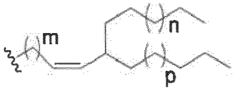 m =0-6; n = 0-6; p = 0-6 | 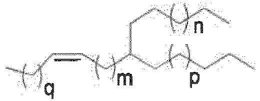 m =0-6; n = 0-6; p = 0-6; q = 0-6 |

In one embodiment, the cationic lipid is the following compound, and salts thereof (including pharmaceutically acceptable salts thereof). This cationic lipid is suitable for forming nucleic acid-lipid particles.

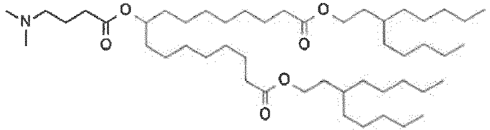

Alternatively, for the compounds above having a head of the formula 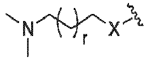 (where X can be, for example, -C(O)O-), the head can have one methylene unit between the X group (or other functional group) and nitrogen atom. For example, the head can be:

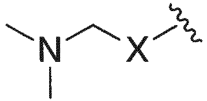

Cationic lipids include those having alternative fatty acid groups and other dialkylamino groups than those shown, including those in which the alkyl substituents are different (e.g., N-ethyl-N-methylamino-, and N-propyl-N-ethylamino-).

Included herein is the free form of the cationic lipids described herein, as well as pharmaceutically acceptable salts and stereoisomers thereof. The cationic lipid can be a protonated salt of the amine cationic lipid. The term "free form" refers to the amine cationic lipids in non-salt form. The free form may be regenerated by treating the salt with a suitable dilute aqueous base solution such as dilute aqueous NaOH, potassium carbonate, ammonia and sodium bicarbonate.

The pharmaceutically acceptable salts of the cationic lipids can be synthesized from the cationic lipids which contain a basic or acidic moiety by conventional chemical methods. Generally, the salts of the basic cationic lipids are prepared either by ion exchange chromatography or by reacting the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base.

Thus, pharmaceutically acceptable salts of the cationic lipids include non-toxic salts of the cationic lipids as formed by reacting a basic instant cationic lipids with an inorganic or organic acid. For example, non-toxic salts include those derived from inorganic acids such as hydrochloric, hydrobromic, sulfuric, sulfamic, phosphoric, nitric and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, and trifluoroacetic (TFA).

When the cationic lipids are acidic, suitable "pharmaceutically acceptable salts" refers to salts prepared form pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, and zinc. In one embodiment, the base is selected from ammonium, calcium, magnesium, potassium and sodium. Salts derived from pharmaceutically acceptable organic non-toxic bases include salts of primary, secondary and tertiary amines, substituted amines including naturally occurring substituted amines, cyclic amines and basic ion exchange resins, such as arginine, betaine caffeine, choline, N,N¹-dibenzylethylenediamine, diethylamin, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, and tromethamine.

It will also be noted that the cationic lipids may potentially be internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety, such as a quaternary nitrogen atom.

One or more additional cationic lipids, which carry a net positive charge at about physiological pH, in addition to those specifically described above, may also be included in the lipid particles and compositions described herein. Such cationic lipids include, but are not limited to N,N-dioleyl-N,N-dimethylammonium chloride ("DODAC"); N-(2,3-dioleyloxy)propyl-N,N-N-triethylammonium chloride ("DOTMA"); N,N-distearyl-N,N-dimethylammonium bromide ("DDAB"); N-(2,3-dioleoyloxy)propyl)-N,N,N-trimethylammonium chloride ("DOTAP"); 1,2-Dioleyloxy-3-trimethylaminopropane chloride salt ("DOTAP.Cl"); 3β-(N-(N',N'-dimethylaminoethane)-carbamoyl)cholesterol ("DC-Chol"), N-(1-(2,3-dioleyloxy)propyl)-N-2-(sperminecarboxamido)ethyl)-N,N-dimethylammonium trifluoracetate ("DOSPA"), dioctadecylamidoglycyl carboxyspermine ("DOGS"), 1,2-dileoyl-sn-3-phosphoethanolamine ("DOPE"), 1,2-dioleoyl-3-dimethylammonium propane ("DODAP"), N, N-dimethyl-2,3-dioleyloxy)propylamine ("DODMA"), and N-(1,2-dimyristyloxyprop-3-yl)-N,N-dimethyl-N-hydroxyethyl ammonium bromide ("DMRIE"). Additionally, a number of commercial preparations of cationic lipids can be used, such as, *e.g.,* LIPOFECTIN (including DOTMA and DOPE, available from GIBCO/BRL), and LIPOFECTAMINE (comprising DOSPA and DOPE, available from GIBCO/BRL).

### The Other Lipid Components

The lipid particles and compositions described herein may also include one or more neutral lipids. Neutral lipids, when present, can be any of a number of lipid species which exist either in an uncharged or neutral zwitterionic form at physiological pH. Such lipids include, for example, diacylphosphatidylcholine, diacylphosphatidylethanolamine, ceramide, sphingomyelin, dihydrosphingomyelin, cephalin, and cerebrosides. In one embodiment, the neutral lipid component is a lipid having two acyl groups (*e.g*., diacylphosphatidylcholine and diacylphosphatidylethanolamine). In one embodiment, the neutral lipid contains saturated fatty acids with carbon chain lengths in the range of C₁₀ to C₂₀. In another embodiment, the neutral lipid includes mono or diunsaturated fatty acids with carbon chain lengths in the range of C₁₀ to C₂₀. Suitable neutral lipids include, but are not limited to, DSPC, DPPC, POPC, DOPE, DSPC, and SM.

The lipid particles and compositions described herein may also include one or more lipids capable of reducing aggregation. Examples of lipids that reduce aggregation of particles during formation include polyethylene glycol (PEG)-modified lipids (PEG lipids, such as PEG-DMG and PEG-DMA), monosialoganglioside Gm1, and polyamide oligomers ("PAO") such as (described in U.S. Patent No. 6,320,017, which is incorporated by reference in its entirety). Suitable PEG lipids include, but are not limited to, PEG-modified phosphatidylethanolamine and phosphatidic acid, PEG-ceramide conjugates (*e.g*., PEG-CerC14 or PEG-CerC20) (such as those described in U.S. Patent No. 5,820,873), PEG-modified dialkylamines and PEG-modified 1,2-diacyloxypropan-3-amines, PEG-modified diacylglycerols and dialkylglycerols, mPEG (mw2000)-diastearoylphosphatidylethanolamine (PEG-DSPE).

The lipid particles and compositions may include a sterol, such as cholesterol.

### Lipid Particles

In a further aspect, the present invent relates to lipid particles that include one or more of the cationic lipids described herein.

Lipid particles include, but are not limited to, liposomes. As used herein, a liposome is a structure having lipid-containing membranes enclosing an aqueous interior.

Another embodiment is a nucleic acid-lipid particle (e.g., a SNALP) comprising a cationic lipid, a non-cationic lipid (such as a neutral lipid), optionally a PEG-lipid conjugate (such as the lipids for reducing aggregation of lipid particles discussed herein), optionally a sterol (e.g., cholesterol), and a nucleic acid. As used herein, the term "SNALP" refers to a stable nucleic acid-lipid particle. A SNALP represents a particle made from lipids, wherein the nucleic acid (e.g., an interfering RNA) is encapsulated within the lipids. In certain instances, SNALPs are useful for systemic applications, as they can exhibit extended circulation lifetimes following intravenous (i.v.) injection, they can accumulate at distal sites (e.g., sites physically separated from the administration site), and they can mediate silencing of target gene expression at these distal sites. The nucleic acid may be complexed with a condensing agent and encapsulated within a SNALP as set forth in International Publication No. WO 00/03683.

For example, the lipid particle may include a cationic lipid, a fusion-promoting lipid (e.g., DPPC), a neutral lipid, cholesterol, and a PEG-modified lipid. In one embodiment, the lipid particle includes the above lipid mixture in molar ratios of about 20-70% cationic lipid: 0.1-50% fusion promoting lipid: 5-45% neutral lipid: 20-55% cholesterol: 0.5-15% PEG-modified lipid (based upon 100% total moles of lipid in the lipid particle).

In another embodiment of the lipid particle, the cationic lipid is present in a mole percentage of about 20% and about 60%; the neutral lipid is present in a mole percentage of about 5% to about 25%; the sterol is present in a mole percentage of about 25% to about 55%; and the PEG lipid is PEG-DMA, PEG-DMG, or a combination thereof, and is present in a mole percentage of about 0.5% to about 15% (based upon 100% total moles of lipid in the lipid particle).

In particular embodiments, the molar lipid ratio, with regard to mol% cationic lipid/DSPC/Chol/PEG-DMG or PEG-DMA) is approximately 40/10/40/10, 35/15/40/10 or 52/13/30/5. This mixture may be further combined with a fusion-promoting lipid in a molar ratio of 0.1-50%, 0.1-50%, 0.5-50%, 1-50%, 5%-45%, 10%-40%, or 15%-35%. In other words, when a 40/10/40/10 mixture of lipid/DSPC/Chol/PEG-DMG or PEG-DMA is combined with a fusion-promoting peptide in a molar ratio of 50%, the resulting lipid particles can have a total molar ratio of (mol°lo cationic lipid/DSPC/Chol/PEG-DMG or PEG-DMA/fusion-promoting peptide) 20/5/20/5/50. In another embodiment, the neutral lipid, DSPC, in these compositions is replaced with POPC, DPPC, DOPE or SM.

In one embodiment, the lipid particles comprise a cationic lipid, a neutral lipid, a sterol and a PEG-modified lipid. In one embodiment, the lipid particles include from about 25% to about 75% on a molar basis of cationic lipid, e.g., from about 35 to about 65%, from about 45 to about 65%, about 60%, about 57.5%, about 57.1%, about 50% or about 40% on a molar basis. In one embodiment, the lipid particles include from about 0% to about 15% on a molar basis of the neutral lipid, e.g., from about 3 to about 12%, from about 5 to about 10%, about 15%, about 10%, about 7.5%, about 7.1% or about 0% on a molar basis. In one embodiment, the neutral lipid is DPPC. In one embodiment, the neutral lipid is DSPC.

In one embodiment, the formulation includes from about 5% to about 50% on a molar basis of the sterol, e.g., about 15 to about 45%, about 20 to about 40%, about 48%, about 40%, about 38.5%, about 35%, about 34.4%, about 31.5% or about 31% on a molar basis. In one embodiment, the sterol is cholesterol.

The lipid particles described herein may further include one or more therapeutic agents. In a preferred embodiment, the lipid particles include a nucleic acid (e.g., an oligonucleotide), such as siRNA or miRNA.

In one embodiment, the lipid particles include from about 0.1% to about 20% on a molar basis of the PEG-modified lipid, e.g., about 0.5 to about 10%, about 0.5 to about 5%, about 10%, about 5%, about 3.5%, about 1.5%, about 0.5%, or about 0.3% on a molar basis. In one embodiment, the PEG-modified lipid is PEG- DMG. In one embodiment, the PEG-modified lipid is PEG-c-DMA. In one embodiment, the lipid particles include 25-75% of cationic lipid, 0.5-15% of the neutral lipid, 5-50% of the sterol, and 0.5- 20% of the PEG-modified lipid on a molar basis.

In one embodiment, the lipid particles include 35-65% of cationic lipid, 3-12% of the neutral lipid, 15-45% of the sterol, and 0.5- 10% of the PEG-modified lipid on a molar basis. In one embodiment, the lipid particles include 45-65% of cationic lipid, 5-10% of the neutral lipid, 25-40% of the sterol, and 0.5- 5% of the PEG-modified lipid on a molar basis. In one embodiment, the PEG modified lipid comprises a PEG molecule of an average molecular weight of 2,000 Da. In one embodiment, the PEG modified lipid is PEG-distyryl glycerol (PEG-DSG). In one preferred embodiment, the PEG modified lipid is 1,2-dimyristoyl-sn-glycerol-methoxy polyethylene glycol (PEG-DMG), such as PEG-DMG with an average polyethylene glycol molecular weight of 2000.

In one embodiment, the ratio of lipid:siRNA (weight [mg]:weight [mg] is at least about 0.5:1, at least about 1:1, at least about 2:1, at least about 3:1, at least about 4:1, at least about 5:1, at least about 6:1, at least about 7:1, at least about 11:1 or at least about 33:1. In one embodiment, the ratio of lipid: siRNA ratio is between about 1:1 to about 35:1, about 3:1 to about 15:1, about 4:1 to about 15:1, or about 5:1 to about 13:1. In one embodiment, the ratio of lipid: siRNA ratio is between about 3:1 to about 12:1.

One embodiment is a lipid particle comprising a biodegradable cationic lipid, a neutral lipid, a sterol, and a lipid capable of reducing aggregation (e.g., PEG-modified lipid), where the molar ratio of the biodegradable cationic lipid to the sterol ranges from about 1.6:1 to about 2.0:1 and/or the molar ratio of the biodegradable cationic lipid to the neutral lipid ranges from about 5.5:1 to about 5.9:1. The inventors have surprisingly found that lipid particles having certain higher contents of biodegradable cationic lipid relative to the amount sterol and/or neutral lipid exhibit enhanced efficacy for the delivery of an active agent (e.g., siRNA). In one embodiment, the biodegradable cationic lipid comprises a lipid moiety, where the lipid moiety has one or more biodegradable groups (such as an ester group (-C(O)O- or -OC(O)-). In one preferred embodiment, the lipid capable of reducing aggregation is 1,2-dimyristoyl-sn-glycerol-methoxy polyethylene glycol (PEG-DMG), such as PEG-DMG with an average polyethylene glycol molecular weight of 2000.

In a further embodiment, the molar ratio of the biodegradable cationic lipid to the sterol is from about 1.7 to about 1.9:1, such as about 1.9:1. In another embodiment, the molar ratio of the biodegradable cationic lipid to the neutral lipid ranges from about 5.5:1 to about 5.8:1, such as about 5.8:1.

In one embodiment, the lipid particle comprises from about 55 to about 60 mol % of the biodegradable cationic lipid, such as about 58 mol % (based on 100 mol % of the lipid components in the lipid particle). The lipid particle may comprise from about 28 to about 33 mol % of the sterol, such as from about 28 to about 32 mol % (based on 100 mol % of the lipid components in the lipid particle). In one embodiment, the lipid particle comprises from about 3 to about 12 mol %, such as from about 5 to about 12 mol %, from about 8 to about 12 mol %, or from about 9 to about 11 mol %, of the neutral lipid (based on 100 mol % of the lipid components in the lipid particle). In another embodiment, the lipid particle comprises about 10 mol % of the neutral lipid (based on 100 mol % of the lipid components in the lipid particle). In yet another embodiment, the lipid particle comprises from about 0.5 to about 10 mol %, such as from about 0.5 to about 5 mol % or from about 1 to about 3 mol %, of the lipid capable of reducing aggregation (e.g., a PEG-modified lipid) (based on 100 mol % of the lipid components in the lipid particle).

Another embodiment is a lipid particle comprising a biodegradable cationic lipid, a neutral lipid, a sterol, and a lipid capable of reducing aggregation (e.g., PEG-modified lipid), where the lipid particle comprises from about 55 to about 60 mol % of the biodegradable cationic lipid and from about 33 to about 28 mol % of the sterol (based on 100 mol % of the lipid components in the lipid particle). In one embodiment, the lipid particle comprises about 58 mol % of the biodegradable cationic lipid (based on 100 mol % of the lipid components in the lipid particle). In another embodiment, the lipid particle comprises from about 3 to about 12% of the neutral lipid, and from about 0.5 to about 10 mol % of the lipid capable of reducing aggregation (based on 100 mol % of the lipid components in the lipid particle). In yet another embodiment, the lipid particle comprises about 10 mol % of the neutral lipid (based on 100 mol % of the lipid components in the lipid particle). In yet another embodiment, the lipid particle comprises about 2 mol % of the lipid capable of reducing aggregation (based on 100 mol % of the lipid components in the lipid particle). In one embodiment, the lipid particle comprises from about 55 to about 60 mol % of the cationic lipid, from about 3 to about 12% of the neutral lipid, from about 28 to about 33 mol % of the sterol, and from about 0.5 to about 10 mol % of the lipid capable of reducing aggregation (based on 100 mol % of the lipid components in the lipid particle). In yet another embodiment, the lipid particle comprises about 58% of the cationic lipid, about 10% of the neutral lipid, about 30% of the sterol, and about 2% of the lipid capable of reducing aggregation (based on 100 mol % of the lipid components in the lipid particle). In yet another embodiment, the lipid particle comprises about 55% of the cationic lipid, about 10% of the neutral lipid, about 33% of the sterol, and about 2% of the lipid capable of reducing aggregation (based on 100 mol % of the lipid components in the lipid particle). In one preferred embodiment, the lipid capable of reducing aggregation is 1,2-dimyristoyl-sn-glycerol-methoxy polyethylene glycol (PEG-DMG), such as PEG-DMG with an average polyethylene glycol molecular weight of 2000.

In one embodiment, the lipid particles are nanoparticles. In additional embodiments, the lipid particles have a mean diameter size of from about 50 nm to about 300 nm, such as from about 50 nm to about 250 nm, for example, from about 50 nm to about 200 nm.

In one embodiment, a lipid particle containing a cationic lipid of any of the embodiments described herein has an *in vivo* half life (t_{1/2}) (e.g., in the liver, spleen or plasma) of less than about 3 hours, such as less than about 2.5 hours, less than about 2 hours, less than about 1.5 hours, less than about 1 hour, less than about 0.5 hour or less than about 0.25 hours.

In another embodiment, a lipid particle containing a cationic lipid of any of the embodiments described herein has an *in vivo* half life (t_{1/2}) (e.g., in the liver, spleen or plasma) of less than about 10 % (e.g., less than about 7.5%, less than about 5%, less than about 2.5%) of that for the same cationic lipid without the biodegrable group or groups.

### Additional Components

The lipid particles and compositions described herein can further include one or more antioxidants. The antioxidant stabilizes the lipid particle and prevents, decreases, and/or inhibits degradation of the cationic lipid and/or active agent present in the lipid particles. The antioxidant can be a hydrophilic antioxidant, a lipophilic antioxidant, a metal chelator, a primary antioxidant, a secondary antioxidant, salts thereof, and mixtures thereof. In certain embodiments, the antioxidant comprises a metal chelator such as EDTA or salts thereof, alone or in combination with one, two, three, four, five, six, seven, eight, or more additional antioxidants such as primary antioxidants, secondary antioxidants, or other metal chelators. In one preferred embodiment, the antioxidant comprises a metal chelator such as EDTA or salts thereof in a mixture with one or more primary antioxidants and/or secondary antioxidants. For example, the antioxidant may comprise a mixture of EDTA or a salt thereof, a primary antioxidant such as a-tocopherol or a salt thereof, and a secondary antioxidant such as ascorbyl palmitate or a salt thereof. In one embodiment, the antioxidant comprises at least about 100 mM citrate or a salt thereof. Examples of antioxidants include, but are not limited to, hydrophilic antioxidants, lipophilic antioxidants, and mixtures thereof. Non-limiting examples of hydrophilic antioxidants include chelating agents (e.g., metal chelators) such as ethylenediaminetetraacetic acid (EDTA), citrate, ethylene glycol tetraacetic acid (EGTA), 1,2-bis(o-aminophenoxy)ethane-N,N,N',N'-tetraacetic acid (BAPTA), diethylene triamine pentaacetic acid (DTPA), 2,3-dimercapto-1-propanesulfonic acid (DMPS), dimercaptosuccinic acid (DMSA), cc-lipoic acid, salicylaldehyde isonicotinoyl hydrazone (SIH), hexyl thioethylamine hydrochloride (HTA), desferrioxamine, salts thereof, and mixtures thereof. Additional hydrophilic antioxidants include ascorbic acid, cysteine, glutathione, dihydrolipoic acid, 2- mercaptoethane sulfonic acid, 2-mercaptobenzimidazole sulfonic acid, 6-hydroxy-2,5,7,8-tetramethylchroman-2-carboxylic acid, sodium metabisulfite, salts thereof, and mixtures thereof. Non-limiting examples of lipophilic antioxidants include vitamin E isomers such as α-, β-, γ-, and δ-tocopherols and α-, β-, γ-, and δ-tocotrienols; polyphenols such as 2-tert-butyl-4-methyl phenol, 2-fert-butyl-5-methyl phenol, and 2-tert-butyl-6-methyl phenol; butylated hydroxyanisole (BHA) (e.g., 2-teri-butyl-4-hydroxyanisole and 3-tert-butyl-4-hydroxyanisole); butylhydroxytoluene (BHT); tert-butylhydroquinone (TBHQ); ascorbyl palmitate; rc-propyl gallate; salts thereof; and mixtures thereof. Suitable antioxidants and formulations containing such antioxidants are described in International Publication No. WO 2011/066651.

In another embodiment, the lipid particles or compositions contain the antioxidant EDTA (or a salt thereof), the antioxidant citrate (or a salt thereof), or EDTA (or a salt thereof) in combination with one or more (e.g., a mixture of) primary and/or secondary antioxidants such as α-tocopherol (or a salt thereof) and/or ascorbyl palmitate (or a salt thereof).

In one embodiment, the antioxidant is present in an amount sufficient to prevent, inhibit, or reduce the degradation of the cationic lipid present in the lipid particle. For example, the antioxidant may be present at a concentration of at least about or about 0.1 mM, 0.5 mM, 1 mM, 10 mM, 100 mM, 500 mM, 1 M, 2 M, or 5M, or from about 0.1 mM to about 1 M, from about 0.1 mM to about 500 mM, from about 0.1 mM to about 250 mM, or from about 0.1 mM to about 100 mM.

The lipid particles and compositions described herein can further include an apolipoprotein. As used herein, the term "apolipoprotein" or "lipoprotein" refers to apolipoproteins known to those of skill in the art and variants and fragments thereof and to apolipoprotein agonists, analogues or fragments thereof described below.

In a preferred embodiment, the active agent is a nucleic acid, such as a siRNA. For example, the active agent can be a nucleic acid encoded with a product of interest, including but not limited to, RNA, antisense oligonucleotide, an antagomir, a DNA, a plasmid, a ribosomal RNA (rRNA), a micro RNA (miRNA) (e.g., a miRNA which is single stranded and 17-25 nucleotides in length), transfer RNA (tRNA), a small interfering RNA (siRNA), small nuclear RNA (snRNA), antigens, fragments thereof, proteins, peptides, vaccines and small molecules or mixtures thereof. In one more preferred embodiment, the nucleic acid is an oligonucleotide (e.g., 15-50 nucleotides in length (or 15-30 or 20-30 nucleotides in length)). An siRNA can have, for instance, a duplex region that is 16-30 nucleotides long. In another embodiment, the nucleic acid is an immunostimulatory oligonucleotide, decoy oligonucleotide, supermir, miRNA mimic, or miRNA inhibitor. A supermir refers to a single stranded, double stranded or partially double stranded oligomer or polymer of ribonucleic acid (RNA) or deoxyribonucleic acid (DNA) or both or modifications thereof, which has a nucleotide sequence that is substantially identical to an miRNA and that is antisense with respect to its target. miRNA mimics represent a class of molecules that can be used to imitate the gene silencing ability of one or more miRNAs. Thus, the term "microRNA mimic" refers to synthetic non-coding RNAs (i.e. the miRNA is not obtained by purification from a source of the endogenous miRNA) that are capable of entering the RNAi pathway and regulating gene expression.

The nucleic acid that is present in a lipid-nucleic acid particle can be in any form. The nucleic acid can, for example, be single-stranded DNA or RNA, or double-stranded DNA or RNA, or DNA-RNA hybrids including their chemically modified analogs. Non-limiting examples of double-stranded RNA include siRNA. Single-stranded nucleic acids include, e.g., antisense oligonucleotides, ribozymes, microRNA, and triplex-forming oligonucleotides. The lipid particles can also deliver nucleic acids which are conjugated to one or more ligands.

### Pharmaceutical Compositions

The lipid particles, particularly when associated with a therapeutic agent, may be formulated as a pharmaceutical composition, e.g., which further comprises a pharmaceutically acceptable diluent, excipient, or carrier, such as physiological saline or phosphate buffer.

The resulting pharmaceutical preparations may be sterilized by conventional, well known sterilization techniques. The aqueous solutions can then be packaged for use or filtered under aseptic conditions and lyophilized, the lyophilized preparation being combined with a sterile aqueous solution prior to administration. The compositions may contain pharmaceutically acceptable auxiliary substances as required to approximate physiological conditions, such as pH adjusting and buffering agents, and tonicity adjusting agents, for example, sodium acetate, sodium lactate, sodium chloride, potassium chloride, and calcium chloride. Additionally, the lipidic suspension may include lipid-protective agents which protect lipids against free-radical and lipid-peroxidative damages on storage. Lipophilic free-radical quenchers, such as α-tocopherol and water-soluble iron-specific chelators, such as ferrioxamine, are suitable.

The concentration of lipid particle or lipid-nucleic acid particle in the pharmaceutical formulations can vary, for example, from less than about 0.01%, to at or at least about 0.05-5% to as much as 10 to 30% by weight.

### Methods of Manufacture

Methods of making cationic lipids, lipid particles containing them, and pharmaceutical compositions containing the cationic lipids and/or lipid particles are described in, for example, International Publication Nos. WO 2010/054406, WO 2010/054401, WO 2010/054405, WO 2010/054384, WO 2010/042877, WO 2010/129709, WO 2009/086558, and WO 2008/042973, and U.S. Patent Publication Nos. 2004/0142025, 2006/0051405 and 2007/0042031.

For example, in one embodiment, a solution of one or more lipids (including a cationic lipid of any of the embodiments described herein) in an organic solution (e.g., ethanol) is prepared. Similarly, a solution of one or more active (therapeutic) agents (such as, for example an siRNA molecule or a 1:1 molar mixture of two siRNA molecules) in an aqueous buffered (e.g., citrate buffer) solution is prepared. The two solutions are mixed and diluted to form a colloidal suspension of siRNA lipid particles. In one embodiment, the siRNA lipid particles have an average particle size of about 80-90 nm. In further embodiments, the dispersion may be filtered through 0.45/2 micron filters, concentrated and diafiltered by tangential flow filtration.

### Definitions

As used herein, the term "cationic lipid" inlcudes those lipids having one or two fatty acid or fatty aliphatic chains and an amino acid containing head group that may be protonated to form a cationic lipid at physiological pH. In some embodiments, a cationic lipid is referred to as an "amino acid conjugate cationic lipid."

A subject or patient in whom administration of the complex is an effective therapeutic regimen for a disease or disorder is preferably a human, but can be any animal, including a laboratory animal in the context of a clinical trial or screening or activity experiment. Thus, as can be readily appreciated by one of ordinary skill in the art, the methods, compounds and compositions described herein are particularly suited to administration to any animal, particularly a mammal, and including, but by no means limited to, humans, domestic animals, such as feline or canine subjects, farm animals, such as but not limited to bovine, equine, caprine, ovine, and porcine subjects, wild animals (whether in the wild or in a zoological garden), research animals, such as mice, rats, rabbits, goats, sheep, pigs, dogs, and cats, avian species, such as chickens, turkeys, and songbirds, i.e., for veterinary medical use.

Many of the chemical groups recited in the generic formulas above are written in a particular order (for example, -OC(O)-). It is intended that the chemical group is to be incorporated into the generic formula in the order presented unless indicated otherwise. For example, a generic formula of the form -(R)ᵢ-(M¹)ₖ-(R)ₘ- where M¹ is -C(O)O- and k is 1 refers to -(R)ᵢ-C(O)O-(R)ₘ- unless specified otherwise. It is to be understood that when a chemical group is written in a particular order, the reverse order is also contemplated unless otherwise specified. For example, in a generic formula -(R)ᵢ-(M¹)ₖ-(R)ₘ- where M¹ is defined as -C(O)NH- (i.e., -(R)ᵢ-C(O)-NH-(R)ₘ-), the compound where M¹ is -NHC(O)- (i.e., -(R)ᵢ-NHC(O)-(R)ₘ-) is also contemplated unless otherwise specified.

The term "biodegradable cationic lipid" refers to a cationic lipid having one or more biodegradable groups located in the mid- or distal section of a lipidic moiety (e.g., a hydrophobic chain) of the cationic lipid. The incorporation of the biodegradable group(s) into the cationic lipid results in faster metabolism and removal of the cationic lipid from the body following delivery of the active pharmaceutical ingredient to a target area.

As used herein, the term "biodegradable group" refers to a group that include one or more bonds that may undergo bond breaking reactions in a biological environment, e.g., in an organism, organ, tissue, cell, or organelle. For example, the biodegradable group may be metabolizable by the body of a mammal, such as a human (e.g., by hydrolysis). Some groups that contain a biodegradable bond include, for example, but are not limited to esters, dithiols, and oximes. Non-limiting examples of biodegradable groups are -OC(O)-, -C(O)O-, -SC(O)-, -C(O)S-, -OC(S)-, - C(S)O-, -S-S-, -C(R⁵)=N-, -N=C(R⁵)-, -C(R⁵)=N-O-, -O-N=C(R⁵)-, -C(O)(NR⁵)-, -N(R⁵)C(O)-, - C(S)(NR⁵)-, -N(R⁵)C(O)-, -N(R⁵)C(O)N(R⁵)-, -OC(O)O-, -OSi(R⁵)₂O-, -C(O)(CR³R⁴)C(O)O-, or -OC(O)(CR³R⁴)C(O)-.

As used herein, an "aliphatic" group is a non-aromatic group in which carbon atoms are linked into chains and is either saturated or unsaturated.

The terms "alkyl" and "alkylene" refer to a straight or branched chain saturated hydrocarbon moiety. In one embodiment, the alkyl group is a straight chain saturated hydrocarbon. Unless otherwise specified, the "alkyl" or "alkylene" group contains from 1 to 24 carbon atoms. Representative saturated straight chain alkyl groups include methyl, ethyl, n-propyl, n-butyl, n-pentyl, and n-hexyl. Representative saturated branched alkyl groups include isopropyl, sec-butyl, isobutyl, tert-butyl, and isopentyl.

The term "alkenyl" refers to a straight or branched chain hydrocarbon moiety having one or more carbon-carbon double bonds. In one embodiment, the alkenyl group contains 1, 2, or 3 double bonds and is otherwise saturated. Unless otherwise specified, the "alkenyl" group contains from 2 to 24 carbon atoms. Alkenyl groups include both cis and trans isomers. Representative straight chain and branched alkenyl groups include ethylenyl, propylenyl, 1-butenyl, 2-butenyl, isobutylenyl, 1-pentenyl, 2-pentenyl, 3-methyl-1-butenyl, 2-methyl-2-butenyl, and 2,3-dimethyl-2-butenyl.

The term "alkynyl" refers to a straight or branched chain hydrocarbon moiety having one or more carbon-carbon triple bonds. Unless otherwise specified, the "alkynyl" group contains from 2 to 24 carbon atoms. Representative straight chain and branched alkynyl groups include acetylenyl, propynyl, 1-butynyl, 2-butynyl, 1-pentynyl, 2-pentynyl, and 3-methyl-1-butynyl.

Unless otherwise specified, the terms "branched alkyl", "branched alkenyl", and "branched alkynyl" refer to an alkyl, alkenyl, or alkynyl group in which one carbon atom in the group (1) is bound to at least three other carbon atoms and (2) is not a ring atom of a cyclic group. For example, a spirocyclic group in an alkyl, alkenyl, or alkynyl group is not considered a point of branching.

Unless otherwise specified, the term "acyl" refers to a carbonyl group substituted with hydrogen, alkyl, partially saturated or fully saturated cycloalkyl, partially saturated or fully saturated heterocycle, aryl, or heteroaryl. For example, acyl groups include groups such as (C₁-C₂₀)alkanoyl (e.g., formyl, acetyl, propionyl, butyryl, valeryl, caproyl, and t-butylacetyl), (C₃-C₂₀)cycloalkylcarbonyl (e.g., cyclopropylcarbonyl, cyclobutylcarbonyl, cyclopentylcarbonyl, and cyclohexylcarbonyl), heterocyclic carbonyl (e.g., pyrrolidinylcarbonyl, pyrrolid-2-one-5-carbonyl, piperidinylcarbonyl, piperazinylcarbonyl, and tetrahydrofuranylcarbonyl), aroyl (e.g., benzoyl) and heteroaroyl (e.g., thiophenyl-2-carbonyl, thiophenyl-3-carbonyl, furanyl-2-carbonyl, furanyl-3-carbonyl, 1H-pyrroyl-2-carbonyl, 1H-pyrroyl-3-carbonyl, and benzo[b]thiophenyl-2-carbonyl).

The term "aryl" refers to an aromatic monocyclic, bicyclic, or tricyclic hydrocarbon ring system. Unless otherwise specified, the "aryl" group contains from 6 to 14 carbon atoms. Examples of aryl moieties include, but are not limited to, phenyl, naphthyl, anthracenyl, and pyrenyl.

The terms "cycloalkyl" and "cycloalkylene" refer to a saturated monocyclic or bicyclic hydrocarbon moiety such as cyclopropyl, cyclobutyl, cyclopentyl, and cyclohexyl. Unless otherwise specified, the "cycloalkyl" or "cycloalkylene" group contains from 3 to 10 carbon atoms.

The term "cycloalkylalkyl" refers to a cycloalkyl group bound to an alkyl group, where the alkyl group is bound to the rest of the molecule.

The term "heterocycle" (or "heterocyclyl") refers to a non-aromatic 5- to 8-membered monocyclic, or 7- to 12-membered bicyclic, or 11- to 14-membered tricyclic ring system which is either saturated or unsaturated, and which contains from 1 to 3 heteroatoms if monocyclic, 1-6 heteroatoms if bicyclic, or 1-9 heteroatoms if tricyclic, independently selected from nitrogen, oxygen and sulfur, and wherein the nitrogen and sulfur heteroatoms may be optionally oxidized, and the nitrogen heteroatom may be optionally quaternized. For instance, the heterocycle may be a cycloalkoxy group. The heterocycle may be attached to the rest of the molecule via any heteroatom or carbon atom in the heterocycle. Heterocycles include, but are not limited to, morpholinyl, pyrrolidinonyl, pyrrolidinyl, piperidinyl, piperizynyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl, and tetrahydrothiopyranyl.

The term "heteroaryl" refers to an aromatic 5-8 membered monocyclic, 7-12 membered bicyclic, or 11-14 membered tricyclic ring system having 1-3 heteroatoms if monocyclic, 1-6 heteroatoms if bicyclic, or 1-9 heteroatoms if tricyclic, where the heteroatoms are selected from O, N, or S (e.g., carbon atoms and 1-3, 1-6, or 1-9 heteroatoms of N, O, or S if monocyclic, bicyclic, or tricyclic, respectively). The heteroaryl groups herein described may also contain fused rings that share a common carbon-carbon bond.

The term "substituted", unless otherwise indicated, refers to the replacement of one or more hydrogen radicals in a given structure with the radical of a specified substituent including, but not limited to: halo, alkyl, alkenyl, alkynyl, aryl, heterocyclyl, thiol, alkylthio, oxo, thioxy, arylthio, alkylthioalkyl, arylthioalkyl, alkylsulfonyl, alkylsulfonylalkyl, arylsulfonylalkyl, alkoxy, aryloxy, aralkoxy, aminocarbonyl, alkylaminocarbonyl, arylaminocarbonyl, alkoxycarbonyl, aryloxycarbonyl, haloalkyl, amino, trifluoromethyl, cyano, nitro, alkylamino, arylamino, alkylaminoalkyl, arylaminoalkyl, aminoalkylamino, hydroxy, alkoxyalkyl, carboxyalkyl, alkoxycarbonylalkyl, aminocarbonylalkyl, acyl, aralkoxycarbonyl, carboxylic acid, sulfonic acid, sulfonyl, phosphonic acid, aryl, heteroaryl, heterocyclic, and an aliphatic group. It is understood that the substituent may be further substituted. Exemplary substituents include amino, alkylamino, dialkylamino, and cyclic amino compounds.

The term "halogen" or "halo" refers to fluoro, chloro, bromo and iodo.

The following abbreviations may be used in this application:
DSPC: distearoylphosphatidylcholine; DPPC: 1,2-Dipalmitoyl-sn-glycero-3-phosphocholine; POPC: 1- palmitoyl-2-oleoyl-sn-phosphatidylcholine; DOPE: 1,2-dileoyl-sn-3-phosphoethanolamine; PEG-DMG generally refers to 1,2-dimyristoyl-sn-glycerol-methoxy polyethylene glycol (e.g., PEG 2000); TBDPSCl: tert-Butylchlorodiphenylsilane; DMAP: dimethylaminopyridine; HMPA: hexamethylphosphoramide; EDC: 1-ethyl-3-(3-dimethylaminopropyl) carbodiimide; DIPEA: diisopropylethylamine; DCM: dichloromethane; TEA: triethylamine; TBAF: tetrabutylammonium fluoride

Methods to prepare various organic groups and protective groups are known in the art and their use and modification is generally within the ability of one of skill in the art (*see, for example,* Green, T.W. et. al., Protective Groups in Organic Synthesis (1999); Stanley R. Sandler and Wolf Karo, Organic Functional Group Preparations (1989); Greg T. Hermanson, Bioconjugate Techniques (1996); and Leroy G. Wade, Compendium Of Organic Synthetic Methods (1980)). Briefly, protecting groups are any group that reduces or eliminates unwanted reactivity of a functional group. A protecting group can be added to a functional group to mask its reactivity during certain reactions and then removed to reveal the original functional group. In some embodiments an "alcohol protecting group" is used. An "alcohol protecting group" is any group which decreases or eliminates unwanted reactivity of an alcohol functional group. Protecting groups can be added and removed using techniques well known in the art.

The compounds may be prepared by at least one of the techniques described herein or known organic synthesis techniques.

### Examples

### Example 1: Synthesis of Ether Linked Lipids

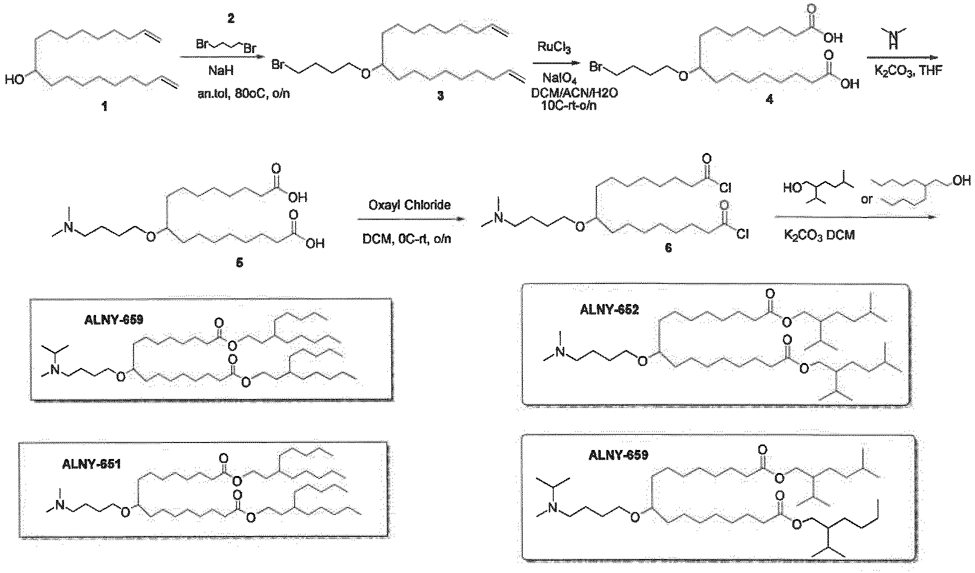

### 10-(4-bromobutoxy)nonadeca-1,18-diene 3

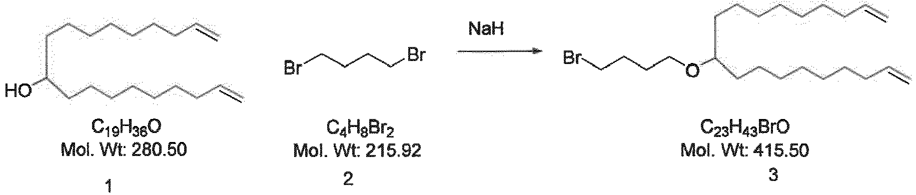

To a solution of nonadeca-1,18-dien-10-ol (30 mmol, 8.145 g) in toluene, added NaH (90 mmol, 60%, 3.6 g). The reaction mixture was allowed to stir at 80 °C for overnight. Cooled to room temperature, to the reaction was added neat 1,4-dibromobutane (300 mmol, 64.78 g). The reaction mixture was then heated at 90 °C for 6 hours. TLC showed the completion of reaction. Cooled to room temperature, the reaction mixture was quenched with ice, extracted with EtOAc, washed with brine. The organic layer was separated and dried over sodium sulfate. The organic layer was filtered and evaporated under reduced pressure. The residue was purified by ISCO (SiO2: 0-10% EtOAc / hexane) to provide the product as a colorless oil (12.46g). ¹H NMR (400 MHz, Chloroform-*d*) δ 5.81 (m, *J* = 16.9, 10.2, 6.7 Hz, 2H), 5.04 - 4.88 (m, 4H), 3.65 - 3.31 (m, 5H), 3.18 (p, *J* = 5.8 Hz, 1H), 2.09 - 1.98 (m, 5H), 2.02 - 1.90 (m, 3H), 1.75 - 1.63 (m, 2H), 1.51 - 1.19 (m, 21H).

### 9-(4-bromobutoxy)heptadecanedioic acid 4

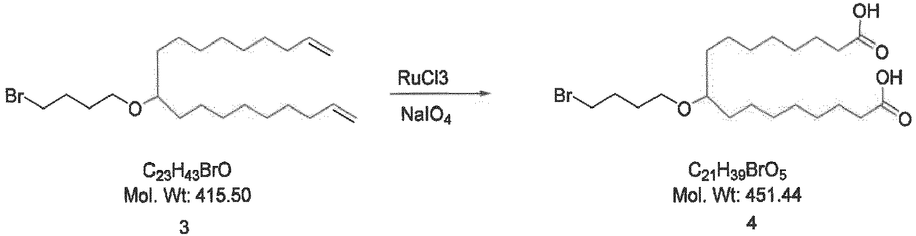

To a 1L RBF, added 10-(4-bromobutoxy)nonadeca-1,18-diene (30 mmol, 12.46 g) in 400 mL (1:1 volume) anhydrous DCM and Acetonitrile, RuCl3 (1.5 mmol, 311 mg) was added and cooled in an ice bath, a solution of NaIO4 (300 mmol, 64 g) in water was added slowly. The reaction mixture was allowed to stir at room temperature overnight. TLC showed completion of reaction. The reaction mixture was diluted with DCM and water. A few drops of 3% sodium sulfite were added for de-colorization. The organic layer was separated and dried over sodium sulfate and evaporated under vacuum. The residue was purified by ISCO (SiO2: 0-10% MeOH/DCM (+0.1% AcOH)) to provide product as a clolorless oil (7.9 g). MS: M+2=453.1. ¹H NMR (400 MHz, Chloroform-d) δ 3.44 (m, *J =* 12.3, 6.2 Hz, 4H), 3.18 (p, *J =* 5.8 Hz, 1H), 3.09 (q, *J* = 7.3 Hz, 3H), 2.32 (t, *J* = 7.4 Hz, 5H), 2.07 (s, 3H), 2.00 -1.88 (m, 2H), 1.73 - 1.54 (m, 7H), 1.47 - 1.39 (m, 4H), 1.47-1.30(m, 10H).

### 9-(4-(dimethylamino)butoxy)heptadecanedioic acid 5

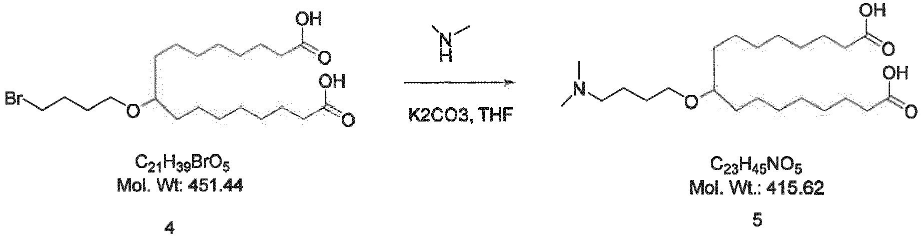

To a 150 mL pressure bottle, added 9-(4-bromobutoxy)heptadecanedioic acid (10 mmol, 4.51 g) in anhydrous THF (80 mL), followed by potassium carbonate (30 mmol, 4.14 g) and 2M solution of dimethylamine (50 mmol, 25 mL) in anhydrous THF. The reaction mixture was sealed and allowed to stir at 65°C overnight. TLC showed completion of reaction. The reaction mixture was acidified by adding 1N HCl to PH~4, the solution was extracted with DCM and water. The organic layer was separated and dried over sodium sulfate. The organic solution was filtered and evaporated under vacuum. The residue was co-evaporated with 3x20 mL toluene to provide product as a light- brown oil (3.64 g).

### 9-(4-(dimethylamino)butoxy)heptadecanedioic dichloride 6

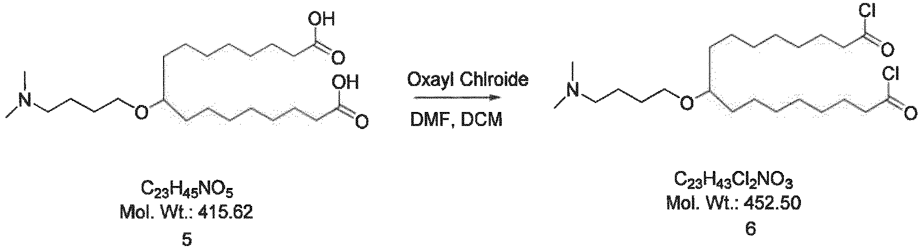

To a solution of 9-(4-(dimethylamino)butoxy)heptadecanedioic acid (8.75 mmol, 3.64 g) in anhydrous DCM(50 mL), was added 5 drops of anhydrous DMF at 0 °C, added oxayl chloride (52.5 mmol, 4.58 mL) dropwise. The reaction mixture was allowed to stir at room temperature overnight. Reaction was completed by checking MS when prepared sample in MeOH, the mass spectrum showed methyl ester mass. The reaction mixture was evaporated under vacuum and provided product as a red oil which was used as such in the next step. MS after treated wth MeOH (Methylester). MS: M+1 =444.3.

### bis(3-pentyloctyl) 9-(4-(dimethylamino)butoxy)heptadecanedioate (ALNY-651)

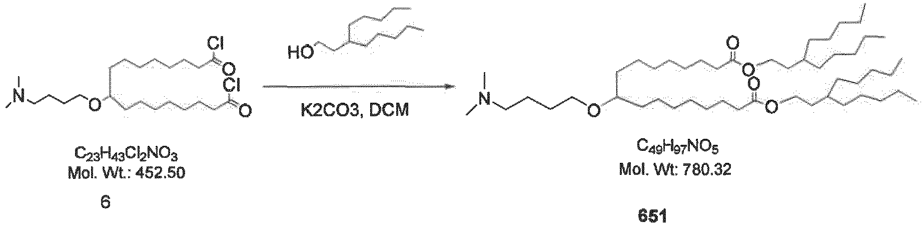

To a solution of 9-(4-(dimethylamino)butoxy)heptadecanedioyl dichloride (3.29 mmol, 1.49 g) in DCM (30mL) was added potassium carbonate (16.45 mmol, 2.27 g) followed by 3-pentyloctan-1-ol (13.16 mmol, 2.64 g). The reaction mixture was allowed to stir at room temperature overnight. TLC showed completion of reaction. The reaction was quenched with water and extracted with DCM. The organic layer was separated and dried over sodium sulfate and evaporated under vacuum. The residue was purified by ISCO (SiO2: 30-100% EtOAc/hexane with 3% TEA) to provide a colorless oil (0.538 g). MS: M+1=780.7. ¹H NMR (500 MHz, Chloroform-*d*) δ 4.07 (t, *J* = 7.1 Hz, 6H), 3.40 (s, 2H), 2.35 - 2.23 (m, 10H), 2.21 (s, 9H), 1.78 (p, *J* = 7.5 Hz, 2H), 1.57 (tt, *J* = 13.8, 6.9 Hz, 17H), 1.46 - 1.36 (m, 8H), 1.29 (t, *J* = 6.9 Hz, 26H), 0.88 (t, *J* = 7.1 Hz, 17H).

### bis(3-pentyloctyl) 9-(4-(isopropyl(methyl)amino)butoxy)heptadecanedioate (ALNY-659)

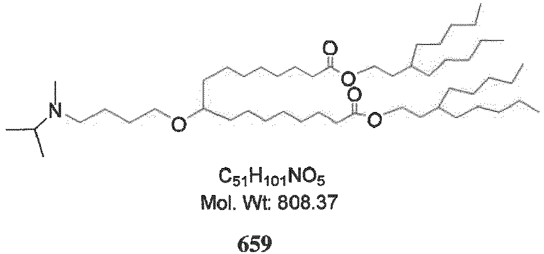

The lipid **659** was synthesized using a similar procedure. MS: M+1=809.7. ¹H NMR (500 MHz, Chloroform-d) δ 4.08 (t, *J* = 7.1 Hz, 6H), 3.40 (t, *J* = 5.9 Hz, 2H), 3.18 (s, 1H), 2.39 - 2.24 (m, 10H), 2.19 (s, 4H), 2.18(m, 1H), 1.65 - 1.46 (m, 19H), 1.40 (dq, *J* = 12.6, 7.5 Hz, 9H), 1.30 (d, *J* = 7.6 Hz, 25H), 0.98 (t, *J* = 5.9 Hz, 8H), 0.88 (t, *J* = 7.0 Hz, 17H).

### bis(2-isopropyl-5-methylhexyl) 9-(4-(dimethylamino)butoxy)heptadecanedioate (ALNY-652)

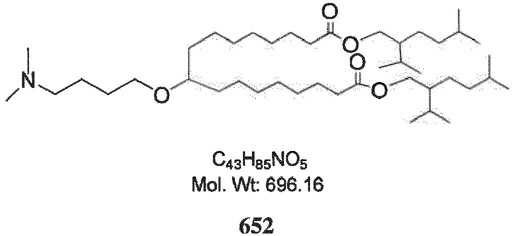

MS: M+1=697.6. ¹H NMR (400 MHz, Chloroform-*d*) δ 4.09 - 3.88 (m, 4H), 3.40 (s, 2H), 3.18(t, 1H) 2.33 - 2.23 (m, 6H), 2.20 (s, 6H) 1.76 (pd, *J* = 6.9, 4.7 Hz, 4H), 1.66 - 1.09 (m, 40H), 0.88 (td, *J* = 6.5, 5.9, 3.0 Hz, 22H). ¹³C NMR (126 MHz, Chloroform-*d*) δ 174.27 , 79.68 , 68.87 , 65.54 , 59.93 , 45.69 , 43.55 , 36.98 , 34.70 , 34.67 , 34.23 , 29.93 , 29.59 , 29.52 , 29.39 , 28.64 , 28.51 , 28.37 , 26.06 , 25.63 , 25.23 , 25.19 , 24.75 , 22.92 , 22.68 , 19.64.

### bis(2-isopropyl-5-methylhexyl) 9-(4-(isopropyl(methyl)amino)butoxy) heptadecanedioate (ALNY-657)

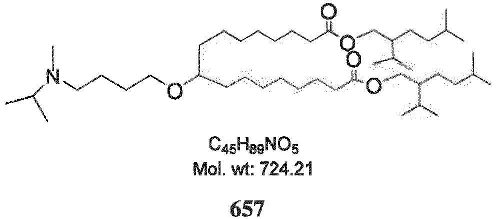

MS: M+1=725.0. ¹H NMR (400 MHz, Chloroform-*d*) δ 4.07 - 3.95 (m, 4H), 3.40 (s, 2H), 3.18 (s, 1H), 2.36 (s, 2H), 2.29 (t, *J* = 7.5 Hz, 4H), 2.19 (s, 3H), 1.76 (qd, *J* = 6.9, 4.7 Hz, 3H), 1.56 - 1.10 (m, 42H), 0.98 (dd, *J* = 6.6, 4.6 Hz, 6H), 0.88 (td, *J* = 6.5, 5.9, 3.0 Hz, 22H).

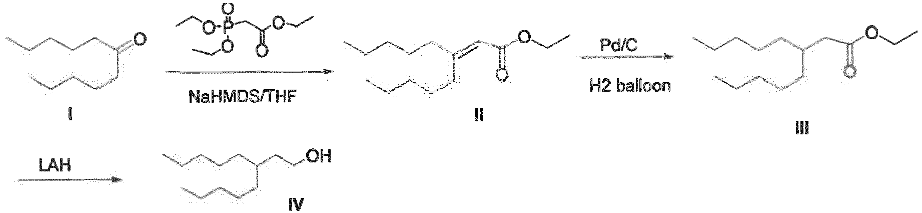

### Ethyl 3-pentyloct-2-enoate II

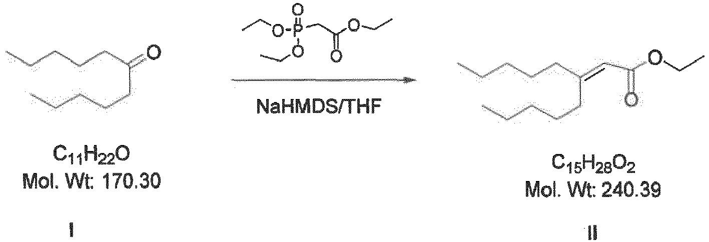

To a solution of triethyl phosphonacetate(100 mmol, 22.42 g) in anhydrous THF(50 mL) and at -10°C, added 1N NaHMDS (IN in THF, 100 mL) via addition funnel dropwise. After the addition, the reaction mixture was stirred at -10 °C for 1hr then 0 °C for 1hr. To this added 6-Undecanone (50 mmol, 8.52 g), warmed to room temperature and stirred at 45°C overnight. The reaction mixture was quenched with water, extracted with diethylether (2X100 mL), the combined ether was washed with brine. The organic layer was dried over sodium sulfate and filtered. The organic solution was evaporated. The residue was purified by ISCO (SiO2: 100% Hexane) to provide product as a clolorless oil (11.7 g). ¹H NMR (400 MHz, Chloroform-*d*) δ 5.61 (s, 1H), 4.13 (q, *J* = 7.1 Hz, 2H), 2.66 - 2.49 (m, 2H), 2.12 (td, *J* = 7.6, 1.2 Hz, 2H), 1.45 (ddt, *J* = 10.1, 7.3, 4.5 Hz, 4H), 1.38 - 1.21 (m, 10H), 0.94 - 0.84 (m, 7H).

### Ethyl 3-pentyloctanoate III

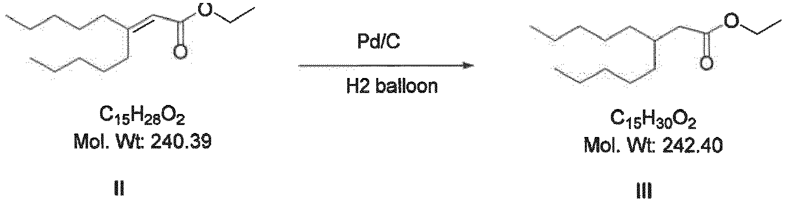

To a 200mL RBF, a solution of ethyl 3-pentyloct-2-enoate (48.6 mmol, 11.7 g) in ethyl acetate (100 mL) was added. The solution was purged the solution with argon 3 times, added Pd/C (5% wt), purged and backfilled with argon followed by hydrogen via hydrogen balloons. The reaction mixture was stirred with H2 balloon overnight. Filtered through celite, washed with thoroughly with ethyl acetate, the combined organic solution was evaporated under reduced pressure to provide product as a colorless oil (11 g). ¹H NMR (400 MHz, Chloroform-d) δ 4.12 (q, *J* = 7.1 Hz, 2H), 2.21 (d, *J* = 6.9 Hz, 2H), 1.84 (p, *J* = 5.8 Hz, 1H), 1.27 (dddd, *J* = 14.1, 11.3, 7.1, 4.8 Hz, 19H), 0.87 (t, *J* = 6.9 Hz, 6H).

### 3-Pentyloctan-1-ol IV

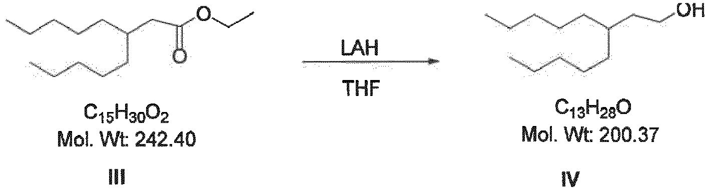

To a 250mL RBF, added a solution of ethyl 3-pentyloctanoate (45.4 mmol, 11 g) in anhydrous THF. Cooled in an ice bath and under argon, added LAH (2N THF, 91 mmol, 45.4 mL) slowly. Warmed to room temperature and allowed to stir at room temperature for 30min then 70°C for overnight. The reaction mixture was cooled to room temperature. At 0 °C, a solution of sat. sodium potassium tartrate tetrahydrate was added dropwise until no bubbling. The mixture was diluted with diethylether, filtered through celite, the combined ether was dried over sodium sulfate and filtered. The organic solution was evaporated under reduced pressure. The residue was purified by ISCO(SiO2: 0-10% EtOAc/Hexane) to provide product 8.26 g. ¹H NMR (400 MHz, Chloroform-*d*) δ 3.61 (t, *J* = 7.1 Hz, 2H), 2.08 (s, 1H), 1.49 (q, *J* = 6.9 Hz, 2H), 1.38 (p, *J* = 4.9, 4.0 Hz, 1H), 1.33 - 1.16 (m, 16H), 0.86 (t, *J =* 6.9 Hz, 6H).

### Example 2: Synthesis of Ester Containing Lipids

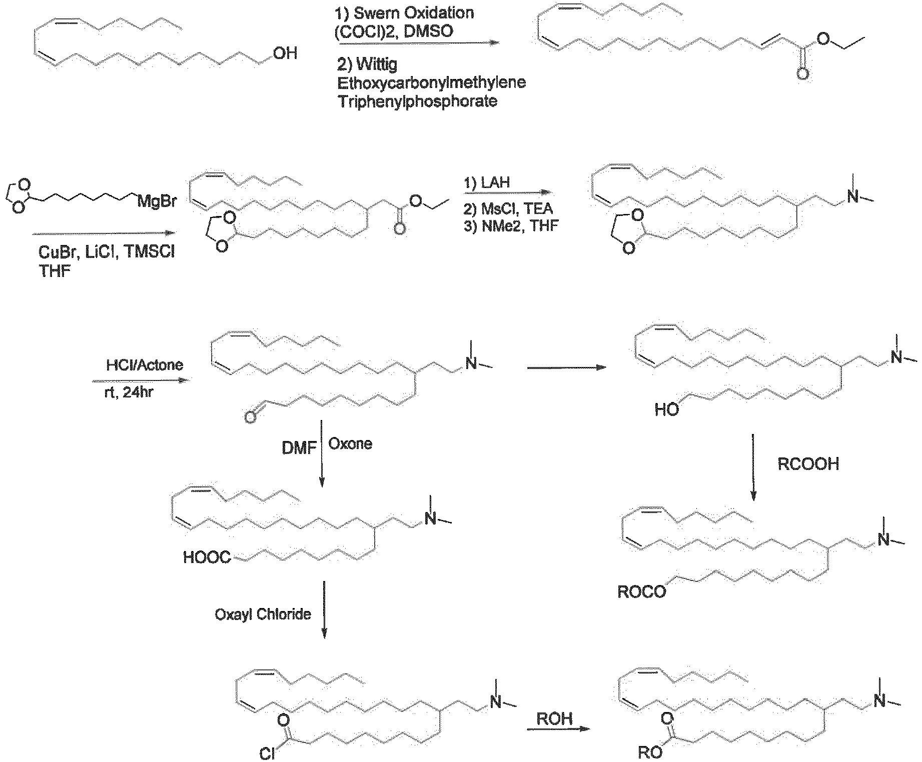

### Ethyl (2E,13Z,16Z)-docosa-2,13,16-trienoate

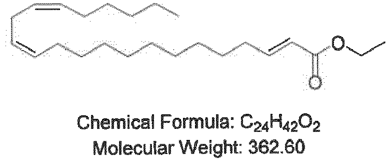

To a 1L RBF, added 750mL DCM and with stirring, added Oxayl chloride (239.1 mmol, 20.5 mL). Cooled to -78°C, DMSO (296.48 mmol, 23.16 g) was added dropwise followed by a solution of (11Z,14Z)-icosa-11,14-dien-1-ol (119.55 mmol, 35.21 g) in DCM (50 mL)slowly in a period of 5min. After stirring at -78°C for 30min, triethylamine (717.3 mmol, 100 mL) was added. The reaction mixture was warmed to room temperature and stirred for additional 50min. To the reaction was added ethoxycarbonylmethyl triphenyl phosphonate (135.47 mmol, 51 g) and allowed to stir at room temperature overnight. TLC showed the completion of reaction. The reaction mixture was quenched with 1N HCl, extracted with DCM, and washed with brine. The organic layer was separated and dried over sodium sulfate and evaporated under vacuum. The residue was purified by ISCO(SiO2: 0-30% EtOAc/Hexane) to provide product as a colorless oil ( 26.75 g). MS: M+1=363.3. ¹H NMR (400 MHz, Chloroform-*d*) δ 6.96 (dt, *J* = 15.7, 7.0 Hz, 1H), 5.81 (dt, *J* = 15.7, 1.6 Hz, 1H), 5.45 - 5.27 (m, 5H), 4.18 (q, *J* = 7.1 Hz, 2H), 2.77 (t, *J* = 6.5 Hz, 2H), 2.19 (qd, *J* = 7.2, 1.6 Hz, 2H), 2.04 (dd, *J* = 7.6, 6.1 Hz, 5H), 1.44 (q, *J =* 7.2 Hz, 2H), 1.41 - 1.28 (m, 22H).

### Ethyl (13Z,16Z)-3-(8-(1,3-dioxolan-2-yl)octyl)docosa-13,16-dienoate

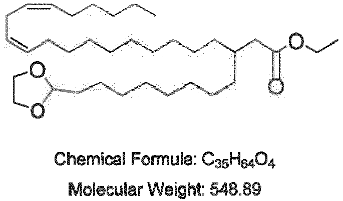

To a pre-dried 250mL RBF, added CuBr (0.76 mmol, 109 mg) and LiCl (1.52 mmol, 64.4 mg) in THF (10 mL) and stirred at room temperature for 10 min, cooled in an ice bath, added ethyl (2E,13Z,16Z)-docosa-2,13,16-trienoate (7.6 mmol, 2.8 g) in DCM (40 mL) followed by TMSCl (8.36 mmol, 908 mg). The reaction mixture was stirred at 0 °C for 15min before added (8-(1,3-dioxolan-2-yl)octyl)magnesium bromide (11.4 mmol, 3.3 g) in THF(16 mL). The reaction mixture was allowed to stir at 0 °C for 1.5hr, and then stirred at room temperature overnight. TLC showed completion of reaction, the reaction was quenched with sat. NH4Cl, diluted with diethylether (200 mL), extracted and separated, washed with brine. The organic layer was dried sodium sulfate and evaporated under vacuum. The residue was purified by ISCO (SiO2: 0-20% EtOAc and hexane) to provide product as a colorless oil (2.16 g). ¹H NMR (500 MHz, Chloroform-d) δ 5.35 (dtd, *J* = 18.0, 10.6, 5.4 Hz, 4H), 4.84 (t, *J* = 4.8 Hz, 2H), 4.12 (q, *J* = 7.2 Hz, 2H), 3.90 (d, *J* = 59.2 Hz, 6H), 3.56 (t, *J* = 6.8 Hz, 1H), 2.77 (t, *J* = 6.9 Hz, 2H), 2.21 (d, *J* = 6.9 Hz, 2H), 2.05 (q, *J* = 7.2 Hz, 4H), 1.83 (t, *J* = 7.3 Hz, 1H), 1.65 (dt, *J* = 8.9, 5.1 Hz, 3H), 1.51 (t, *J =* 6.8 Hz, 1H), 1.46 - 1.26 (m, 33H), 0.89 (t, *J* = 6.6 Hz, 3H).

### (13Z,16Z)-3-(8-(1,3-dioxolan-2-yl)octyl)docosa-13,16-dien-1-ol

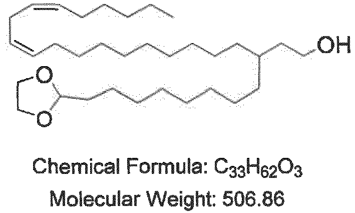

At 0 °C, to a solution of ethyl (13Z,16Z)-3-(8-(1,3-dioxolan-2-yl)octyl)docosa-13,16-dienoate (10.87 mmol, 5.97 g) in anhydrous THF(60mL) was added lithium aluminum hydride (1N in THF, 21.74 mmol, 22 mL) slowly. The reaction mixture was allowed to stir at 0°C for 15min then at room temperature for 4hrs. Cooled in an ice bath, sat. sodium potassium tartrate tetrahydrate solution was added dropwise until no bubbling. The reaction mixture was diluted with EtOAc and extracted. The organic layer was separated and dried over sodium sulfate and concentrated to a colorless oil. The residue was co-evaporated with toluene to remove any trace of water. Yield product 3.61 g.

### (13Z,16Z)-3-(8-(1,3-dioxolan-2-yl)octyl)docosa-13,16-dien-1-yl methanesulfonate

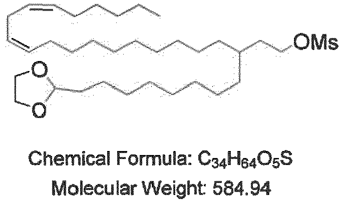

A solution of (13Z,16Z)-3-(8-(1,3-dioxolan-2-yl)octyl)docosa-13,16-dien-1-ol (7.132 mmol, 3.61 g) in anhydrous DCM (50 mL) at 0 °C was added trimethylamine (21.39 6mmol, 2.99 mL) followed by MsCl (14.264 mmol, 1.1 mL). The reaction mixture was allowed to stir at room temperature for 5hrs. TLC showed the completion of reaction. Quenched reaction with sat. NaHCO3, diluted and extracted with DCM. The organic layer was separated and dried over sodium sulfate. The organic solution was filtered and evaporated under reduced pressure to afford product (1.77 g). ¹H NMR (500 MHz, Chloroform-*d*) δ 5.35 (tdt, *J* = 13.8, 9.7, 5.0 Hz, 4H), 4.84 (t, *J* = 4.8 Hz, 1H), 4.24 (t, *J* = 7.0 Hz, 2H), 4.12 (q, *J* = 7.2 Hz, 3H), 3.96 (s, 2H), 3.90 - 3.77 (m, 2H), 3.00 (s, 3H), 2.77 (t, *J* = 6.8 Hz, 2H), 2.05 (d, *J* = 6.9 Hz, 9H), 1.77 - 1.59 (m, 5H), 1.52 - 1.15 (m, 28H), 0.89 (t, *J =* 6.8 Hz, 3H).

### (13Z,16Z)-3-(8-(1,3-dioxolan-2-yl)octyl)-N,N-dimethyldocosa-13,16-dien-1-amine

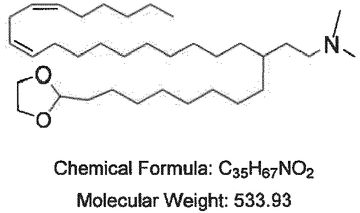

To a 150 mL pressure bottle, added a solution of (13Z,16Z)-3-(8-(1,3-dioxolan-2-yl)octyl)docosa-13,16-dien-1-yl methanesulfonate (2.37 mmol, 1.39 g)in THF (20mL), added dimethylamine (2M in THF, 14.22 mmol, 7.11 mL). The reaction mixture was sealed and heated at 65 °C for overnight. The reaction mixture was cooled at 0 °C and quenched with brine, diluted and extracted with EtOAc. The organic layer was separated and dried over sodium sulfate and evaporated under vacuum to a yellow oil. The residue was purified by ISCO (SiO2: 30-100% EtOAc/Hexane (3% trimethylamine added)) to provide product as a light yellow oil (1.38 g). ¹H NMR (400 MHz, Chloroform-*d*) δ 5.35 (qd, *J* = 10.9, 5.3 Hz, 4H), 4.05 - 3.76 (m, 4H), 2.77 (t, *J* = 6.5 Hz, 2H), 2.20 (s, 6H), 2.05 (q, *J* = 6.9 Hz, 4H), 1.70 - 1.56 (m, 2H), 1.49 - 1.21 (m, 42H), 0.89 (t, *J* = 6.8 Hz, 3H).

### (20Z,23Z)-10-(2-(dimethylamino)ethyl)nonacosa-20,23-dienal

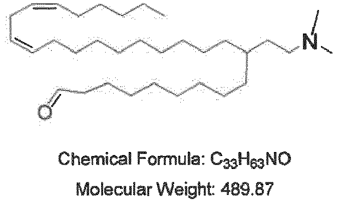

To a solution of (13Z,16Z)-3-(8-(1,3-dioxolan-2-yl)octyl)-N,N-dimethyldocosa-13,16-dien-1-amine (2.58 mmol, 1.38 g) in acetone(10mL) was added 1N HCl (7 mL). The reaction mixture was allowed to stir at room temperature overnight. TLC showed the completion of reaction, to the reaction mixture slowly added 20% K₂CO₃ aqueous solution until PH-10, diluted with EtOAc, the organic solution was extracted and separated, dried over sodium sulfate and concentrated to a colorless oil. The residue was dried under high vacuum and yield title product (1.1 g). ¹H NMR (500 MHz, Chloroform-d) δ 9.70(s, 1H), 5.36 (tdd, *J* = 17.9, 11.3, 7.1 Hz, 4H), 2.77 (t, *J* = 6.9 Hz, 2H), 2.42 (td, *J* = 7.4, 1.9 Hz, 2H), 2.21 (s, 8H), 2.05 (q, *J* = 7.1 Hz, 4H), 1.63 (p, *J* = 7.4 Hz, 2H), 1.46 - 1.14 (m, 36H), 0.89 (t, *J* = 6.7 Hz, 3H).

### (20Z,23Z)-10-(2-(dimethylamino)ethyl)nonacosa-20,23-dien-1-ol

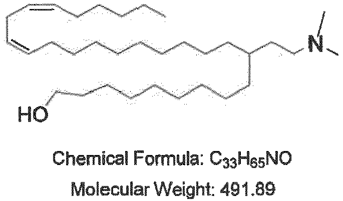

At 0 °C, to a solution of (20Z,23Z)-10-(2-(dimethylamino)ethyl)nonacosa-20,23-dienal (0.327 mmol, 160 mg) in anhydrous MeOH (4 mL) added NaBH4 (0.653 mmol, 25 mg). The reaction mixture was allowed at stir at room temperature overnight. TLC showed completion of reaction. The reaction mixture was quenched with sat NH4Cl, diluted with EtOAc and extracted. The organic layer was dried over sodium sulfate and concentrated to colorless oil. The residue was purified by ISCO (SiO2; 30-80% EtOAc/Hexane) to provide product 145 mg. ¹H NMR (500 MHz, Chloroform-*d*) δ 5.36 (dtd, *J =* 17.9, 11.2, 7.2 Hz, 4H), 4.12 (q, *J* = 7.2 Hz, 2H), 3.64 (t, *J* = 6.6 Hz, 2H), 2.77 (t, *J* = 6.9 Hz, 2H), 2.21 (s, 8H), 2.05 (d, *J* = 6.5 Hz, 7H), 1.47 - 1.23 (m, 34H), 0.88 (tt, *J* = 9.9, 5.2 Hz, 6H).

### (20Z,23Z)-10-(2-(dimethylamino)ethyl)nonacosa-20,23-dien-1-yl 2-ethylhexanoate (ALNY-654)

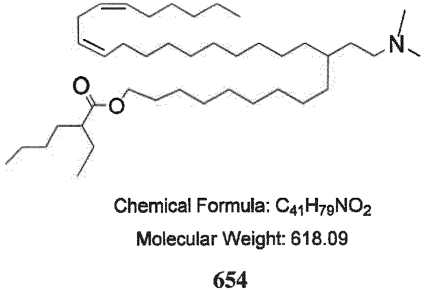

To a 100 mL RBF, added a solution of (20Z,23Z)-10-(2-(dimethylamino)ethyl)nonacosa-20,23-dien-1-ol (0.295 mmol, 145 mg) in anhydrous DCM (3 mL), to this added K₂CO₃ (0.885 mmol, 122 mg), cooled in an ice bath, added 2-ethylhexanoyl chloride (0.442 mmol, 71.9 mg). The reaction mixture was allowed to stir at room temperature overnight. Quenched the reaction with water, and extracted with DCM. The organic layer was separated and dried over sodium sulfate. The combined organic solution was evaporated under reduced pressure. The residue was purified by ISCO (SiO2: 30-80% EtOAc and Hexane (3% TEA added)) to provide product as a colorless oil (78.2 mg). MS: M+1=618.7. ¹H NMR (500 MHz, Chloroform-*d*) δ 5.36 (tdd, *J* = 17.8, 11.4, 7.1 Hz, 4H), 4.07 (t, *J* = 6.7 Hz, 2H), 2.78 (t, *J* = 6.9 Hz, 2H), 2.21 (s, 12H), 2.05 (q, *J* = 7.0 Hz, 5H), 1.44 - 1.14 (m, 43H), 0.88 (ddd, *J* = 7.3, 5.5, 3.3 Hz, 11H).

### 9-Bromononanal

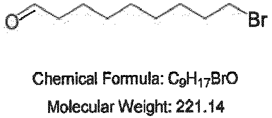

To a 200mL RBF, added a suspension of pyridinium chlorochromate (30 mmol, 6.47 g) and MgSO4 (2.5 g) in DCM (50 mL). Cooled in an ice bath, added a solution of 9-bromo-i-nonanol (20 mmol, 4.46 g) in anhydrous DCM (10 mL) slowly. Allowed to stir at 0 °C for 1hr then rt for 1hr. Added diethylether (2x50 mL) to the reaction mixture and stirred vigorously for 5min, filtered through celite. The residue was washed with 3x50mL of ether. The combined ether solution was concentrated to product as a brown oil (4.06 g). ¹H NMR (400 MHz, Chloroform-*d*) δ 9.76 (d, *J* = 1.8 Hz, 1H), 3.40 (t, *J* = 6.8 Hz, 3H), 2.43 (td, *J* = 7.3, 1.8 Hz, 2H), 1.84 (p, *J* = 6.9 Hz, 3H), 1.62 (p, *J* = 7.2 Hz, 3H), 1.42 (dq, *J* = 13.0, 6.9 Hz, 5H).

### 2-(8-bromooctyl)-1,3-dioxolane

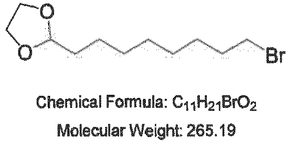

To 250mL RBF, added a solution of 9-bromononanal (18.09 mmol, 4.0 g) in anhydrous toluene (60 mL), added p-TsOH (0.946 mmol, 180 mg), followed by ethylene glycol (53.97 mmol, 3.35 g). The reaction was equipped with dean stark and condenser and refluxed at 130°C overnight. The reaction mixture was quenched with sat. NaHCO3, extracted with EtOAc, washed with brine. The organic solution was dried over sodium sulfate and evaporated under reduced pressure to colorless oil. The residue was purified by ISCO (SiO2: 0-20% EtOAC/Hexane) to provide product 3.12 g. ¹H NMR (400 MHz, Chloroform-*d*) δ 4.84 (t, *J* = 4.8 Hz, 1H), 4.07 - 3.76 (m, 4H), 3.40 (t, *J* = 6.9 Hz, 2H), 1.84 (p, *J* = 7.0 Hz, 2H), 1.70 - 1.59 (m, 2H), 1.47 - 1.26 (m, 10H).

### (8-(1,3-dioxolan-2-yl)octyl)magnesium bromide

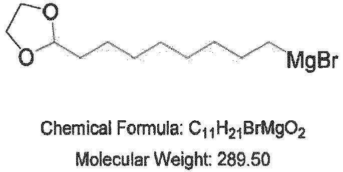

To a 100mL 2-neck RBF, added pre-activated Mg-turnings (12.67 mmol, 308 mg) in THF (16 mL), added a solution of 2-(8-bromooctyl)-1,3-dioxolane (6.34 mmol, 1.68 g) in THF followed by 1-2 crystals of Iodine. The reaction was allowed to stir at 60°C for 35min. The solution was decolorized. Cooled to room temperature and used as is.

### (20Z,23Z)-10-(2-(dimethylamino)ethyl)nonacosa-20,23-dienoic acid

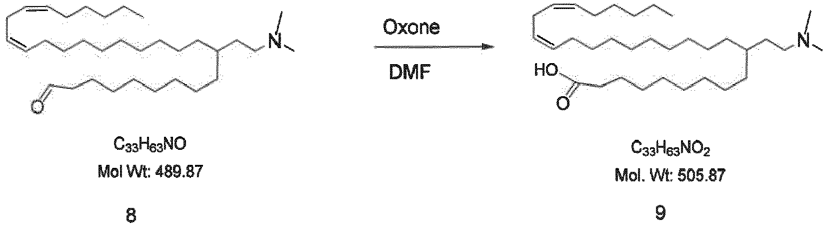

To a solution of (20Z,23Z)-10-(2-(dimethylamino)ethyl)nonacosa-20,23-dienal (1.45 mmol, 711 mg) in anhydrous DMF (6 mL) was added Oxone (1.45 mmol, 892.3 mg). The reaction mixture was allowed to stir at room temperature overnight. TLC showed completion of reaction, water was added to quench the reaction, diluted and extracted with EtOAc, washed with brine. The organic layer was separated and dried over sodium sulfate. The combined organic layer was evaporated under reduced pressure. The residue was co-evaporated with toluene to remove any water. Yield product (733 mg). MS: M+1=506.5.

### (20Z,23Z)-10-(2-(dimethylamino)ethyl)nonacosa-20,23-dienoyl chloride

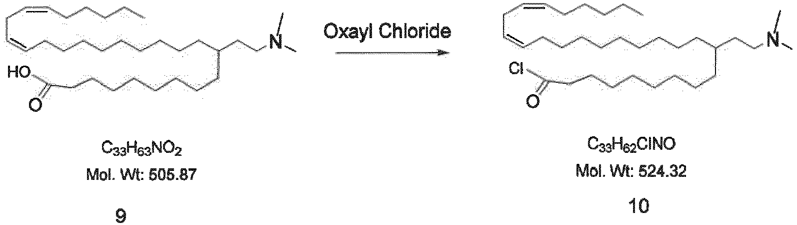

At 0 °C, to a solution of (20Z,23Z)-10-(2-(dimethylamino)ethyl)nonacosa-20,23-dienoic acid (1.45 mmol, 733 mg) in anhydrous DCM (8 mL) was added 2 drops of anhydrous DMF followed by neat oxayl chloride dropwise. The reaction mixture was stirred at room temperature overnight. TLC showed completion of reaction (small sample of reaction mixture was treated with MeOH). The reaction solution was concentrated to dark red oil.

### 3-pentyloctyl (20Z,23Z)-10-(2-(dimethylamino)ethyl)nonacosa-20,23-dienoate

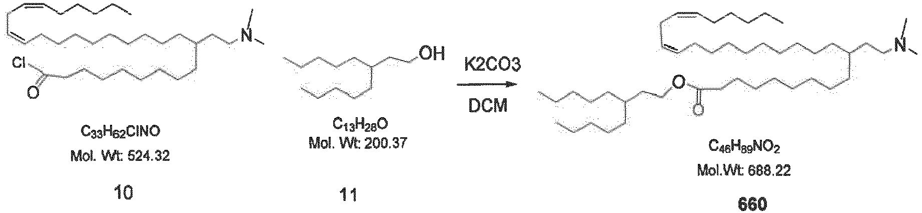

To a solution of (20Z,23Z)-10-(2-(dimethylamino)ethyl)nonacosa-20,23-dienoyl chloride (0.944 mmol, 495 mg) in DCM(10 mL), K₂CO₃ (3.30 mmol, 457 mg) was added followed by a solution of 3-pentyloctan-1-ol (1.89 mmol, 378.1 mg) in DCM(2 mL). The reaction mixture was stirred at room temperature overnight. TLC showed completion of reaction. The reaction mixture was quenched with water, diluted with DCM and extracted. The organic layer was separated and dried over sodium sulfate and the organic solution was filtered and evaporated under reduced pressure. The residue was purified by ISCO (SiO2: 0-40% EtOAc and Hexane) to provide product (195.6 mg). MS: M+1=689.7. ¹H NMR (400 MHz, Chloroform-d) δ 5.35 (qd, *J* = 10.9, 5.6 Hz, 4H), 4.10 (dt, *J* = 20.3, 7.1 Hz, 4H), 2.77 (t, *J* = 6.5 Hz, 2H), 2.31 - 2.24 (m, 3H), 2.22 (s, 7H), 2.05 (d, *J* = 5.5 Hz, 6H), 1.44 - 1.15 (m, 55H), 0.89 (td, *J* = 7.0, 2.7 Hz, 9H).

### 2-Isopropyl-5-methylhexyl (20Z,23Z)-10-(2-(dimethylamino)ethyl)nonacosa-20,23-dienoate

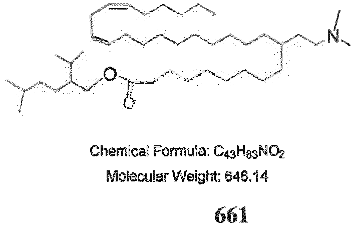

MS: M+1=647.6. ¹H NMR (400 MHz, Chloroform-*d*) δ 5.35 (tq, *J* = 10.6, 6.3, 5.4 Hz, 4H), 4.14 - 3.91 (m, 3H), 2.77 (t, *J* = 6.5 Hz, 2H), 2.29 (t, *J* = 7.5 Hz, 2H), 2.22 (s, 8H), 2.05 (q, *J* = 6.9 Hz, 4H), 1.77 (pd, *J* = 6.9, 4.7 Hz, 1H), 1.47 - 1.14 (m, 42H), 0.88 (td, *J =* 6.2, 3.1 Hz, 17H).

### 3-Pentyloctyl (20Z,23Z)-10-(2-(isopropyl(methyl)amino)ethyl)nonacosa-20,23-dienoate

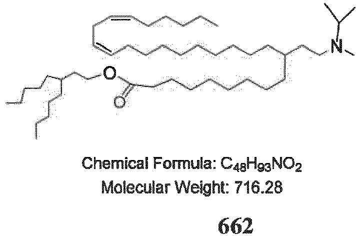

MS: M+1=717.8. ¹H NMR (400 MHz, Chloroform-*d*) δ 5.36 (dq, *J* = 12.4, 6.7, 5.6 Hz, 4H), 4.20 - 3.99 (m, 12H), 2.80 (dt, *J* = 18.0, 6.5 Hz, 2H), 2.30 (dt, *J* = 21.5, 7.6 Hz, 4H), 2.19 (s, 3H), 2.04 (s, 16H), 1.34 - 1.25 (m, 43H), 1.00 (d, *J* = 6.5 Hz, 5H), 0.88 (h, *J* = 2.9, 2.3 Hz, 9H).

### 2-Isopropyl-5-methylhexyl (20Z,23Z)-10-(2-(isopropyl(methyl)amino)ethyl) nonacosa-20,23-dienoate

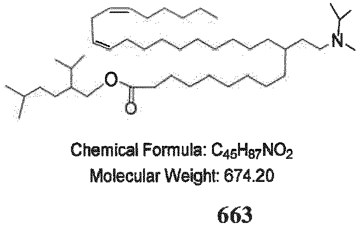

MS: M+1=675.7. ¹H NMR (400 MHz, Chloroform-*d*) δ 5.45 - 5.24 (m, 4H), 4.12 (q, *J* = 7.2 Hz, 9H), 4.07 - 3.94 (m, 2H), 2.80 (dt, *J* = 21.8, 6.5 Hz, 3H), 2.31 (dt, *J* = 20.1, 7.7 Hz, 4H), 2.19 (s, 3H), 2.04 (s, 16H), 1.26 (t, *J* = 7.1 Hz, 21H), 1.00 (d, *J* = 6.5 Hz, 6H), 0.88 (ddt, *J* = 9.3, 6.3, 2.7 Hz, 19H).

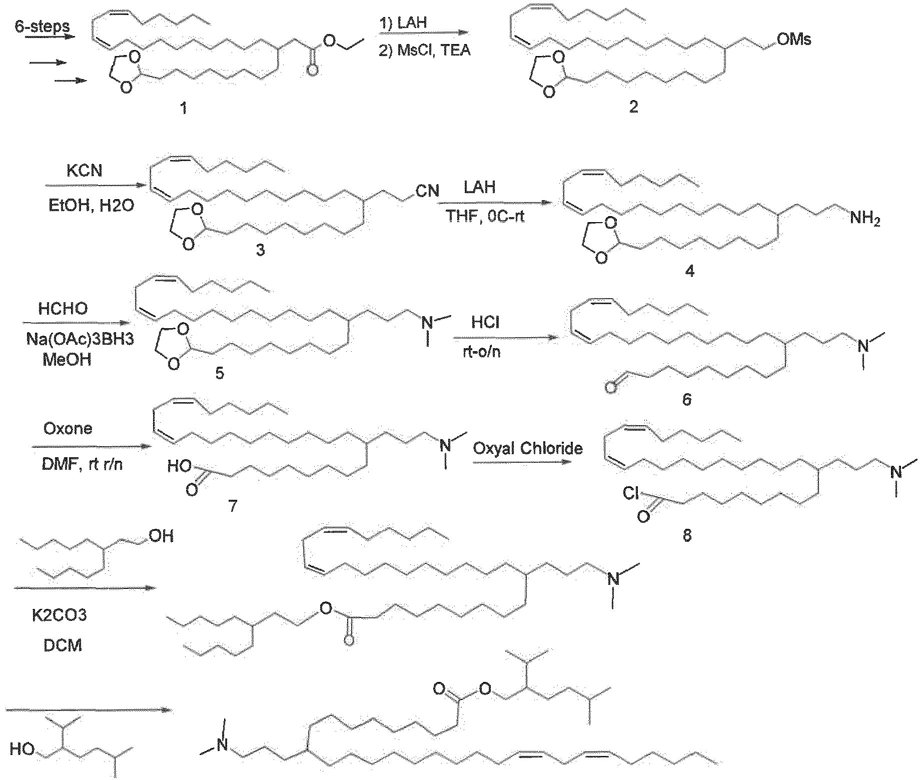

### (14Z,17Z)-4-(8-(1,3-dioxolan-2-yl)octyl)tricosa-14,17-dienenitrile

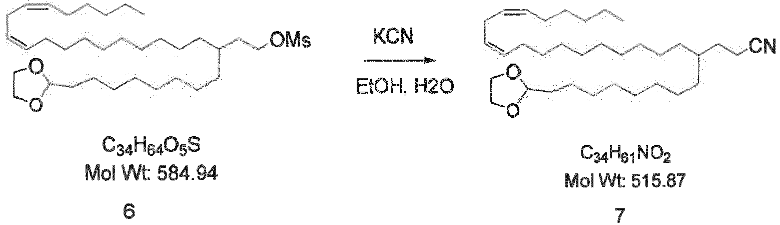

To a solution of (13Z,16Z)-3-(8-(1,3-dioxolan-2-yl)octyl)docosa-13,16-dien-1-yl methanesulfonate (5.13 mmol, 3 g) in EtOH (200 proof) was added aqueous solution of KCN(15.39 mmol, 1.02 g), the reaction was refluxed at 80 °C overnight. The condenser was connected to a KOH trapper to neutralize the HCN released. The reaction mixture was quenched with sat. NaHCO3, extracted with ether, washed with brine. The organic layer was dried over sodium sulfate and filtered. The organic solution was evaporated under reduced pressure to a colorless oil. The residue was purified by ISCO (SiO2: 0-20% EtOAc/Hexane) to provide product (2.08 g). MS: ES⁻(M-1=514.3). ¹H NMR (400 MHz, Chloroform-*d*) δ 5.34 (ddd, *J* = 17.5, 11.3, 6.6 Hz, 4H), 4.84 (t, *J* = 4.8 Hz, 1H), 4.05 - 3.73 (m, 4H), 2.77 (t, *J* = 6.5 Hz, 2H), 2.31 (t, *J* = 7.6 Hz, 2H), 2.14 - 1.93 (m, 4H), 1.70 - 1.57 (m, 4H), 1.56 (s, 2H), 1.47 - 1.18 (m, 34H), 0.89 (t, *J* = 6.8 Hz, 3H).

### (14Z,17Z)-4-(8-(1,3-dioxolan-2-yl)octyl)tricosa-14,17-dien-1-amine

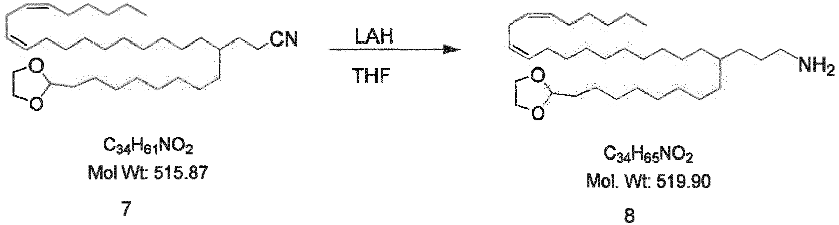

A solution of (14Z,17Z)-4-(8-(1,3-dioxolan-2-yl)octyl)tricosa-14,17-dienenitrile (4.03 mmol, 2.08 g) in anhydrous THF at 0 °C, added 1N lithium aluminum hydrate in THF (8.06 mmol, 8.06 mL). The reaction mixture was stirred at 0 °C for 25min then at room temperature for 4 hours. Cooled in an ice bath, to the reaction mixture was added a sat. sodium potassium tartrate tetrahydrate solution dropwise until no bubbling and a ppt was formed. The ppt was filtered through celite. The solution was extracted with EtoAC and brine. The organic layer was separated and dried over sodium sulfate and filtered. The organic solution was evaporated under reduced pressure to a colorless oil. The residue was dried in the high vacuum overnight yield 2 g product. MS: M+1=520.5. ¹H NMR (400 MHz, Chloroform-*d*) δ 5.35 (qd, *J* = 10.8, 5.3 Hz, 4H), 4.84 (t, *J* = 4.9 Hz, 1H), 4.04 - 3.80 (m, 4H), 2.77 (t, *J* = 6.5 Hz, 2H), 2.66 (t, *J* = 7.1 Hz, 1H), 2.11 - 1.98 (m, 4H), 1.71 - 1.59 (m, 3H), 1.47 - 1.06 (m, 43H), 0.89 (t, *J* = 6.8 Hz, 3H).

### (14Z,17Z)-4-(8-(1,3-dioxolan-2-yl)octyl)-N,N-dimethyltricosa-14,17-dien-1-amine

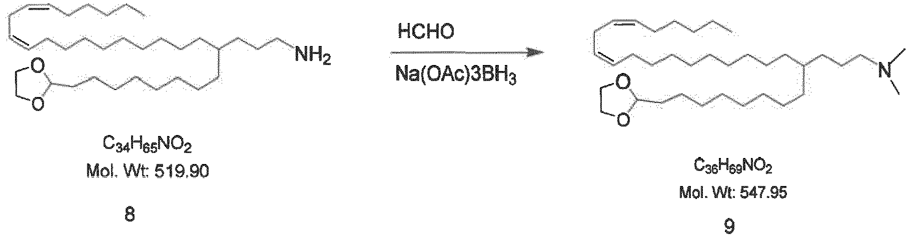

To a solution of (14Z,17Z)-4-(8-(1,3-dioxolan-2-yl)octyl)tricosa-14,17-dien-1-amine (3.85 mmol, 2 g) in anhydrous MeOH (20 mL) was added formal aldehyde (30% in water, 10 mL) followed by Na(OAC)3BH3(15.39 mmol, 3.26 g). The reaction mixture was stirred at room temperature overnight. TLC showed completion of reaction. The reaction mixture was quenched with 1N NaOH, extracted with EtOAc, washed with brine. The combined organic layer was dried over sodium sulfate and filtered. The organic solution was evaporated under reduced pressure to a colorless oil. The residue was purified by ISCO (SiO2, 0-80% EtOAc/Hexane) to provide product (2.3 g). MS: M+1=548.5. ¹H NMR (400 MHz, Chloroform-*d*) δ 5.49 - 5.23 (m, 4H), 4.96 - 4.67 (m, 2H), 4.03 - 3.90 (m, 2H), 3.90 - 3.76 (m, 2H), 3.39 (s, 1H), 2.77 (t, *J* = 6.5 Hz, 2H), 2.25 (s, 1H), 2.16 (s, 1H), 2.11 (s, 1H), 2.09 - 1.99 (m, 5H), 1.78 (s, 1H), 1.69 - 1.61 (m, 2H), 1.59 (s, 5H), 1.46 - 1.14 (m, 37H), 0.89 (t, *J* = 6.8 Hz, 3H).

### (20Z,23Z)-10-(3-(dimethylamino)propyl)nonacosa-20,23-dienal

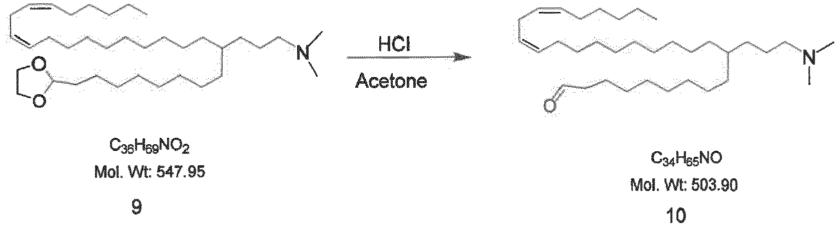

To a solution of (14Z,17Z)-4-(8-(1,3-dioxolan-2-yl)octyl)-N,N-dimethyltricosa-14,17-dien-1-amine (3.65 mmol, 2.3 g) in Acetone (25 mL), added 1N HCl (10 mL). The reaction mixture was stirred at room temperature overnight. The reaction mixture was quenched with 20% K₂CO₃ aqueous solution, extracted with EtOAc, washed with brine. The organic layer was dried over sodium sulfate and filtered. The organic solution was evaporated under reduced pressure to a colorless oil. Yield product 1.88 g. ¹H NMR (400 MHz, Chloroform-*d*) δ 9.76 (d, *J* = 1.9 Hz, 1H), 5.36 (tq, *J* = 11.0, 5.6, 4.3 Hz, 4H), 4.12 (q, *J* = 7.1 Hz, 1H), 2.77 (t, *J* = 6.5 Hz, 2H), 2.41 (td, *J* = 7.4, 1.9 Hz, 2H), 2.22 (s, 4H), 2.10 - 1.99 (m, 6H), 1.68 - 1.55 (m, 2H), 1.47 - 1.10 (m, 40H), 0.89 (t, *J* = 6.8 Hz, 3H).

### (20Z,23Z)-10-(3-(dimethylamino)propyl)nonacosa-20,23-dienoic acid

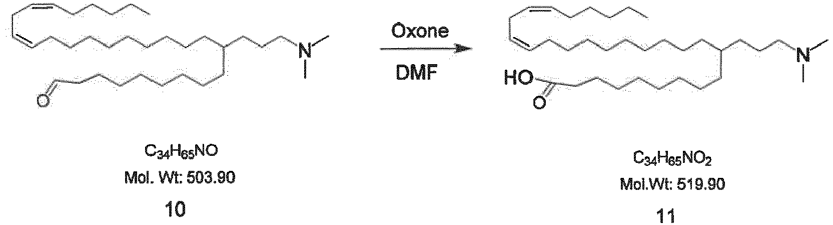

To a solution of (20Z,23Z)-10-(3-(dimethylamino)propyl)nonacosa-20,23-dienal (3.78 mmol, 1.88 g) in anhydrous DMF(15 mL) was added Oxone(3.72 mmol, 2.29 g). The reaction mixture was stirred at room temperature overnight. TLC showed completion of reaction. The reaction mixture was quenched with brine, diluted with EtOAc and extracted. The organic layer was separated and dried over sodium sulfate and filtered. The organic solution was evaporated under reduced pressure to a colorless oil. The residue was co-evaporated with toluene 2x20mL to remove any trace of water. Yield 1.99 g product. MS: M+1=520.5

### (20Z,23Z)-10-(3-(dimethylamino)propyl)nonacosa-20,23-dienoyl chloride

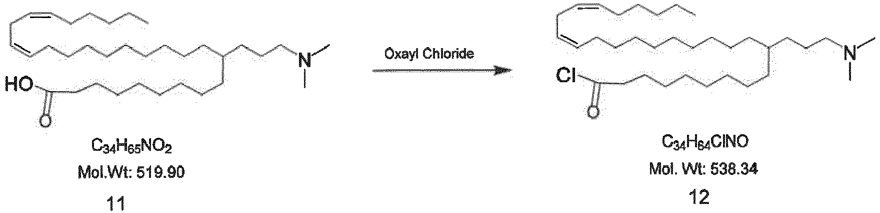

To a solution of (20Z,23Z)-10-(3-(dimethylamino)propyl)nonacosa-20,23-dienoic acid (3.82 mmol, 1.99 g) in anhydrous DCM at 0°C, added 2 drops of anhydrous DMF followed by oxayl chloride(9.57 mmol, 833 uL) slowly. The reaction mixture was stirred at room temperature overnight. TLC showed completion of reaction. The reaction mixture was concentrated to provide product as brownish red oil.

### 3-pentyloctyl (20Z,23Z)-10-(3-(dimethylamino)propyl)nonacosa-20,23-dienoate

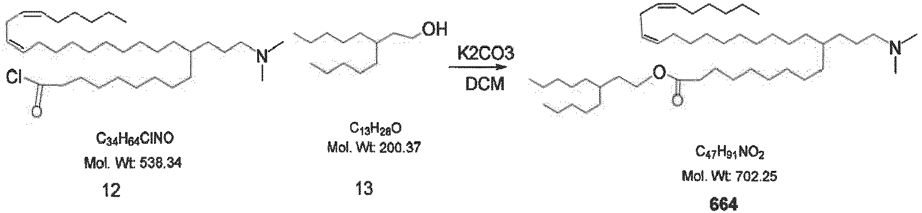

To a solution of (20Z,23Z)-10-(3-(dimethylamino)propyl)nonacosa-20,23-dienoyl chloride (2.22 mmol, 1.2 g) in anhydrous DCM(10 mL), added K₂CO₃ (7.77 mmol, 1.07 g) followed by a solution of 3-pentyloctan-1-ol (4.46 mmol, 0.893 g)in anhydrous DCM. The reaction mixture was stirred at room temperature overnight. TLC showed completion of reaction. The reaction mixture was quenched with brine, and diluted with DCM. The organic layer was extracted, separated, and dried over sodium sulfate. The organic solution was filtered and evaporated under reduced pressure. The residue was purified by ISCO (SiO₂: 0-40% EtOAc and hexane) to provide product as a brown-red oil (99.5 mg). MS: M+1=703.7. ¹H NMR (400 MHz, Chloroform-*d*) δ 5.36 (tq, *J* = 10.9, 5.5, 4.2 Hz, 4H), 4.08 (t, *J* = 7.1 Hz, 2H), 2.77 (t, *J* = 6.5 Hz, 2H), 2.28 (t, *J* = 7.5 Hz, 2H), 2.21 (s, 6H), 2.05 (q, *J* = 6.9 Hz, 4H), 1.57 (q, *J* = 6.8 Hz, 10H), 1.44 - 1.14 (m, 52H), 0.89 (td, *J* = 6.9, 2.6 Hz, 8H).

### 2-Isopropyl-5-methylhexyl (20Z,23Z)-10-(3-(dimethylamino)propyl)nonacosa-24,23-dienoate

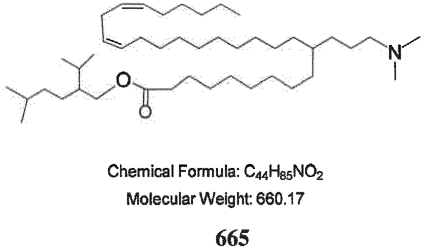

MS: M+1=661.7. ¹H NMR (400 MHz, Chloroform-*d*) δ 5.35 (tq, *J* = 11.1, 7.0, 5.6 Hz, 4H), 4.15 - 3.88 (m, 2H), 2.77 (t, *J* = 6.5 Hz, 2H), 2.42 - 2.16 (m, 9H), 2.05 (q, *J* = 6.9 Hz, 4H), 1.77 (pd, *J* = 6.9, 4.6 Hz, 1H), 1.61 (p, *J* = 7.1 Hz, 2H), 1.53 - 1.03 (m, 46H), 0.88 (td, *J =* 6.2, 3.1 Hz, 14H).

### Example 3: Preparation of Lipid Nanoparticles

The cationic lipids described herein are used to formulate liposomes containing the AD-1661 duplex (shown in the table below) using an in-line mixing method as described in International Publication No. WO 2010/088537. Unless indicated otherwise, the lipid nanoparticles had the formulation shown in the table below.

| Component | Mole Percentage (Based on 100% of the lipid components in the LNP) |
|---|---|
| Cationic lipid | 50% |
| Distearoylphosphatidylcholine (DSPC) | 10% |
| Cholesterol | 38.5% |
| 1-(monomethoxy-polyethyleneglycol)-2,3-dimyristoylglycerol (PEG-DMG) (with an average PEG molecular weight of 2000) | 1.5% |
| siRNA (AD-1661) | - |

Formulations having a formulation of "58/10/30/2" had the formulation shown in the table below.

| Component | Mole Percentage (Based on 100% of the lipid components in the LNP) |
|---|---|
| Cationic lipid | 58% |
| DSPC | 10% |
| Cholesterol | 30% |
| PEG-DMG (with an average PEG molecular weight of 2000) | 2% |
| siRNA (AD-1661) | - |

Formulations having a formulation of "55/10/33/2" had the formulation shown in the table below.

| Component | Mole Percentage (Based on 100% of the lipid components in the LNP) |
|---|---|
| Cationic lipid | 55% |
| DSPC | 10% |
| Cholesterol | 33% |
| PEG-DMG (with an average PEG molecular weight of 2000) | 2% |
| siRNA (AD-1661) | - |

Formulations having a formulation of "50/10/38/2" contain 50 mol % cationic lipid, 10 mol % DSPC, 38 mol % cholesterol, and 2 mol % PEG-DMG.

The siRNA AD-1661 duplex has the sequence shown below.

| Duplex | Sequence 5'-3' | SEQ ID NO: | Target |
|---|---|---|---|
| AD-1661 | GGAfUfCAfUfCfUfCAAGfUfCfUfUAfCdTsdT | 1 | FVII |
| | GfUAAGAfCfUfUGAGAfUGAfUfCfCdTsdT | 2 | |

Lower case is 2'OMe modification and Nf is a 2'F modified nucleobase, dT is deoxythymidine, s is phosphothioate

The lipid nanoparticles were prepared as follows. Cationic lipid, DSPC, cholesterol, and PEG-DMG in the ratio recited in the table above were solubilized in ethanol at a total lipid concentration of 25 mg/mL.

A siRNA stock solution was prepared by solubilizing the siRNA AD-1661 in a low pH acetate or citrate buffer (pH=4) at 0.8 mg/mL.

The stock solutions should be completely clear and the lipids should be completely solubilized before combining with the siRNA. Therefore, if it was determined appropriate, the stock solutions were heated to completely solubilize the lipids.

The individual stock solutions were combined by pumping each solution to a T-junction (i.e., by in-line mixing). Specifically, the ethanol solution (at 5 ml/min, via 0.01 in. PEEK tube) and aqueous buffer solution (at 15 mL/min, via 0.02 in. PEEK tube) were mixed through a T-junction (PEEK Tee body, IDEX).

After the T-junction a single tubing is placed where the combined stream will emit. Ethanol is removed and exchanged for PBS by dialysis. The lipid formulations are then concentrated using centrifugation or diafiltration to an appropriate working concentration.

Lipid nanoparticles containing the cationic lipids listed in the table in Example 36 were prepared as described above.

### Example 4: Efficacy of Lipid Nanoparticles

Factor VII (FVII), a prominent protein in the coagulation cascade, is synthesized in the liver (hepatocytes) and secreted into the plasma. FVII levels in plasma can be determined by a simple, plate-based colorimetric assay. As such, FVII represents a convenient model for determining siRNA-mediated downregulation of hepatocyte-derived proteins.

Test formulations of the lipid nanoparticles prepared in Example 3 were assessed for their FVII knockdown in female 7-9 week old, 15-25g, female C57Bl/6 mice at 0.01 and 0.03 mg/kg with 3 mice per treatment group. All studies included animals receiving either phosphate-buffered saline (PBS, control group) or a benchmark formulation. Formulations were diluted to the appropriate concentration in PBS immediately prior to testing. Mice were weighed and the appropriate dosing volumes calculated (10 µl/g bodyweight). Test and benchmark formulations as well as PBS (for control animals) were administered intravenously via the lateral tail vein. Animals were anesthetised 24 hours later with an intraperitoneal injection of ketamine/xylazine and 500-700 µl of blood was collected by cardiac puncture into serum separator tubes (BD Microtainer). Blood was centrifuged at 2,000 x g for 10 minutes at 15° C and serum was collected and stored at -70° C until analysis. Serum samples were thawed at 37° C for 30 minutes, diluted in PBS and aliquoted into 96-well assay plates. Factor VII levels were assessed using a chromogenic assay (Biophen FVII kit, Hyphen BioMed) according to the manufacturer's instructions and absorbance was measured in a microplate reader equipped with a 405 nm wavelength filter.

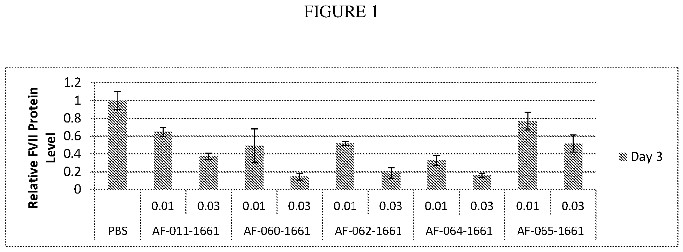

The efficacy of lipid nanoparticle formulations containing the cationic lipids below was determined by the procedure above. Figure 1 shows the relative FVII protein levels at day 3 (at an siRNA concentration of 0.01 and 0.03 mg/kg). Formulation AF-011 contained the cationic lipid known as MC3.

| Formulation | Cationic Lipid |
|---|---|
| AF-011 | 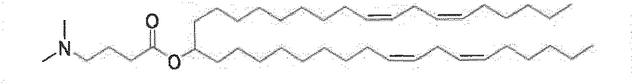 |
| AD-1661 | |
| AF-060 | 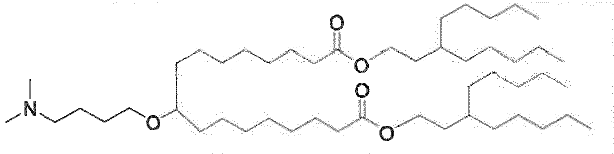 |
| AD-1661 | |
| AF-062 | 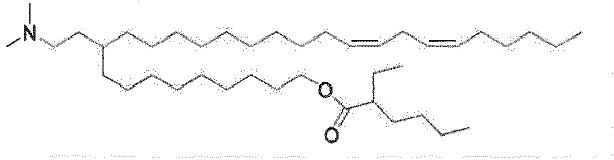 |
| AD-1661 | |
| AF-064 | 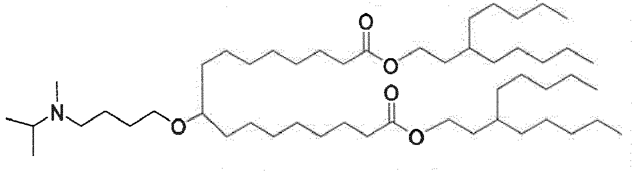 |
| AD-1661 | |
| AF-065 | 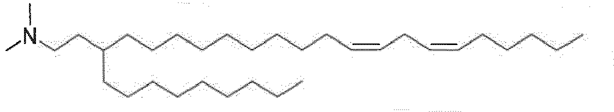 |
| AD-1661 | |

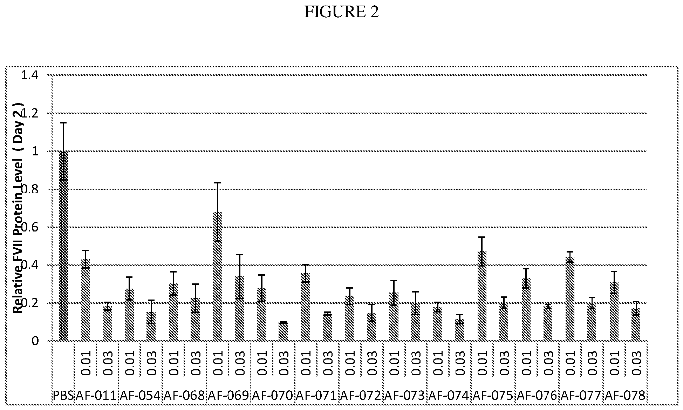

The efficacy of lipid nanoparticle formulations containing the cationic lipids below was determined by the procedure above. Figure 2 shows the relative FVII protein levels at day 2 (at an siRNA concentration of 0.01 and 0.03 mg/kg). The logP values for the cationic lipids listed in the table below were calculated using the software available at http://www.molinspiration.com/services/logp.html from Molinspiration Cheminformatics of Slovensky Grob, Slovak Republic.

| Formulation | Cationic Lipid | Formulation Ratio / N/P Ratio | Log P |
|---|---|---|---|
| AF-011 | 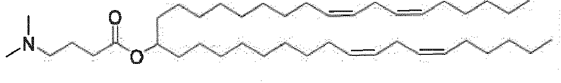 | 50/10/38.5/1.5 | 10.201 |
| AD-1661 | | N/P =2.97 | |
| AF-068 | 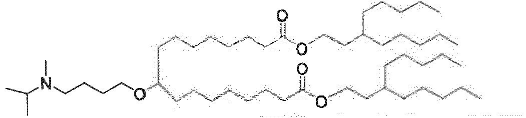 | 58/10/30/2 | 10.42 |
| AD-1661 | | N/P =2.97 | |
| AF-054* | 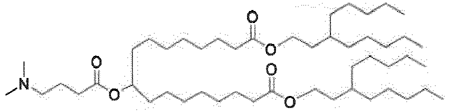 | 50/10/38.5/1.5 | 10.313 |
| AD-1661 | | N/P =3.01 | |
| AF-069 | 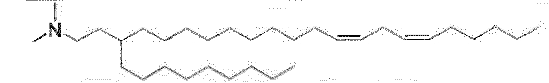 | 58/10/30/2 | 9.94 |
| AD-1661 | | N/P =3.01 | |
| AF-070 | 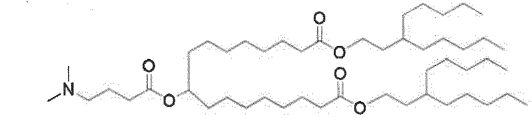 | 58/10/30/2 | 10.313 |
| AD-1661 | | N/P =2.97 | |
| AF-071 | 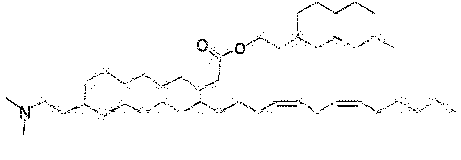 | 50/10/38.5/1.5 | 10.43 |
| AD-1661 | | N/P =2.97 | |
| AF-072 | 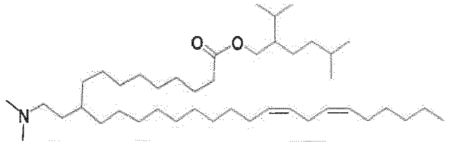 | 50/10/38.5/1.5 | 10.28 |
| AD-1661 | | N/P =2.97 | |
| AF-073 | 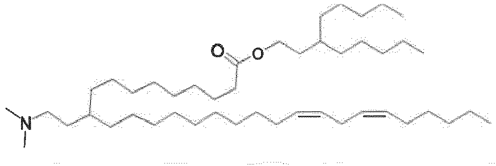 | 58/10/30/2 | 10.43 |
| AD-1661 | | N/P =2.97 | |
| AF-074 | 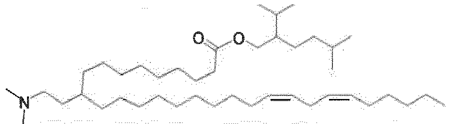 | 58/10/30/2 | 10.28 |
| AD-1661 | | N/P =2.97 | |
| AF-075 | 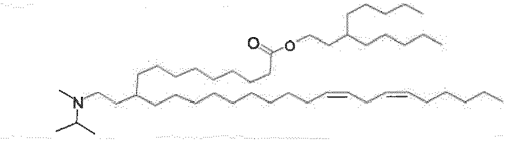 | 50/10/38.5/1.5 | 10.48 |
| AD-1661 | | N/P =2.97 | |
| AF-076 | 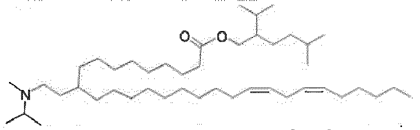 | 50/10/38.5/1.5 | 10.35 |
| AD-1661 | | N/P =3.01 | |
| AF-077 | 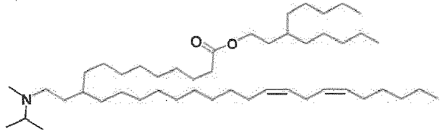 | 58/10/30/2 | 10.48 |
| AD-1661 | | N/P =2.97 | |
| AF-078 | 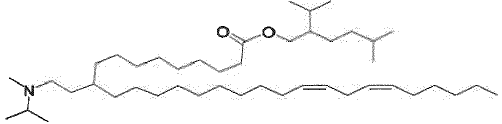 | 58/10/30/2 | 10.28 |
| AD-1661 | | N/P =2.97 | |

| | | | |
|---|---|---|---|
| * PEG-DPG (1,2-dipalmityl-sn-glycerol-methoxy polyethylene glycol) was used instead of PEG-DMG. | | | |

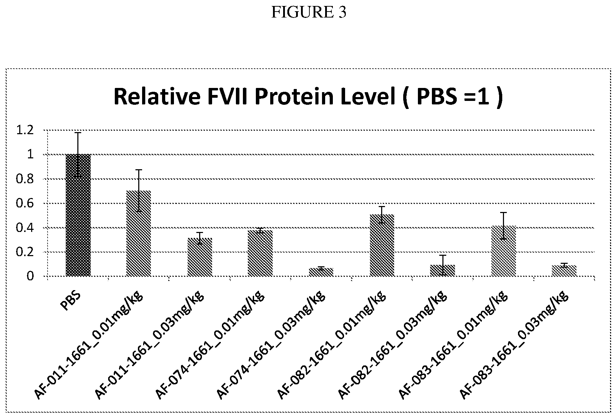

The efficacy of lipid nanoparticle formulations containing the cationic lipids below was determined by the procedure above. Figure 3 shows the relative FVII protein levels (at a siRNA concentration of 0.01 and 0.03 mg/kg). N/P refers to the ratio of amino groups of the cationic lipids to phosphate groups in the siRNA.

| Formulation | Cationic Lipid | Formulation Ratio | N/P Ratio |
|---|---|---|---|
| AF-011 | 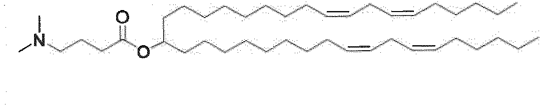 | 50/10/38.5/1.5 | 3.0 |
| AD-167990 | | | |
| AF-079 | 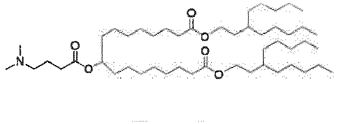 | 50/10/38/2 | 2.628 |
| AD-167990 | | | |
| AF-093 | 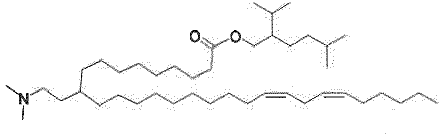 | 55/10/33/2 | 3.0 |
| AD-167990 | | | |
| AF-083 | 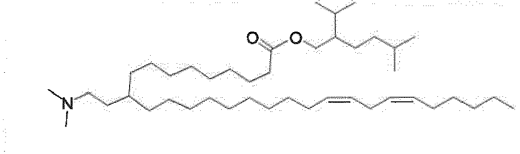 | 55/10/33/2 | 3.76 |
| AD-167990 | | | |
| AF-073 | 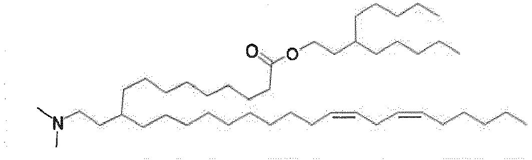 | 58/10/30/2 | 3.03 |
| AD-167990 | | | |
| AF-094 | 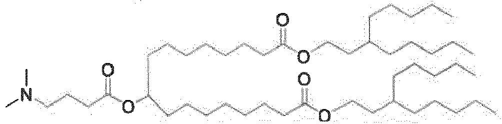 | 50/10/38/2 | 3.0 |
| AD-167990 | | | |

The structure of AD-167990 is shown below.

| Duplex | Sequence 5'-3' | SEQ ID NO: | Target |
|---|---|---|---|
| AD-167990 | gsasaacuCfaAfLTfAfaagugcuuusa | 3 | FXII |
| | usAfsaagCfacuuuauUfgAfguuucususg | 4 | |

Lower case is 2'OMe modification and Nf is a 2'F modified nucleobase, dT is deoxythymidine, s is phosphothioate

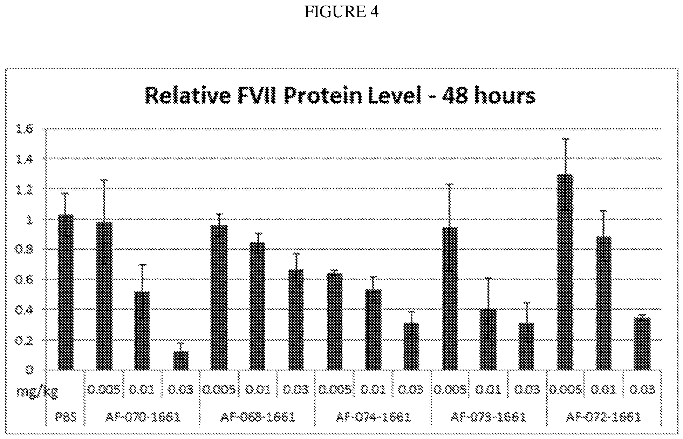

The efficacy of lipid nanoparticle formulations containing the cationic lipids below was determined by the procedure above. Figure 4 shows the relative FVII protein levels after 48 hours (at a siRNA concentration of 0.005, 0.01, and 0.03 mg/kg).

| Formulation | Cationic Lipid | Formulation Ratio / N/P Ratio |
|---|---|---|
| AF-070 | 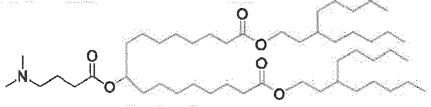 | 58:10:30:2 |
| AD-1661 | | N/P=3 |
| AF-068 | 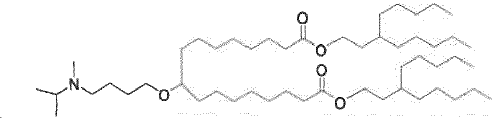 | 58:10:30:2 |
| AD-1661 | | N/P=3 |
| AF-072 | 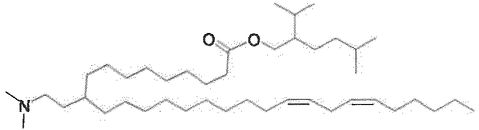 | 50/10/38.5/1.5 |
| AD-1661 | | N/P=3 |
| AF-073 | 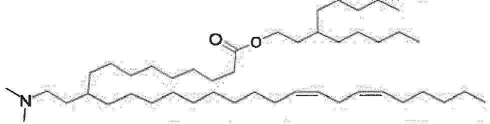 | 58:10:30:2 |
| AD-1661 | | N/P=3 |
| AF-074 | 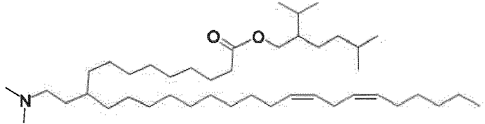 | 58/10/30/2 |
| AD-1661 | | N/P=3 |

### Example 5: Non-Human Primate Study

The purpose of this study is to determine the pharmacodynamics of multiple Lipid Nano Particles (LNPs) targeting F12 protein after a single intravenous infusion dose to male and/or female cynomolgus monkeys. Five lipids nanoparticle formulations with different lipids (AF-011, AF-070, AF-079, AF-073 and AF-074) having F12 siRNA AD-167990 were tested in this study. Each of these formulations were administered to cynomolgus monkeys on Day 1 by intravenous infusion over approximately 60 minutes (3 animals/group/formulation) with a targeted dose of 0.3 mg/kg. For all groups, blood (approximately 1 mL) were collected from each animal on Days -5, -1, 1 (predose), 2, 3, 5, 8, 15, 22, 29, 36, 43, 50, 57, and 64. F12 plasma protein levels were determined using F12 assay using human factor XII total antigen assay ELISA kit from Molecular Innovations (HFXIIKT-TOT).

The formulations described in the table below were prepared as described in Example 3 with the F12siRNA AD-167990.

| Formulation | Cationic Lipid | Formulation Ratio | N/P Ratio |
|---|---|---|---|
| AF-011 | 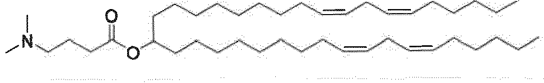 | 50/10/38.5/1.5 | 3 |
| AD-167990 | | | |
| AF-070 | 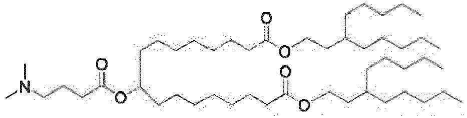 | 58/10/30/2 | 2.628 |
| AD-167990 | | | |
| AF-079 | 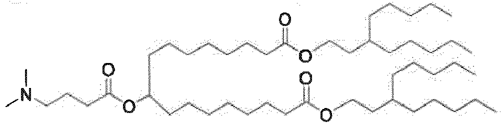 | 50/10/38/2 | 2.628 |
| AD-167990 | | | |
| AF-073 | 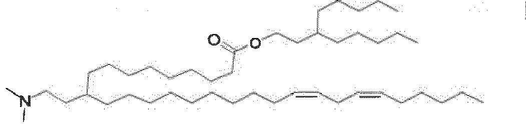 | 58/10/30/2 | 3.03 |
| AD-167990 | | | |
| AF-074 | 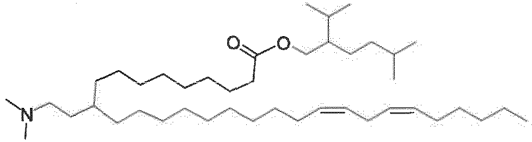 | 58/10/30/2 | 3.23 |
| AD-167990 | | | |

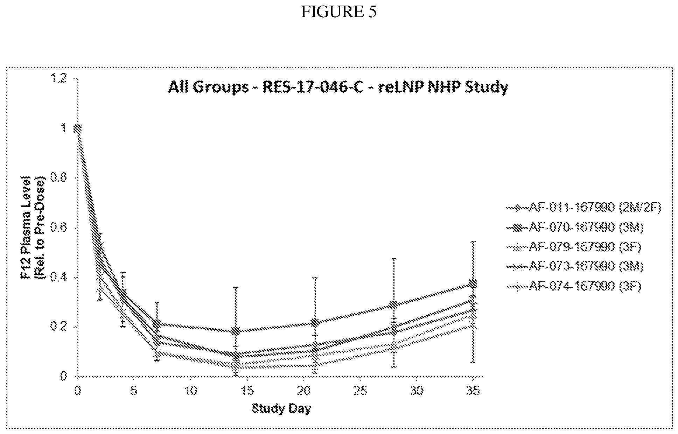

The table below provides the protocol used for each formulation. Figure 5 shows the relative F12 plasma levels (relative to pre-dose).

| Formulation | Group Number | N | Dose Level (mg/kg) | Route and Regimen |
|---|---|---|---|---|
| AF-011(MC3)-AD-167990 | 1 | 4(2F=2M) | 0.3 | 60 minutes |
| | | | | IV Infusion |
| AF-070(ALNY369)-AD-167990 | 2 | 3(3M) | 0.3 | 60 minutes |
| | | | | IV Infusion |
| AF-079(ALNY369)-AD-167990 | 3 | 3(3F) | 0.3 | 60 minutes |
| | | | | IV Infusion |
| AF-074(ALNY369)-AD-167990 | 4 | 3(3F) | 0.3 | 60 minutes |
| | | | | IV Infusion |
| AF-073(ALNY369)-AD-167990 | 5 | 3(3M) | 0.3 | 60 minutes |
| | | | | IV Infusion |

### Example 6: Non-Human Primate Study

This was a single-dose pharmacodynamic (PD) / pharmacokinetic (PK) study of multiple Lipid Nano Particles (LNPs) targeting Factor XII (F12) protein after a single intravenous infusion dose to male and/or female cynomolgus monkeys. Six lipids nanoparticle formulations with different lipids (AF-079, AF-093, AF-0783, AF-073, AF-094 and AF-011) having F12 siRNA AD-167990 were tested in this study. Each of these formulations were administered to cynomolgus monkeys on Day 1 by intravenous infusion over approximately 60 minutes (3 animals/group/formulation) with a targeted dose of 0.03, 0.1 or 0.3 mg/kg. F12 plasma protein levels were determined using a F12 assay using a human factor XII total antigen assay ELISA kit from Molecular Innovations (HFXIIKT-TOT) as reported earlier.

The study design is shown in the table below.

| **Group** | **Test Article** | **Number of Males** | **Dose Route** | **Vehicle** | **Dose Level (mg/kg)** | **Dose Volume (mL/kg)** | **Concentration (mg/mL)^{B}** | **Collection Intervals** |
|---|---|---|---|---|---|---|---|---|
| 1 | AD-167990.1 in AF-011 | 3 | IV Infusion | A | 0.3 | 10 | 0.030 | Blood^{C,D} |
| 2 | AD-167990.1 in AF-094 | 3 | IV Infusion | A | 0.03 | 10 | 0.003 | Blood^{C,D} |
| 3 | AD-167990.1 in AF-094 | 3 | IV Infusion | A | 0.1 | 10 | 0.010 | Blood^{C,D} |
| 4 | AD-167990.1 in AF-094 | 3 | IV Infusion | A | 0.3 | 10 | 0.030 | Blood^{C,D} |
| 5 | AD-167990.1 in AF-079 | 3 | IV Infusion | A | 0.3 | 10 | 0.030 | Blood^{C,D} |
| 6 | AD-167990.1 in AF-073 | 3 | IV Infusion | A | 0.03 | 10 | 0.003 | Blood^{C,D} |
| 7 | AD-167990.1 in AF-073 | 3 | IV Infusion | A | 0.1 | 10 | 0.010 | Blood^{C,D} |
| 8 | AD-167990.1 in AF-073 | 3 | IV Infusion | A | 0.3 | 10 | 0.030 | Blood^{C,D} |
| 9 | AD-167990.1 in AF-093 | 3 | IV Infusion | A | 0.03 | 10 | 0.003 | Blood^{C,D} |
| 10 | AD-167990.1 in AF-093 | 3 | IV Infusion | A | 0.1 | 10 | 0.010 | Blood^{C,D} |
| 11 | AD-167990.1 in AF-093 | 3 | IV Infusion | A | 0.3 | 10 | 0.030 | Blood^{C,D} |
| 12 | AD-167990.1 in AF-083 | 3 | IV Infusion | A | 0.1 | 10 | 0.010 | Blood^{C,D} |
| 13 | AD-167990.1 in AF-083 | 3 | IV Infusion | A | 0.3 | 10 | 0.030 | Blood^{C,D} |
| ^{A} 0.9% Sodium Chloride | | | | | | | | |
| ^{B} Dosed based on siRNA concentration | | | | | | | | |
| ^{C} PK: Blood samples will be collected predose and at 0 (just prior to the end of infusion), 0.033, 0.25, 0.5, 1, 2, 4, 8, 24, 48, 96, 168, 336, 672 and 1344 hours postdose. Sample collection times will be based off the end of infusion. | | | | | | | | |
| ^{D} PD: Blood samples will be collected on Days -5, -1, predose and at 24, 48, 96, 168, 336, 504, 672, 840, 1008, 1176, 1344 and 1512 hours postdose. Sample collection times will be based off the end of infusion. | | | | | | | | |

The formulations described in the table below were prepared as described in Example 3 with the F12siRNA AD-167990.

| Formulation | Cationic Lipid | Formulation Ratio | N/P Ratio |
|---|---|---|---|
| AF-0079 | 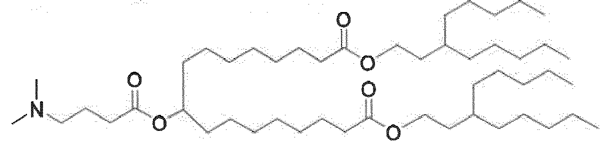 | 50/10/38/2 | 6 |
| AD-167990 | | | |
| AF-093 | 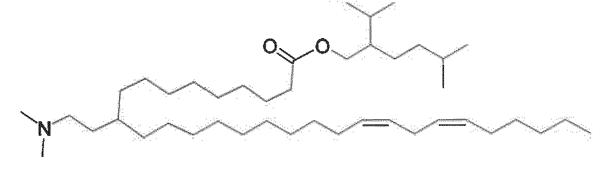 | 55/10/33/2 | 6 |
| AD-167990 | | | |
| AF-083 | 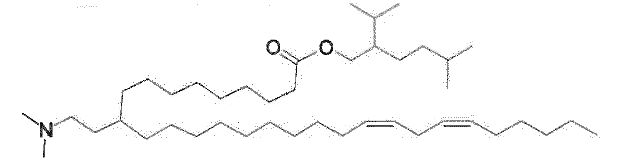 | 55/10/33/2 | 7 |
| AD-167990 | | | |
| AF-073 | 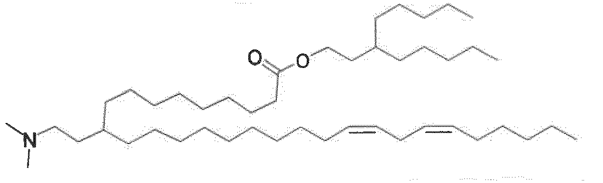 | 58/10/30/2 | 6 |
| AD-167990 | | | |
| AF-094 | 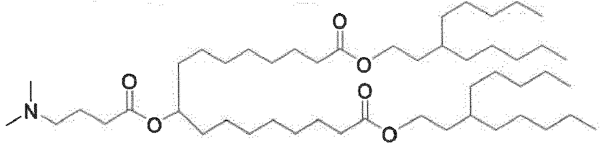 | 58/10/30/2 | 7 |
| AD-167990 | | | |
| AF-011 | 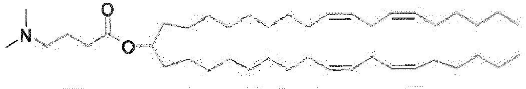 | 58/38.5/10/1.5 | 5.71 |
| AD-167990 | | | |

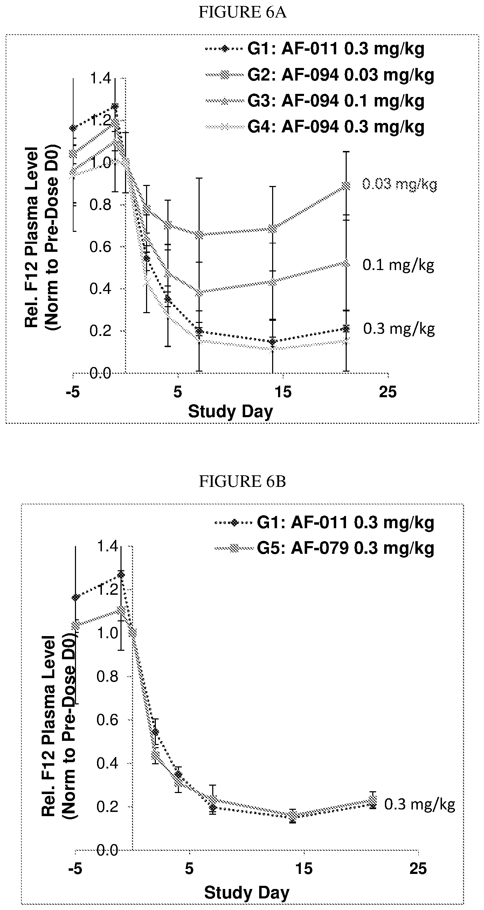

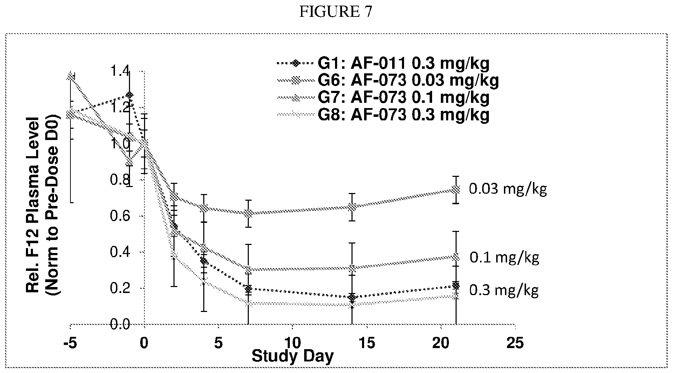

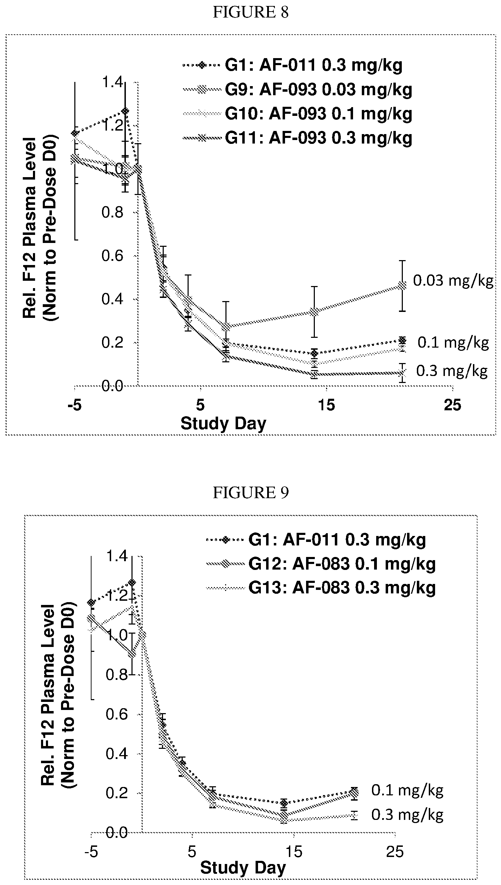

Figures 6A, 6B, and 7-9 show the dose response with AF-094, a single dose of AF-079, AF-073, AF-093, and AF-083, respectively.

## Claims

1. A compound of formula (A): 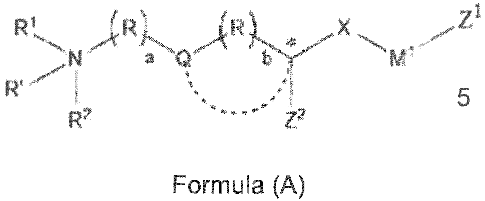 or a salt thereof, wherein
R' is absent, hydrogen, or alkyl;
with respect to R¹ and R²,
(i) R¹ and R² are each, independently, optionally substituted alkyl, alkenyl, alkynyl, cycloalkylalkyl, heterocycle, or R¹⁰;
(ii) R¹ and R², together with the nitrogen atom to which they are attached, form an optionally substituted heterocylic ring; or
(iii) one of R¹ and R² is an optionally substituted alkyl, alkenyl, alkynyl, cycloalkyl, cycloalkylalkyl, or heterocycle, and the other forms a 4-10 membered heterocyclic ring or heteroaryl with (a) the adjacent nitrogen atom and (b) the (R)ₐ group adjacent to the nitrogen atom;
each occurrence of R is, independently, -(CR³R⁴)-;
each occurrence of R³ and R⁴ are, independently H, halogen, OH, alkyl, alkoxy, -NH₂, R¹⁰, alkylamino, or dialkylamino;
each occurrence of R¹⁰ is independently selected from PEG and polymers based on poly(oxazoline), polyethylene oxide), poly(vinyl alcohol), poly(glycerol), poly(N-vinylpyrrolidone), poly[N-(2-hydroxypropyl)methacrylamide] and poly(amino acid)s, wherein (i) the PEG or polymer is linear or branched, (ii) the PEG or polymer is polymerized by n subunits, (iii) n is a number-averaged degree of polymerization between 10 and 200 units, and (iv) the compound has at most two R¹⁰ groups;
the dashed line to Q is absent or a bond;
when the dashed line to Q is absent then Q is absent or is -O-, -NH-, -N(R⁵)-, -S-, -C(O)-, -C(O)O-, -OC(O)-, -C(O)N(R⁴)-, -N(R⁵)C(O)-, -S-S-, -OC(O)O-, -O-N=C(R⁵)-, -C(R⁵)=N-O-, -OC(O)N(R⁵)-, -N(R⁵)C(O)N(R⁵)-, -N(R⁵)C(O)O-, -C(O)S-, -C(S)O- or -C(R⁵)=N-O-C(O)-; or
when the dashed line to Q is a bond then (i) b is 0 and (ii) Q and the tertiary carbon adjacent to it (C*) form a substituted or unsubstituted, mono- or bi-cyclic heterocyclic group having from 5 to 10 ring atoms;
each occurrence of R⁵ is, independently, H or alkyl;
X is alkylene or alkenylene;
M¹ is a biodegradable group, selected from -OC(O)-, -C(O)O-, -SC(O)-, -C(O)S-, -OC(S)-, -C(S)O-, -S-S-, -C(R5)=N-, -N=C(R⁵)-, -C(R⁵)=N-O-, -O-N=C(R⁵)-, -C(O)(NR⁵)-, -N(R⁵)C(O)-, -C(S)(NR⁵)-, -N(R⁵)C(O)-, -N(R⁵)C(O)N(R⁵)-, -OC(O)O-, -OSi(R⁵)₂O-, -C(O)(CR³R⁴)C(O)O-, -OC(O)(CR³R⁴)C(O)-, and 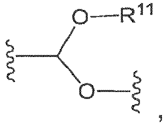 wherein R¹¹ is a C₂-C₈ alkyl or alkenyl;
a is 1, 2, 3, 4, 5 or 6;
b is 0, 1, 2, or 3;
Z¹ is a C₆-C₁₄ branched alkyl group; and
Z² is C₄-C₂₀ alkenyl, wherein the alkenyl group may optionally be substituted with one or two fluorine atoms at the alpha position to a double bond which is between the double bond and the terminus of Z².

2. The compound of claim 1, wherein
(i) R'R¹R²N-(R)ₐ-Q-(R)_{b}- is (CH₃)₂N-(CH2)2-, (CH₃)₂N-(CH₂)₃-C(O)O-, (CH₃)₂N-(CH₂)₂-NH-C(O)O-, (CH₃)₂N-(CH₂)₂-OC(O)-NH-, or (CH₃)₂N-(CH₂)₃-C(CH₃)=N-O-;
(ii) R¹ and R² are both alkyl;
(iii) M¹ is -OC(O)- or -C(O)O-;
(iv) Z¹ is a C₆-C₁₀ branched alkyl group, preferably wherein Z¹ is -CH(CH₂CH₃)(CH₂CH₂CH₂CH₃), -CH₂CH(ⁱPr)(CH₂CH₂ⁱPr) or -CH₂CH(n-Bu)₂; and/or
(v) Z² is a C₁₉ alkenyl containing one or two double bonds, preferably wherein Z² is -(CH₂)₉CH=CHCH₂CH=CH(CH₂)₄CH₃.

3. A compound according to claim 1 selected from selected from: 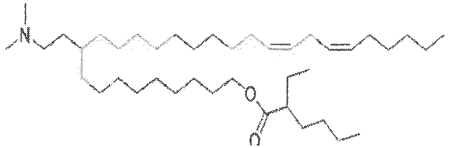 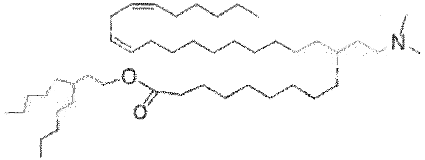 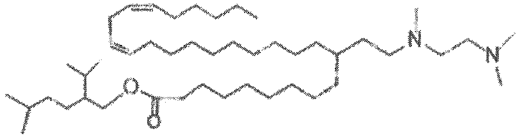 and salts thereof.

4. The compound of any of the preceding claims, wherein the compound is in the form of a pharmaceutically acceptable salt, or in the form of a cationic lipid.

5. A lipid particle comprising a neutral lipid, a PEG lipid, and a cationic lipid of claim 4.

6. The lipid particle of claim 5, wherein the neutral lipid is selected from distearoylphosphatidylcholine (DSPC), 1,2-dipalmitoyl-sn-glycero-3-phosphocholine (DPPC), 1-palmitoyl-2-oleoyl-sn-phosphatidylcholine (POPC), 1,2-dileoyl-sn-3-phosphoethanolamine (DOPE), or SM; and the lipid particle further comprises a sterol.

7. The lipid particle of any one of claims 5 and 6, wherein the cationic lipid is present in a mole percentage of 20% and 60%; the neutral lipid is present in a mole percentage of 5% to 25%; the sterol is present in a mole percentage of 25% to 55%; and the PEG lipid is PEG-DMA, 1,2-dimyristoyl-sn-glycerol-methoxy polyethylene glycol (PEG-DMG), or a combination thereof, and is present in a mole percentage of 0.5% to 15%.

8. The lipid particle of claim 5, wherein the PEG lipid is 1,2-dimyristoyl-sn-glycerol-methoxy polyethylene glycol (PEG-DMG).

9. The lipid particle of claim 8, wherein the lipid particle comprises 50 mole % of the cationic lipid, 10% distearoylphosphatidylcholine (DSPC), 38.5% cholesterol, and 1.5% 1,2-dimyristoyl-sn-glycerol-methoxy polyethylene glycol (PEG-DMG), based on 100% of the lipid components in the lipid particle; or
wherein the lipid particle comprises 58 mole % of the cationic lipid, 10% distearoylphosphatidylcholine (DSPC), 30% cholesterol, and 2% 1,2-dimyristoyl-sn-glycerol-methoxy polyethylene glycol (PEG-DMG), based on 100% of the lipid components in the lipid particle.

10. The lipid particle of any of claims 6-9, further comprising an active agent, preferably wherein the active agent is a nucleic acid selected from a plasmid, an immunostimulatory oligonucleotide, an siRNA, an antisense oligonucleotide, a microRNA, an antagomir, an aptamer, and a ribozyme.

11. A pharmaceutical composition comprising a lipid particle of any one of claims 6-10 and a pharmaceutically acceptable carrier.

12. A lipid particle of claim 10 for use in a method of modulating the expression of a target gene in a cell, preferably wherein the active agent is a nucleic acid is an siRNA.

13. The pharmaceutical composition of claim 11 for use in a method of treating a disease or disorder **characterized by** the overexpression of a polypeptide in a subject, wherein the active agent is a nucleic acid selected from the group consisting of an siRNA, a microRNA, and an antisense oligonucleotide, and wherein the siRNA, microRNA, or antisense oligonucleotide includes a polynucleotide that specifically binds to a polynucleotide that encodes the polypeptide, or a complement thereof.

14. The pharmaceutical composition of claim 11 for use in a method of treating a disease or disorder **characterized by** underexpression of a polypeptide in a subject, wherein the active agent is a plasmid that encodes the polypeptide or a functional variant or fragment thereof.

15. The pharmaceutical composition of claim 11 for use in a method of inducing an immune response in a subject, wherein the active agent is an immunostimulatory oligonucleotide.

## Patentansprüche

1. Verbindung der Formel (A): 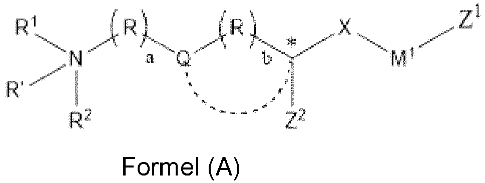 oder ein Salz davon, wobei
R' abwesend, Wasserstoff oder Alkyl ist;
in Bezug auf R¹ und R²,
(i) R¹ und R² jeweils unabhängig voneinander gegebenenfalls substituiertes Alkyl, Alkenyl, Alkinyl, Cycloalkylalkyl, Heterocyclus oder R10 sind;
(ii) R¹ und R² zusammen mit dem Stickstoffatom, an das sie gebunden sind, einen gegebenenfalls substituierten heterocyclischen Ring bilden; oder
(iii) einer von R¹ und R² ein gegebenenfalls substituiertes Alkyl, Alkenyl, Alkinyl, Cycloalkyl, Cycloalkylalkyl oder Heterocyclus ist und der andere einen 4-10-gliedrigen heterocyclischen Ring oder Heteroaryl mit (a) dem benachbarten Stickstoffatom und (b) der zum Stickstoffatom benachbarten (R)ₐ-Gruppe bildet;
jedes Auftreten von R unabhängig voneinander -(CR³R⁴)- ist;
jedes Auftreten von R³ und R⁴ unabhängig voneinander H, Halogen, OH, Alkyl, Alkoxy, -NH₂, R¹⁰, Alkylamino oder Dialkylamino ist;
jedes Auftreten von R¹⁰ unabhängig ausgewählt ist aus PEG und Polymeren auf der Basis von Poly(oxazolin), Poly(ethylenoxid), Poly(vinylalkohol), Poly(glycerin), Poly(N-vinylpyrrolidon), Poly[N-(2-hydroxypropyl)methacrylamid] und Poly(aminosäure)n, wobei (i) das PEG oder Polymer linear oder verzweigt ist, (ii) das PEG oder Polymer durch n Untereinheiten polymerisiert ist, (iii) n ein zahlengemittelter Polymerisationsgrad zwischen 10 und 200 Einheiten ist und (iv) die Verbindung höchstens zwei R¹⁰-Gruppen aufweist;
die gestrichelte Linie zu Q abwesend oder eine Bindung ist;
wenn die gestrichelte Linie zu Q abwesend ist, dann Q abwesend ist oder -O-, -NH-, -N(R⁵)-, -S-, -C(O)-, -C(O)O-, -OC(O)-, -C(O)N(R⁴)-, -N(R⁵)C(O)-, -S-S-, -OC(O)O-, -O-N=C(R⁵)-, -C(R⁵)=N-O-, -OC(O)N(R⁵)-, -N(R⁵)C(O)N(R⁵)-, -N(R⁵)C(O)O-, -C(O)S-, -C(S)O- oder -C(R⁵)=N-O-C(O)- ist; oder
wenn die gestrichelte Linie zu Q eine Bindung ist, dann (i) b 0 ist und (ii) Q und der ihm benachbarte tertiäre Kohlenstoff (C*) eine substituierte oder unsubstituierte, mono- oder bicyclische heterocyclische Gruppe mit 5 bis 10 Ringatomen bilden;
jedes Vorkommen von R⁵ unabhängig H oder Alkyl ist;
X Alkylen oder Alkenylen ist;
M1 eine biologisch abbaubare Gruppe ist, ausgewählt aus -OC(O)-, -C(O)O-, -SC(O)-, -C(O)S-, -OC(S)-, -C(S)O-, -S-S-, -C(R⁵)=N-, -N=C(R⁵)-, -C(R⁵)=N-O-, -O-N=C(R⁵)-, -C(O)(NR⁵)-, -N(R⁵)C(O)-, -C(S)(NR⁵)-, -N(R⁵)C(O)-, -N(R⁵)C(O)N(R⁵)-, -OC(O)O-, -OSi(R⁵)₂O-, -C(O)(CR³R⁴)C(O)O-, -OC(O)(CR³R⁴)C(O)- und 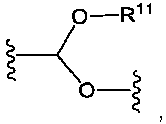 wobei R¹¹ ein C₂-C₈-Alkyl oder -Alkenyl ist;
a 1, 2, 3, 4, 5 oder 6 ist;
b 0, 1, 2 oder 3 ist;
Z¹ eine verzweigte C₆-C₁₄-Alkylgruppe ist; und
Z² eine C₄-C₂₀-Alkenylgruppe ist, wobei die Alkenylgruppe gegebenenfalls mit einem oder zwei Fluoratomen in der Alpha-Position zu einer Doppelbindung, die sich zwischen der Doppelbindung und dem Ende von Z² befindet, substituiert sein kann.

2. Verbindung nach Anspruch 1, wobei
(i) R'R¹R²N-(R)ₐ-Q-(R)_{b}- (CH₃)₂N-(CH2)2-, (CH₃)₂N-(CH₂)₃-C(O)O-, (CH₃)₂N-(CH₂)₂-NH-C(O)O-, (CH₃)₂N-(CH₂)₂-OC(O)-NH-, oder (CH₃)₂N-(CH₂)₃-C(CH₃)=N-O- ist;
(ii) R¹ und R² beide Alkyl sind;
(iii) M¹ -OC(O)- oder -C(O)O- ist;
(iv) Z¹ eine verzweigte C₆-C₁₀-Alkylgruppe ist, wobei Z¹ vorzugsweise -CH(CH₂CH₃)(CH₂CH₂CH₂CH₃), -CH₂CH(ⁱPr)(CH₂CH₂ⁱPr) oder -CH₂CH (n-Bu)₂ ist; und/oder
(v) Z² ein C₁₉-Alkenyl ist, das eine oder zwei Doppelbindungen enthält, wobei Z² vorzugsweise -(CH₂)₉CH=CHCH₂CH=CH(CH₂)₄CH₃ ist.

3. Verbindung nach Anspruch 1, ausgewählt aus ausgewählt aus: 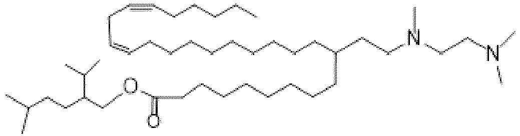 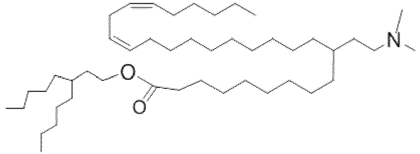 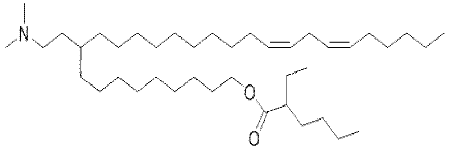 und Salzen davon.

4. Verbindung nach einem der vorhergehenden Ansprüche, wobei die Verbindung in Form eines pharmazeutisch verträglichen Salzes oder in Form eines kationischen Lipids vorliegt.

5. Lipidpartikel, umfassend ein neutrales Lipid, ein PEG-Lipid und ein kationisches Lipid nach Anspruch 4.

6. Lipidpartikel nach Anspruch 5, wobei das neutrale Lipid ausgewählt ist aus Distearoylphosphatidylcholin (DSPC), 1,2-Dipalmitoyl-sn-glycero-3-phosphocholin (DPPC), 1-Palmitoyl-2-oleoyl-sn-phosphatidylcholin (POPC), 1,2-Dileoyl-sn-3-phosphoethanolamin (DOPE) oder SM; und das Lipidpartikel weiter ein Sterol umfasst.

7. Lipidpartikel nach einem der Ansprüche 5 und 6, wobei das kationische Lipid in einem Molprozentsatz von 20% bis 60% vorhanden ist; das neutrale Lipid in einem Molprozentsatz von 5% bis 25% vorhanden ist; das Sterol in einem Molprozentsatz von 25% bis 55% vorhanden ist; und das PEG-Lipid PEG-DMA, 1,2-Dimyristoyl-sn-glycerin-methoxypolyethylenglycol (PEG-DMG) oder eine Kombination davon ist und in einem Molprozentsatz von 0,5% bis 15% vorhanden ist.

8. Lipidpartikel nach Anspruch 5, wobei das PEG-Lipid 1,2-Dimyristoyl-sn-glycerin-methoxypolyethylenglycol (PEG-DMG) ist.

9. Lipidpartikel nach Anspruch 8, wobei das Lipidpartikel 50 Mol-% des kationischen Lipids, 10% Distearoylphosphatidylcholin (DSPC), 38,5% Cholesterin und 1,5% 1,2-Dimyristoyl-sn-glycerin-methoxypolyethylenglycol (PEG-DMG), bezogen auf 100% der Lipidbestandteile im Lipidpartikel, umfasst; oder
wobei das Lipidpartikel 58 Mol-% des kationischen Lipids, 10% Distearoylphosphatidylcholin (DSPC), 30% Cholesterin und 2% 1,2-Dimyristoyl-sn-glycerin-methoxypolyethylenglykol (PEG-DMG), bezogen auf 100% der Lipidbestandteile im Lipidpartikel, umfasst.

10. Lipidpartikel nach einem der Ansprüche 6 bis 9, weiter umfassend einen Wirkstoff, wobei der Wirkstoff vorzugsweise eine Nukleinsäure ist, ausgewählt aus einem Plasmid, einem immunstimulatorischen Oligonukleotid, einer siRNA, einem Antisense-Oligonukleotid, einer microRNA, einem Antagomir, einem Aptamer und einem Ribozym.

11. Pharmazeutische Zusammensetzung, umfassend ein Lipidpartikel nach einem der Ansprüche 6-10 und einen pharmazeutisch verträglichen Träger umfasst.

12. Lipidpartikel nach Anspruch 10 zur Verwendung in einem Verfahren zur Modulation der Expression eines Zielgens in einer Zelle, wobei der Wirkstoff vorzugsweise eine Nukleinsäure ist eine siRNA ist.

13. Pharmazeutische Zusammensetzung nach Anspruch 11 zur Verwendung in einem Verfahren zur Behandlung einer Krankheit oder Störung, die durch die Überexpression eines Polypeptids in einem Subjekt gekennzeichnet ist, wobei der Wirkstoff eine Nukleinsäure ist, ausgewählt aus der Gruppe, bestehend aus einer siRNA, einer microRNA und einem Antisense-Oligonukleotid, und wobei die siRNA, microRNA oder das Antisense-Oligonukleotid ein Polynukleotid oder ein Komplement enthält, das spezifisch an ein Polynukleotid bindet, welches das Polypeptid kodiert.

14. Pharmazeutische Zusammensetzung nach Anspruch 11 zur Verwendung in einem Verfahren zur Behandlung einer Krankheit oder Störung, die durch Unterexpression eines Polypeptids in einem Subjekt gekennzeichnet ist, wobei der Wirkstoff ein Plasmid ist, welches das Polypeptid oder eine funktionelle Variante oder ein Fragment davon kodiert.

15. Pharmazeutische Zusammensetzung nach Anspruch 11 zur Verwendung in einem Verfahren zur Induktion einer Immunantwort in einem Subjekt, wobei der Wirkstoff ein immunstimulierendes Oligonukleotid ist.

## Revendications

1. Composé de formule (A) : 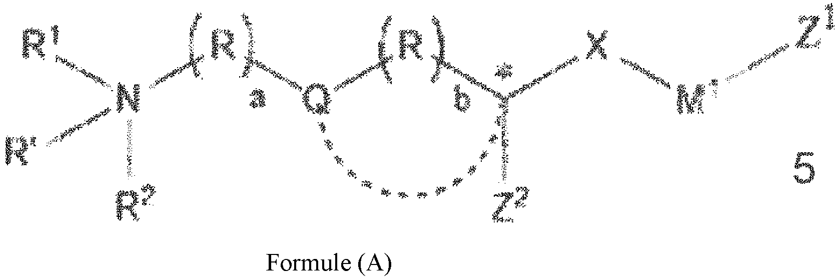 ou sel de celui-ci, dans laquelle
R' est absent, hydrogène, ou alkyle ;
par rapport à R¹ et R²,
(i) R¹ et R² sont chacun, indépendamment, alkyle substitué en option, alkényle, alkynyle, cycloalkylalkyle, hétérocycle, ou R¹⁰ ;
(ii) R¹ et R², conjointement à l'atome d'azote auquel ils sont liés, forment un anneau hétérocyclique substitué en option ; ou
(iii) un de R¹ et R² est un alkyle substitué en option, alkényle, alkynyle, cycloalkylalkyle, ou hétérocycle, et l'autre forme un anneau hétérocyclique à 4 10 membres ou hétéroaryle avec (a) l'atome d'azote adjacent et (b) le groupe (R)ₐ adjacent à l'atome d'azote ;
chaque occurrence de R est, indépendamment, -(CR³R⁴)- ;
chaque occurrence de R³ et R⁴ sont, indépendamment, H, halogène, OH, alkyle, alcoxy, -NH₂, R¹⁰, alkylamino, ou dialkylamino ;
chaque occurrence de R¹⁰ est indépendamment sélectionné parmi PEG et des polymères à base de poly(oxazoline), oxyde de poly(éthylène), alcool poly(vinylique), poly(glycérol), poly(N-vinylpyrrolidone), poly[N-(2-hydroxypropyl)méthacrylamide] et acide(s) poly(aminé(s)), dans laquelle (i) le PEG ou polymère est linéaire ou ramifié, (ii) le PEG ou polymère est polymérisé par n sous-unités, (iii) n est un degré de polymérisation moyen en nombre compris entre 10 et 200 unités, et (iv) le composé a au plus deux groupes R¹⁰;
la ligne en pointillés à Q est absente ou une liaison ;
lorsque la ligne en pointillés à Q est absente, alors Q est absent ou est -O-, -NH-, -N(R⁵)-, -S-, -C(O)-, -C(O)O-, -OC(O)-, -C(O)N(R⁴)-, -N(R⁵)C(O)-, -S-S-, - OC(O)O-, -O-N=C(R⁵)-, -C(R⁵)=N-O-, -OC(O)N(R⁵)-, -N(R⁵)C(O)N(R⁵)-, - N(R⁵)C(O)O-, -C-(O)S-, -C(S)O- ou -C(R⁵)=N-O-C(O)- ; ou
lorsque la ligne en pointillés à Q est une liaison, alors (i) b est 0 et (ii) Q et le carbone tertiaire adjacent à lui (C*) forment un groupe hétérocyclique mono- ou bicyclique, substitué ou non substitué, ayant 5 à 10 atomes cycliques ;
chaque occurrence de R⁵ est, indépendamment, H ou alkyle ;
X est alkylène ou alkénylène ;
M¹ est un groupe biodégradable, sélectionné parmi -OC(O)-, -C(O)O-, -SC(O)-, -C(O)S-, -OC(S)-, -C(S)O-, -S-S-, -C(R⁵)=N-, -N=C(R⁵)-, -C(R⁵)=N-O-, -ON=C(R⁵)-, -C(O)(NR⁵)-, -N(R⁵)C(O)-, -C(S)(NR⁵)-, -N(R⁵)C(O)-, - N(R⁵)C(O)N(R⁵)-, -OC(O)O-, -OSi(R⁵)₂O-, -C(O)(CR³R⁴)C(O)O-, - OC(O)(CR³R⁴)C(O)-, et 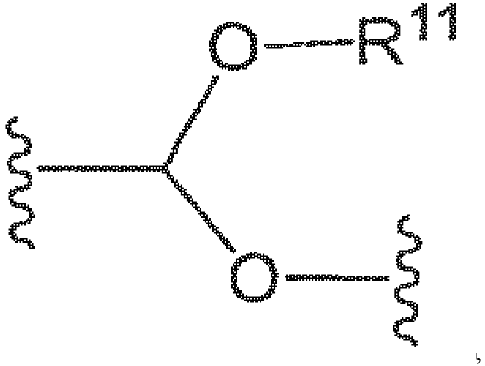 dans laquelle R¹¹ est un alkyle ou alkényle en C₂-C₈ ;
a est 1, 2, 3, 4, 5 ou 6 ;
b est 0,1, 2 ou 3 ;
Z¹ est un groupe alkyle ramifié en C₆-C₁₄ ; et
Z² est alkényle en C₄-C₂₀, dans laquelle le groupe alkényle peut être substitué en option par un ou deux atomes de fluor à la position alpha à une double liaison qui est entre la double liaison et la terminaison de Z².

2. Composé selon la revendication 1, dans lequel
(i) R'R¹R²N-(R)ₐ-Q-(R)_{b}- est (CH₃)₂N-(CH2)2-, (CH₃)₂N-(CH₂)₃-C(O)O-, (CH₃)₂N-(CH₂)₂-NH-C(O)O-, (CH₃)₂N-(CH₂)₂-OC(O)-NH-, ou (CH₃)₂N-(CH₂)₃-C(CH₃)=N-O- ;
(ii) R¹ et R² sont tous deux alkyle ;
(iii) M¹ est -OC(O)- ou -C(O)O- ;
(iv) Z¹ est un groupe alkyle ramifié en C₆-C₁₀, de préférence dans lequel Z¹ est - CH(CH₂CH₃)(CH₂CH₂CH₂CH₃), -CH₂CH(ⁱPr)(CH₂CH₂ⁱPr) ou -CH₂CH(n-Bu)₂ ; et/ou
(v) Z² est un alkényle en C₁₉ contenant une ou deux doubles liaisons, de préférence dans lequel Z² est -(CH₂)₉CH=CHCH₂CH=CH(CH₂)₄CH₃.

3. Composé selon la revendication 1, sélectionné parmi sélectionné parmi : 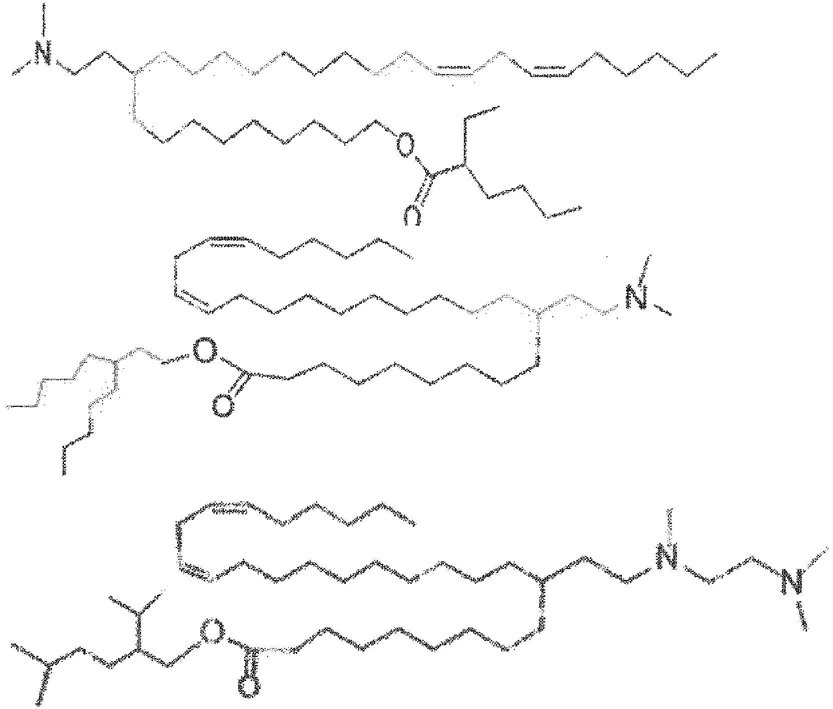 et des sels de ceux-ci.

4. Composé selon l'une quelconque des revendications précédentes, dans lequel le composé est sous la forme d'un sel pharmaceutiquement acceptable, ou sous la forme d'un lipide cationique,

5. Particule lipidique comprenant un lipide neutre, un lipide de PEG, et un lipide cationique selon la revendication 4.

6. Particule lipidique selon la revendication 5, dans laquelle le lipide neutre est sélectionné parmi la distéaroylphosphatidylcholine (DSPC), 1,2-dipalmitoyl-sn-glycéro-3-phosphocholine (DPPC), 1-palmitoyl-2-oléoyl-sn-phosphatidylcholine (POPC), 1,2-diléoyl-sn-3-phosphoéthanolamine (DOPE), ou SM ; et la particule lipidique comprend en outre un stérol.

7. Particule lipidique selon l'une quelconque des revendications 5 et 6, dans laquelle le lipide cationique est présent en un pourcentage molaire de 20 % et 60 % ; le lipide neutre est présent en un pourcentage molaire de 5 % à 25 % ; le stérol est présent en un pourcentage molaire de 25 % à 55 % ; et le lipide de PEG est PEG-DMA, 1,2-dimyristoyl-sn-glycérol-méthoxy polyéthylène glycol (PEG-DMG), ou une combinaison de ceux-ci, et est présent en un pourcentage molaire de 0,5 % à 15 %.

8. Particule lipidique selon la revendication 5, dans laquelle le lipide de PEG est le 1,2-dimyristoyl-sn-glycérol-méthoxy polyéthylène glycol (PEG-DMG).

9. Particule lipidique selon la revendication 8, dans laquelle la particule lipidique comprend 50 % en mole du lipide cationique, 10 % de distéaroylphosphatidylcholine (DSPC), 38,5 % de cholestérol, et 1,5 % de 1,2-dimyristoyl-sn-glycérol-méthoxy polyéthylène glycol (PEG-DMG), basés sur 100 % des composants lipidiques dans la particule lipidique ; ou
dans laquelle la particule lipidique comprend 58 % en mole du lipide cationique, 10 % de distéaroylphosphatidylcholine (DSPC), 30 % de cholestérol, et 2 % de 1,2-dimyristoyl-sn-glycérol-méthoxy polyéthylène glycol (PEG-DMG), basés sur 100 % des composants lipidiques dans la particule lipidique.

10. Particule lipidique selon l'une quelconque des revendications 6 à 9, comprenant en outre un agent actif, de préférence dans laquelle l'agent actif est un acide nucléique sélectionné parmi un plasmide, un oligonucléotide immunostimulateur, un ARNsi, un oligonucléotide antisens, un microARN, un antagomir, un aptamère, et un ribozyme.

11. Composition pharmaceutique comprenant une particule lipidique selon l'une quelconque des revendications 6 à 10 et un support pharmaceutiquement acceptable,

12. Particule lipidique selon la revendication 10 destinée à être utilisée dans un procédé de modulation de l'expression d'un gène cible dans une cellule, de préférence dans laquelle l'agent actif est un acide nucléique est un ARNsi.

13. Composition pharmaceutique selon la revendication 11 destinée à être utilisée dans un procédé de traitement d'une maladie ou d'un trouble caractérisé(e) par la surexpression d'un polypeptide chez un sujet, dans laquelle l'agent actif est un acide nucléique sélectionné parmi le groupe constitué d'un ARNsi, d'un microARN, et d'un oligonucléotide antisens, et dans laquelle l'ARNsi, le microARN, ou l'oligonucléotide antisens inclut un polynucléotide qui se lie spécifiquement à un polynucléotide qui code pour le polypeptide, ou un complément de celui-ci.

14. Composition pharmaceutique selon la revendication 11 destinée à être utilisée dans un procédé de traitement d'une maladie ou d'un trouble caractérisé(e) par la sous-expression d'un polypeptide chez un sujet, dans laquelle l'agent actif est un plasmide qui code pour le polypeptide ou un variant fonctionnel ou fragment de celui-ci.

15. Composition pharmaceutique selon la revendication 11 destinée à être utilisée dans un procédé d'induction d'une réponse immunitaire chez un sujet, dans laquelle l'agent actif est un oligonucléotide immunostimulateur,
